(19) Europäisches Patentamt
European Patent Office
Office européen des brevets

(11)  **EP 3 307 765 B1**

(12)  **EUROPEAN PATENT SPECIFICATION**

(45) Date of publication and mention
of the grant of the patent:
**10.04.2024   Bulletin 2024/15**

(21) Application number: **16808475.4**

(22) Date of filing: **10.06.2016**

(51) International Patent Classification (IPC):
*C07K 14/435* (2006.01)      *D01F 4/00* (2006.01)
*D01D 5/00* (2006.01)      *C07K 14/00* (2006.01)
*D02G 3/02* (2006.01)      *D02G 3/04* (2006.01)
*D02G 3/36* (2006.01)      *D03D 15/00* (2021.01)

(52) Cooperative Patent Classification (CPC):
**D02G 3/02; C07K 14/00; C07K 14/435; D01F 4/00;
D03D 15/00; D03D 15/233; D03D 15/573;**
D10B 2211/00

(86) International application number:
**PCT/US2016/037084**

(87) International publication number:
**WO 2016/201369 (15.12.2016 Gazette 2016/50)**

(54)  **RECOMBINANT PROTEIN FIBER YARNS WITH IMPROVED PROPERTIES**

GARNE AUS REKOMBINANTEN PROTEINFASERN MIT VERBESSERTEN EIGENSCHAFTEN

FILS DE FIBRES DE PROTÉINE RECOMBINÉE AYANT DES PROPRIÉTÉS AMÉLIORÉES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **11.06.2015   US 201562174377 P
11.06.2015   US 201562174382 P
11.06.2015   US 201562174366 P**

(43) Date of publication of application:
**18.04.2018   Bulletin 2018/16**

(73) Proprietor: **Bolt Threads, Inc.
Emeryville, CA 94608 (US)**

(72) Inventors:
• **WRAY, Lindsay
Emweyville, CA 94608 (US)**

• **KITTLESON, Joshua
Emeryville, CA 94608 (US)**
• **BRESLAUER, David
Emeryville, CA 94608 (US)**
• **BAINBRIDGE, Jamie, McAusland
Emeryville, CA 94608 (US)**

(74) Representative: **Elkington and Fife LLP
Prospect House
8 Pembroke Road
Sevenoaks, Kent TN13 1XR (GB)**

(56) References cited:
EP-A1- 3 271 471        WO-A1-03/060207
WO-A1-2010/123450    WO-A1-2016/201369
WO-A2-2015/042164    WO-A2-2015/042164
US-A1- 2004 102 614    US-A1- 2011 230 911

**Description**

**TECHNICAL FIELD**

The present disclosure relates generally to filament, spun, and blended yarns, and to textiles comprising these yarns. Specifically, the present invention relates to filament, spun, and blended yarns comprising engineered recombinant protein fibers and to textiles comprising these yarns.

**BACKGROUND**

There are many demands for yarns and textiles with improved properties in a wide range of articles such as garments, upholstery textiles and linens. Yarns produced from synthetic fibers typically have some attractive properties such as strength and water repellency, but are inferior to natural fibers in other areas such as water wicking, thermal properties and comfort. Natural fibers tend to have better moisture absorbency, but lack in one or more of mechanical properties, washability and stain resistance.

Some typical synthetic fibers are nylon, acrylic and polyester. There are numerous varieties of each of these types of fibers. Nylon is a general name for a class of aliphatic or semi-aromatic polyamides, which are melt processed into fibers, or other form factors. Acrylic fibers are made from polyacrylonitrile polymers with high molecular weights. Polyester fibers are composed of polymers with the ester functional group in their main chain, most commonly polyethylene terephthalate (PET). There are also some specialty synthetic fibers such as Kevlar, which is the trade name for poly-paraphenylene terephthalamide. Polyester and nylon typically have a tenacity of 5-10 gpd (grams per Denier) and elongation at break of 10-20%, however have relatively poor comfort against the skin, mainly due to the poor moisture management properties (such as absorption and wicking). Dacron polyester, for instance, has a diameter change of only about 0.3% upon immersion in water. Kevlar has a very high tenacity of about 23 gpd, but an elongation at break of only 2-3%.

Rayon is in a sub-set of man-made fibers, which is made from regenerated cellulose. The tensile strength of rayon significantly changes if the fibers are dry or wet. In the dry state, the tensile strength of rayon is approximately 1.5-2.4 gpd. However, in the wet state, the tensile strength drops to approximately 0.7-1.2 gpd. Rayon can also be produced in a high tenacity variety, and the tensile strength can be as high as 3-4.6 gpd in the dry state, and 1.9 to 3.0 gpd in the wet state. Rayon typically has an extensibility of 15-30%. However, rayon suffers from poor durability, poor wrinkle resistance, and poor washability and stain resistance.

Cotton, wool and silk are examples of common natural fibers. Cotton has a tenacity of about 5 gpd, and excellent water absorption properties, but relatively low extensibility (roughly 5%). Wool typically has an extensibility of 30-40%, and excellent water absorption and heat of wetting, but has relatively low tenacity (roughly 1 gpd). Typical silkworm silk has tenacity of roughly 4 gpd, extensibility of 20-30% and good moisture absorbency, however, has poor washability and stain resistance.

Individual fibers are made into yarns to be used in textiles. There are different methods of forming yarns from fibers, which produce yarns with different structures and properties. Different fibers also have different properties, and often require different spinning methods and equipment to produce yarns. Three main types of yarns are filament yarns, spun yarns and blended yarns.

Filament yarns fall into two main classes, flat and textured. Textured yarns have noticeably greater apparent volume than a conventional flat yarn of the same fiber, count and linear density. Some methods of texturing include false twist texturing, air jet texturing, or stuffer box texturing. Fabrics constructed from flat filament yarns will have larger interstices than fabrics constructed from textured yarns. Textured filament yarns have better coverage since the bulk of the yarn fills the interstices between stitches or picks. Fabrics constructed from textured filament yarns therefore have a lower luster and tend to be more absorbent and softer than flat filament yarns. Filament yarns are used in many applications including carpeting and carpet backing, industrial textile products (such as tire cord and tire fabric, seat belts, industrial webbing and tape, tents, fishing line and nets, rope, and tape reinforcement), apparel fabrics (such as women's sheer hosiery, underwear, nightwear, sports apparel, anklets and socks), interior and household products (such as bed ticking, furniture upholstery, curtains, bedspreads, sheets, and draperies).

One of the most common methods of forming a yarn from fibers is spinning, where shorter staple fibers are twisted together to form a longer yarn. There are different methods of spinning yarns, such as ring spinning, open end spinning and air-jet spinning. Ring spinning is a continuous process where the roving (unspun thread with a slight twist) is first attenuated by drawing rollers, then spun and wound around a rotating spindle with the assistance of a traveler which moves along a ring. Open end spinning utilizes a spinning rotor to provide twist to the staple fibers. Air-jet spinning utilizes jets of air to provide twist to the yarn. The structure of the yarn produced by each of these methods is somewhat different. Ring spun yarns typically have an outer sheath of fibers with greater twist (lesser inclination) than in the center core of the yarn. In contrast, yarns produced from the rotor spinning tend to have higher twist towards the core of the yarn than at the periphery. The simplest types of air-jet spun yarns have fibers at the core with substantially no twist, and covering fibers with twist. However, more complex systems of air-jet spinning can produce yarns with more complex structures.

Blending fibers to create yarns is a process where fibers of different types, origins, length, thickness, color or other properties are combined to make a yarn. Blending is typically done in spun yarns, but can also be done in filament or compound yarns. In blended yarns, synthetic fibers are often combined with other synthetic or natural fibers to impart characteristics not achievable with a single type of fiber, such as improved strength, durability, drape, moisture management properties, comfort, washability, cost reduction, or to achieve mixed color or texture effects. For example, polyester is a commonly blended fiber because polyester fibers have certain desirable properties such as strength, abrasion resistance and washability, but poor moisture absorption. Polyester blended with cotton in roughly even proportions creates yarns, which are capable of forming fabrics that are more easily washable and comfortable with a good hand feel, and are commonly used in many garments and home linens. Blends of polyester and worsted wool can create yarns which are capable of being made into fabrics with the drape and feel of wool, with improved durability and resistance to wrinkles.

Since the yarns produced from different fibers and different spinning methods have different properties, the textiles produced from these different yarns also have different properties. For instance, textiles produced from fully twisted ring-spun yarns, which have higher twist at yarn periphery, typically have higher tensile strength but lower abrasion resistance than textiles produced from open-end spun yarns. In contrast, textiles produced from open-end spun yarns, which have higher twist at the yarn core than the periphery, typically have lower strength and higher abrasion resistance than textiles produced from ring-spun yarns. Air-jet spun yarns, which have genuine twist of the fibers at the yarn sheath, typically have very low hairiness, which provide a textile with good resistance to wear, abrasion and piling, and good washability. Some studies have shown that the ratio of woven fabric strength divided by yarn strength is lower for ring-spun yarns as compared to open-end or air-jet spun yarns. It is suggested that the mechanism is that the yarn-to-yarn friction force is lower for ring-spun yarns.

Almost all natural fibers are staple fibers, which have short lengths, and therefore can only be made into spun yarns and cannot be made into filament yarns. The only natural filament fiber (i.e., that occurs in lengths long enough to produce filament yarns) currently used in commercial textiles is silkworm silk.

There are a variety of test methods that have been developed for fiber, yarns and fabrics. The American Association of Textile Chemists and Colorists (AATCC) has developed a series of tests for fibers and textiles. The standard AATCC tests are known to persons of ordinary skill in the textile arts and can be found at in the 2016 AATCC Technical Manual (ISBN 978-1-942323-01-3).

WO-A-2010/123450 describes a method of producing polymers of an isolated spider silk protein involving providing a solution of said spider silk protein in a liquid medium at p H 6.4 or higher and/or an ion composition that prevents polymerisation of the spider silk protein. WO-A-2015/042164 describes methods and compositions directed to synthetic block copolymer proteins, expression constructs for their secretion, recombinant microorganisms for their production, and synthetic fibers (including advantageously, microfibers) comprising these proteins that recapitulate many properties of natural silk. The recombinant microorganisms can be used for the commercial production of silk-like fibers.

Below are examples of specific embodiments for carrying out the present invention. The examples are offered for illustrative purposes only, and are not intended to limit the scope of the present invention in any way. Efforts have been made to ensure accuracy with respect to numbers used (e.g., amounts, temperatures, etc.), but some experimental error and deviation should, of course, be allowed for.

The reagents employed in the examples are generally commercially available or can be prepared using commercially available instrumentation, methods, or reagents known in the art. The foregoing examples illustrate various aspects described herein and practice of the methods described herein. The examples are not intended to provide an exhaustive description of the many different embodiments of the invention. Thus, although the forgoing invention has been described in some detail by way of illustration and example for purposes of clarity of understanding, those of ordinary skill in the art will realize readily that many changes and modifications can be made thereto.

## SUMMARY

The present invention addresses the shortcomings of existing yarns and textiles. Recombinant protein fibers (RPFs) whose properties can be influenced by their composition, structure and processing to obtain improved combinations of mechanical properties, chemical properties, and antimicrobial properties for a given application are presented, along with methods of producing those fibers. The present disclosure also presents filament yarns, spun yarns, and blended yarns formed using these fibers that can be used to manufacture textiles suitable for different applications. Additionally, the combinations of RPFs with certain properties, and yarns and textiles produced from those yarns with certain structures yield yarns and textiles with certain properties designed for various applications. Other advantages of the present invention are described in greater detail below.

An aspect of the invention provides for a yarn, comprising: a plurality of recombinant protein fibers twisted around a common axis, wherein the mean initial modulus of the recombinant protein fiber measured using ASTM D2256-10 or ASTM D3822-14 is greater than 550 cN/tex, and the recombinant protein fiber comprises at least two occurrences of a repeat unit, the repeat unit comprising: more than 150 amino acid residues and having a molecular weight of at least 10kDal; an alanine-rich region with 6 or more consecutive amino acids, comprising an alanine content of at least 80%; and a glycine-rich region with 12 or more consecutive amino acids, comprising a glycine content of at least 40% and an alanine content of less than 30%. In certain embodiments, the yarn is optionally a filament yarn, a spun yarn, or a blended yarn. In certain embodiments, the initial modulus of the recombinant protein fibers measured using ASTM D2256-10 or ASTM D3822-14 is optionally from 550 cN/tex to 1000 cN/tex. In certain embodiments, the recombinant protein fiber comprises at least two occurrences of a repeat unit, the repeat unit comprising: more than 150 amino acid residues and having a molecular weight of at least 10kDal; an alanine-rich region with 6 or more consecutive amino acids, comprising an alanine content of at least 80%; a glycine-rich region with 12 or more consecutive amino acids, comprising a glycine content of at least 40% and an alanine content of less than 30%. In some embodiments, the recombinant protein fiber repeat unit optionally comprises from 150 to 1000 amino acid residues. In some embodiments, the repeat unit optionally has a molecular weight from 10 kDal to 100 kDal. In various embodiments, the repeat unit optionally comprises from 2 to 20 alanine-rich regions. In certain embodiments, each alanine-rich region optionally comprises from 6 to 20 consecutive amino acids and an alanine content from 80% to 100%. In some embodiments, the repeat unit comprises from 2 to 20 glycine-rich regions. In certain embodiments, each glycine-rich region optionally comprises from 12 to 150 consecutive amino acids and a glycine content from 40% to 80%.

The invention provides any of the preceding yarn compositions, wherein the repeat unit optionally comprises 315 amino acid residues, 6 alanine-rich regions, and 6 glycine-rich regions, wherein the alanine-rich regions comprise from 7 to 9 consecutive amino acids, and alanine content of 100%, and wherein the glycine-rich regions comprise from 30 to 70 consecutive amino acids, and glycine content from 40 to 55%.

The invention also provides any of the preceding yarn compositions, wherein the recombinant protein fiber protein sequence optionally comprises repeat units, wherein each repeat unit has at least 95% sequence identity to a sequence that comprises from 2 to 20 quasi-repeat units, each quasi-repeat unit having a composition comprising {GGY-[GPG-X1]n1-GPS-(A)n2}, wherein for each quasi-repeat unit: X1 is independently selected from the group consisting of SGGQQ, GAGQQ, GQGPY, AGQQ, and SQ; and n1 is from 4 to 8, and n2 is from 6 to 10. In certain embodiments, n1 of the quasi-repeat unit is from 4 to 5 for at least half of the quasi-repeat units. In some embodiments, n2 of the quasi-repeat unit is from 5 to 8 for at least half of the quasi-repeat units. In various embodiments, the quasi-repeat unit optionally has at least 95% sequence identity to a MaSp2 dragline silk protein subsequence.

Various embodiments of the invention are any of the preceding yarn compositions, wherein: the alanine-rich regions optionally form a plurality of nanocrystalline beta-sheets; and the glycine-rich regions optionally form a plurality of beta-turn structures.

In certain aspects, the repeat unit of the proteinaceous block copolymer of any of the preceding yarn compositions optionally comprises SEQ ID NO: 1. The invention also provides any of the preceding yarn compositions, wherein the mean diameter change of the fibers is optionally greater than 5% when submerged in water at a temperature of 21 °C +/- 1 °C, and /or wherein the yarn optionally has a mean diameter change from 20% to 35% when submerged in water at a temperature of 21 °C +/- 1 °C. Certain embodiments of the invention are any of the preceding yarn compositions, wherein the mean maximum tensile strength of the recombinant protein fibers measured using ASTM D2256-10 or ASTM D3822-14 is optionally greater than 15 cN/tex, and/or optionally from 15 cN/tex to 100 cN/tex. Various embodiments of the invention are any of the preceding yarn compositions, wherein the mean extensibility of the recombinant protein fibers measured using ASTM D2256-10 or ASTM D3822-14 is optionally is greater than 3% and/or from 3% to 20%.

Certain embodiments of the invention are any of the preceding yarn compositions wherein the mean linear density of the recombinant protein fibers is optionally less than 1 denier, and/or less than 5 denier or between 5 and 10 denier.

Certain embodiments of the invention are any of the preceding yarn compositions wherein the recombinant protein fibers optionally have a mean toughness greater than 2 cN/tex measured using ASTM D2256-10 or ASTM D3822-14.

In certain embodiments, the recombinant protein fibers of any of the preceding yarn compositions optionally have a cross-section that is substantially circular. In some embodiments, the recombinant protein fibers of any of the preceding yarn compositions optionally have a longitudinal axis, an inner surface and an outer surface, the inner surface defining a hollow core parallel to the longitudinal axis of the fiber. In various embodiments, the recombinant protein fibers of any of the preceding yarn compositions optionally have a longitudinal axis and an outer surface, the outer surface including a plurality of corrugations, each corrugation of the plurality substantially parallel to the longitudinal axis of the fiber.

In certain embodiments, any of the preceding yarn compositions optionally have recombinant protein fiber that does not comprise a chemical residue or finish selected from the group consisting of an antimicrobial finish, such as brominated phenols, quaternary ammonium compounds, zirconium peroxide, ethylene oxide, organo-silver and/or tin compounds, a luster finish, such as calendaring, beetling and/or burning-out, a drape finish, such as parchmentizing, acid designs, burning-out and/or sizing, a texture finish, such as shearing, brushing, 3D or raised embossing, pleating, flocking, embroidery, expanded foam, and/or napping, a softening finish, such as silicone compounds, emulsified oils, sulphonated oils, and/or waxes, a wrinkle resistant finish, such as formaldehyde, di-methylol urea, di-methylol ethylene urea, di-methylol di-hydroxyl ethylene urea, and/or modified di-methylol di-hydoxyl ethylene urea, and/or a functional finish, such as a waterproof finish (such as with a resin, wax and/or oil), a water repellant finish (such as silicones, fluorocarbons, and/or paraffins), a flame retardant finish (such as tetrakis hydroxymethyl phosphonium chloride), a moth proof finish (such as fluorine compounds, naphthalene, DDT, paradichloro benzene), a mildew fungus prevention finish (such as boric acid), and an antistatic finishes (such as moisture absorbing films).

In certain other embodiments, any of the preceding yarn compositions optionally have recombinant protein fiber that comprises a chemical residue or finish selected from the group consisting of an antimicrobial finish, such as brominated phenols, quaternary ammonium compounds, zirconium peroxide, ethylene oxide, organo-silver and/or tin compounds, a luster finish, such as calendaring, beetling and/or burning-out, a drape finish, such as parchmentizing, acid designs, burning-out and/or sizing, a texture finish, such as shearing, brushing, 3D or raised embossing, pleating, flocking, embroidery, expanded foam, and/or napping, a softening finish, such as silicone compounds, emulsified oils, sulphonated oils, and/or waxes, a wrinkle resistant finish, such as formaldehyde, di-methylol urea, di-methylol ethylene urea, di-methylol di-hydroxyl ethylene urea, and/or modified di-methylol di-hydoxyl ethylene urea, and/or a functional finish, such as a waterproof finish (such as with a resin, wax and/or oil), a water repellant finish (such as silicones, fluorocarbons, and/or paraffins), a flame retardant finish (such as tetrakis hydroxymethyl phosphonium chloride), a moth proof finish (such as fluorine compounds, naphthalene, DDT, paradichloro benzene), a mildew fungus prevention finish (such as boric acid), and an antistatic finishes (such as moisture absorbing films).

In certain embodiments, any of the preceding yarn compositions optionally are a blended yarn comprising recombinant protein fibers; and fibers selected from the group consisting of cotton, wool, merino, mohair, polyamide, linen, acrylic, polyester, spandex, and combinations thereof.

In an embodiment, any of the preceding yarn compositions optionally have a yarn twist is from 5 to 100 turns per centimeter.

In certain embodiments, the maximum tensile strength of any of the preceding yarn compositions is optionally greater than 1 cN/tex, and/or from 1 cN/tex to 100 cN/tex measured using ASTM D2256-10.

In certain embodiments, the initial modulus of the yarn of any of the preceding yarn compositions is optionally greater than 550 cN/tex and/ or from 550 cN/tex to 1000 cN/tex measured using ASTM D2256-10.

In an embodiment, the extensibility of any of the preceding yarn compositions is optionally greater than 3%, and/or from 3% to 20% measured using ASTM D2256-10.

In an embodiment, the recombinant protein fibers of any of the preceding yarn compositions are texturized.

An aspect of the invention provides a textile comprising any of the preceding yarn compositions, wherein the textile comprises a plain weave 1/1 textile with warp density of 72 warps/cm and pick density of 40 picks/cm and wherein the textile has a mean horizontal wicking rate greater than 1 mm/s when tested using a standard moisture wicking assay. In an embodiment, the textile optionally has an increase in colony forming units less than 100 times in 24 hours when tested using a standard antimicrobial assay. In an embodiment, the textile optionally is a knitted textile. In certain embodiments, the textile is optionally selected from the group consisting of a circular-knitted textile, flat-knitted textile, or a warp-knitted textiles. In an embodiment, the textile is a woven textile. In certain embodiments, the textile is selected from the group consisting of a plain weave textile, dobby weave textile, and jacquard weave textile. In various embodiments, the textile is a non-woven textile. In certain embodiments, the textile is selected from the group consisting of a needle punched textile, spunlace textile, wet-laid textile, dry-laid textile, melt-blown textile, and 3-D printed non-woven textile.

In certain aspects, the invention provides for a highly comfortable textile, comprising any of the preceding yarn compositions, wherein: when submerged in water at a temperature of 21 °C +/- 1 °C, the recombinant protein fiber has a mean diameter change of greater than 5%; and a mean denier less than 5; and when tested using a standard moisture wicking assay, the textile has a mean horizontal wicking rate greater than 1 mm/s. In an embodiment, the highly comfortable textile comprises a plain weave 1/1 textile with warp density of 72 warps/cm, and a pick density of 40 picks/cm.

In an aspect, the invention provides for an ultra-soft textile, comprising any of the preceding yarn compositions, wherein the textile is a knitted textile, a woven textile, or a non-woven textile, and the yarn comprises: an outer sheath comprising the recombinant protein fiber, wherein the outer sheath comprises a greater twist (lesser inclination) as compared to a twist in a center core of the filament yarn; and wherein the mean dernier of the recombinant protein fiber is less than 5.

**BRIEF DESCRIPTION OF THE DRAWINGS**

FIG. 1 schematically illustrates a molecular structure of a block copolymer of the present disclosure, in an embodiment.

FIGS. 2A-2D show stress-strain curves measured from fibers of the present disclosure, in embodiments.

FIG. 3A shows optical microscope images of dry and hydrated fibers of the present disclosure, in an embodiment. Scale bar = 200 μm.

FIG. 3B shows a plot of the weight of a fiber of the present disclosure, as it is being heated at 110 °C and losing moisture, in an embodiment.

FIG. 4 shows images of a filament yarn, a spun yarn, and three blended yarns, all comprising RPFs of the present disclosure, in embodiments.

FIGS. 5A-5E show stress-strain curves measured from yarns of the present disclosure, in embodiments.

The figures depict various embodiments of the present disclosure for purposes of illustration only. One skilled in the art will readily recognize from the following discussion that alternative embodiments of the structures and methods illustrated herein may be employed without departing from the principles described herein.

**DEFINITIONS**

Filament yarns are yarns that are composed of more than one fiber filaments that run the whole length of the yarn. Filament yarns can also be referred to as multi-filament yarns. The structure of a filament yarn is influenced by the amount of twist, and in some cases the fiber texturing. The properties of the filament yarn can be influenced by the structure of the yarn, fiber to fiber friction of the constituent fibers, and the properties of the constituent fibers. In some embodiments, the yarn structure and the recombinant protein fiber properties are chosen to impart various characteristics to the resulting yarns. The properties of the yarn can also be influenced by the number of fibers (i.e., filaments) in the yarn. The filament yarns in this application can be multifilament yarns. Throughout this disclosure "filament yarns" can refer to flat filament yarns, textured filament yarns, drawn filament yarns, undrawn filament yarns, or filament yarns of any structure.

Spun yarn is made by twisting staple fibers together to make a cohesive yarn (or thread, or "single"). The structure of a spun yarn is influenced by the spinning methods parameters. The properties of the spun yarn are influenced by the structure of the yarn, as well as the constituent fibers.

Blended yarns are a type of yarn comprising various fibers being blended together. In different embodiments, the recombinant protein fibers can be blended with cotton, wool, other animal fibers, polyamide, acrylic, nylon, linen, polyester, and/or combinations thereof. Recombinant protein fibers can be blended with non-recombinant protein fibers (non-RPFs), or with more than one other type of non-recombinant protein fibers. Recombinant protein fibers can also be blended with a second type of recombinant protein fiber with different properties than the first type of recombinant protein fibers. In this disclosure, blended yarns specifically refer to recombinant protein fibers (RPFs) blended with non-recombinant protein fibers or a second type of recombinant protein fibers into a yarn. Even though spandex is generally incorporated into a yarn using somewhat different methods and structures than the other blended yarns described above (e.g., a wrapped RFP/spandex yarn has spandex core wrapped with RPF in order to hide the spandex from view in the textile), a composite RPF/spandex yarn therefore is another example of a blended yarn.

Recombinant protein fibers (RPFs) are fibers that are produced from recombinant proteins. In some cases, the proteins making up the RPFs can contain concatenated repeat units and quasi-repeat units. Repeat units are defined as amino acid sequences that are repeated exactly within the polypeptide. Quasi-repeats are inexact repeats, i.e., there is some sequence variation from quasi-repeat to quasi-repeat. Each repeat can be made up of concatenated quasi-repeats.

The standard test method for measuring tensile properties of yarns (or multiple fibers in a tow) by the single-strand method is ASTM D2256-10. The standard test method for measuring tensile properties of single fibers is ASTM D3822-14. All fiber and yarn mechanical properties measured in this disclosure are measured using one of these standards.

"Textured" fibers or yarns are fibers or yarns that have been subjected to processes that arrange the straight filaments into crimped, coiled or looped filaments. Some examples of methods used for processing textured fibers and yarns are air jet texturing, false twist texturing, or stuffer box texturing.

The "work of rupture" of a fiber or yarn is the work done from the point of the pretension load to the point of the breaking load. The energy required to bring a fiber or yarn to the breaking load can be obtained from the area under the load-elongation curve. The units of work of rupture can therefore be cN*cm. The "toughness" of a fiber or yarn is the energy per unit mass required to rupture the fiber or yarn. The toughness is the integral of the stress-strain curve, and can be calculated by dividing the work of rupture by the mass of the sample of fiber or yarn being tested. The units of toughness can therefore be cN/tex.

Throughout this disclosure, and in the claims, when percentages of amino acids are recited, that percentage indicates a mole fraction percentage (not a weight fraction percentage).

Throughout this disclosure, and in the claims, where method steps are recited, the order in which the steps are carried out can be varied from the order in which they are described, so long as an operable method results.

**DETAILED DESCRIPTION**

ENGINEERING RECOMBINANT PROTEIN FIBERS FOR YARNS

Recombinant protein fibers can be engineered to have different mechanical, structural, chemical, and biological properties. Some methods to engineer recombinant protein fibers for different properties are protein sequence design (e.g., higher ratio of GPG to poly-alanine to improve elasticity, where glycine is between 25-50% of the polypeptide), and/or microorganism strain design and/or growth conditions and/or protein purification (e.g., utilizing secretion pathways to increase monodispersity to improve tensile strength), and/or fiber spinning conditions (e.g., changing spinneret diameter to tune fiber diameter).

Embodiments of the present disclosure include filament yarns, spun yarns, and blended yarns comprising recombinant protein fibers. In many embodiments, the recombinant protein fibers are engineered to comprise various improved mechanical, structural, chemical and biological properties. In embodiments, the yarn structure and the recombinant protein fiber properties are chosen to impart various characteristics to the resulting yarns, and textiles fabricated from the yarns.

In some embodiments, the hydrophilicity and/or moisture absorption of the fibers can be engineered by changing the protein sequence. In some embodiments, the recombinant protein fiber (i.e., RPF) hydrophilicity and/or moisture absorptivity is increased by increasing the ratio of substantially hydrophilic to substantially hydrophobic amino acids in the sequence, without disrupting fiber forming features such as poly-alanine stretches. Examples of relatively polar (relatively hydrophilic) amino acids in recombinant spider silk polypeptide sequences are glutamine, serine and tyrosine, while glycine and alanine are relatively hydrophobic. In some embodiments, a filament yarn, or spun yarn, or blended yarn comprising hydrophilic recombinant protein fibers comprises greater than 25% glycine, or greater than 30% glycine, or greater than 35% glycine, or greater than 40% glycine, or greater than 45% glycine, or between 25% and 45% or between 25% and 40% or between 25% and 35% glycine, or between 35% and 45% glycine, or between 35% and 40% glycine, or between 40% and 45% glycine. In some embodiments, filament yarn, or spun yarn, or blended yarn comprising hydrophilic recombinant protein fibers comprises greater than 5% glutamine, or greater than 10% glutamine, or greater than 15% glutamine, or greater than 20% glutamine, or greater than 25% glutamine, or between 5% and 10% glutamine, or between 10% and 15% glutamine, or between 15% and 20% glutamine, or between 20% and 25% glutamine. In some embodiments, a filament yarn, or spun yarn, or blended yarn comprising highly moisture absorbing recombinant protein fibers comprises greater than 25% glycine, or greater than 30% glycine, or greater than 35% glycine, or greater than 40% glycine, or greater than 45% glycine, or between 25% and 45% or between 25% and 40% or between 25% and 35% glycine, or between 35% and 45% glycine, or between 35% and 40% glycine, or between 40% and 45% glycine. In some embodiments, a filament yarn, or spun yarn, or blended yarn comprising highly moisture absorbing recombinant protein fibers comprises greater than 5% glutamine, or greater than 10% glutamine, or greater than 15% glutamine, or greater than 20% glutamine, or greater than 25% glutamine, or between 5% and 10% glutamine, or between 10% and 15% glutamine, or between 15% and 20% glutamine, or between 20% and 25% glutamine. In some embodiments, a highly moisture absorbing RPF, upon being submerged in water at a temperature of 21 °C +/- 1 °C, can have a median or mean diameter change greater than 10%, or greater than 15%, or greater than 20%, or greater than 25%, or greater than 30%, or greater than 35%, or greater than 40%, or greater than 45%, or greater than 50%, or greater than 60%, or greater than 70%, or greater than 80%, or greater than 90%, or from 10% to 20%, or from 20% to 30%, or from 30% to 40%, or from 40% to 50%, or from 50% to 60%, or from 60% to 70%, or from 70% to 80%, or from 80% and 90%, or from 90% to 100%, or from 20% to 35%, or from 15% to 40%, or from 15% to 35%.

In some embodiments, the wickability of textiles can be engineered by changing the spinning parameters of the fibers making up the textile. In some embodiments, the fiber cross-section shape can be changed by changing the residence time in the coagulation bath, or by changing the ratio of protein solvent to protein non-solvent in the coagulation bath. The fibers of the present disclosure processed with residence times in coagulation baths at the longer end of the disclosed range (such as greater than 60 seconds) produce corrugated cross sections. That is, each fiber has a plurality of corrugations (or alternatively "grooves") disposed at an outer surface of a fiber. Each of these corrugations is parallel to a longitudinal axis of the corresponding fiber on which the corrugations are disposed. These corrugations can act as channels to assist in the wicking of liquids including water. Theses RPFs with tailored cross-sections can be formed into filament yarns, or spun yarns, or blended yarns. Filament yarn, or spun yarn, or blended yarn containing RPFs with tailored cross-sections can be used to make textiles with tailored moisture transport properties, such as higher wicking rates.

In some embodiments, antimicrobial protein motifs are added to the protein sequence to impart antimicrobial properties to the resulting fibers, as well as improve the antimicrobial properties of filament yarns, or spun yarns, or blended yarns, and fabrics comprising the recombinant protein fibers. Some examples of antimicrobial protein sequence motifs are the human antimicrobial peptides human neutrophil defensin 2 (HNP-2), human neutrophil defensins 4 (HNP-4) and hepcidin. These antimicrobial amino acid sequences can be added to the spider silk-derived polypeptide sequence after every quasi-repeat unit, or every 2 quasi-repeat units, or every 3 quasi-repeat units, or every 4 quasi-repeat units, or every 5 quasi-repeat units, or every 6 quasi-repeat units, or every 7 quasi-repeat units, or every 8 quasi-repeat units, or every 9 quasi-repeat units, or every 10 quasi-repeat units, or every 12 quasi-repeat units, or every 14 quasi-repeat units, or every 16 quasi-repeat units, or every 18 quasi-repeat units, or every 20 quasi-repeat units, or every 30 quasi-repeat units, or every 40 quasi-repeat units, or every 50 quasi-repeat units, or every 60 quasi-repeat units, or every 70 quasi-repeat units, or every 80 quasi-repeat units, or every 90 quasi-repeat units, or every 100 quasi-repeat units. In some embodiments, a textile, comprising filament yarn, or spun yarn, or blended yarn, comprising recombinant protein fibers with such antimicrobial amino acid sequences, is tested using AATCC test method 100-2012, and has an increase in colony forming units less than 100 times in 24 hours, or has an increase in colony forming units less than 500 times in 24 hours, or has an increase in colony forming units less than 1000 times in 24 hours, or has a change in colony forming units from a 100 times reduction to a 1000 times increase in 24 hours.

In some embodiments, the extensibility of the fiber is increased by increasing the ratio of GPG to poly-alanine in the protein sequence. In some embodiments, a yarn comprising recombinant protein fibers with a high degree of extensibility (such as extensibility greater than 3%, or greater than 10%, or greater than 20%, or greater than 30%, or from 3 to 30%, or from 3 to 100%), comprises greater than 25% glycine, or greater than 30% glycine, or greater than 35% glycine, or greater than 40% glycine. In some embodiments, a yarn comprising recombinant protein fibers with a high degree of elasticity comprises greater than 45% glycine, or between 25% and 45% or from 25% to 40% or from 25% to 35% glycine, or from 35% to 45% glycine, or from 35% to 40% glycine, or from 40% to 45% glycine.

In some embodiments, the maximum tensile strength of the fiber is increased by increasing the monodispersity of the protein. In some embodiments, the monodispersity of the protein is improved by engineering the strain of the microorganism used to produce the recombinant protein to secrete the protein. In turn, improved monodispersity improves the maximum tensile strength of the fibers. In some embodiments, the proteins of the spin dope (the synthesis of which is described in WO2015042164 A2, especially at paragraphs 114-134), expressed from any of the polypeptides of the present disclosure, comprising the recombinant protein fibers with a high tensile strength (such as greater than 10 cN/tex), are substantially monodisperse. In this disclosure, "substantially monodisperse" can be >50%, or >55%, or >60%, or >65%, or >70%, or >75%, or >80%, or >85%, or >90%, or >95%, or >99% of the protein in the spin dope (percentages here are mass percentages) having molecular weight >50%, or >55%, or >60%, or >65%, or >70%, or >75%, or >80%, or >85%, or >90%, or >95%, or >99% of the full-length molecular weight of the encoded protein. In this disclosure "substantially monodisperse" also encompasses spin dope mixtures in which from 50% to 100%, or from 60% to 100%, or from 70% to 100%, or from 80% to 100%, or from 90% to 100%, or from 50% to 99%, or from 60% to 99%, or from 70% to 99%, or from 80% to 99%, or from 90% to 99% of the protein in the spin dope (percentages here are mass percentages) having molecular weight from 50% to 100%, or from 60% to 100%, or from 70% to 100%, or from 80% to 100%, or from 90% to 100%, or from 50% to 99%, or from 60% to 99%, or from 70% to 99%, or from 80% to 99%, or from 90% to 99% of the full-length molecular weight of the encoded protein.

Work of rupture is a measure of toughness and combines elasticity and tenacity. Therefore, in some embodiments, the toughness of the RPFs is increased by combining protein sequence engineering and strain engineering to simultaneously increase the elasticity and the tenacity, as described in this disclosure. In some embodiments, a filament yarn, or spun yarn, or blended yarn comprising recombinant protein fibers with a high degree of toughness (such as greater than 100 cN/tex measured using ASTM D2256-10 or ASTM D3822-14), comprises greater than 25% glycine, or greater than 30% glycine, or greater than 35% glycine, or greater than 40% glycine, or greater than 45% glycine, or between 25% and 45% or between 25% and 40% or between 25% and 35% glycine, or between 35% and 45% glycine, or between 35% and 40% glycine, or between 40% and 45% glycine. In some embodiments, a filament yarn, or spun yarn, or blended yarn comprising recombinant protein fibers with a high work of rupture (such as greater than 0.5 cN*cm measured using ASTM D2256-10 or ASTM D3822-14), comprises greater than 25% glycine, or greater than 30% glycine, or greater than 35% glycine, or greater than 40% glycine, or greater than 45% glycine, or between 25% and 45% or between 25% and 40% or between 25% and 35% glycine, or between 35% and 45% glycine, or between 35% and 40% glycine, or between 40% and 45% glycine. In some embodiments, the proteins of the spin dope (the synthesis of which is described in WO2015042164 A2, especially at paragraphs 114-134), expressed from any of the polypeptides of the present disclosure, comprising the recombinant protein fibers with a high degree of toughness (such as greater than 100 cN/tex measured using ASTM D2256-10 or ASTM D3822-14) or a high work of rupture (such as greater than 0.5 cN*cm measured using ASTM D2256-10 or ASTM D3822-14), are substantially monodisperse.

In some embodiments, the initial modulus of the fiber is increased by engineering the proteins to have better intermolecular forces. In some embodiments, intermolecular forces are increased by adding protein blocks that provide hydrogen bonding and cross-linking bonds between the molecules that comprise the fiber. One example of a protein motif that improves the intermolecular forces is by increasing the number of polyalanine segments for intermolecular crystallization. Another example of polypeptide engineering to increase intermolecular forces is through the addition of amino acids that are capable of covalently cross-linking such as the disulfide bridges of cysteine. A filament yarn, or spun yarn, or blended yarn can comprise RPFs with tailored intermolecular forces and have high initial modulus. In some embodiments an RPF with engineered polypeptides described above can have a high initial modulus greater than 50 cN/tex, or greater than 115 cN/tex, or greater than 200 cN/tex, or greater than 400 cN/tex, or greater than 550 cN/tex, or greater than 600 cN/tex, or greater than 800 cN/tex, or greater than 1000 cN/tex, or greater than 2000 cN/tex, or greater than 3000 cN/tex, or greater than 4000 cN/tex, or greater than 5000 cN/tex, or from 200 to 900 cN/tex, or from 100 to 7000 cN/tex, or from 500 to 7000 cN/tex, or from 50 to 7000 cN/tex, or from 100 to 5000 cN/tex, or from 500 to 5000 cN/tex, or from 50 to 5000 cN/tex, or from 100 to 2000 cN/tex, or from 500 to 2000 cN/tex, or from 50 to 2000 cN/tex, or from 100 to 1000 cN/tex, or from 500 to 1000 cN/tex, or from 50 to 1000 cN/tex, or from 50 to 500 cN/tex, or from 100 to 1000 cN/tex, or from 500 to 1000 cN/tex, or from 100 to 700 cN/tex (measured using ASTM D2256-10 or ASTM D3822-14).

In some embodiments, the initial modulus of the fiber is increased by increasing the draw ratio of the fiber during spinning. In some embodiments, a yarn comprising recombinant protein fibers with a high initial modulus has a draw ratio of greater than 1.5X, or greater than 2X, or greater than 3X, or greater than 4X, or greater than 5X, or greater than 6X, or greater than 8X, or greater than 10X, or greater than 15X, or greater than 20X, or greater than 25X, or greater than 30X, or from 1.5X to 30X, or from 1.5X to 20X, or from 1.5X to 15X, or from 1.5X to 10X, or from 1.5X to 6X, or 1.5X to 4X, or from 2X to 30X, or from 2X to 20X, or from 2X to 15X, or from 2X to 10X, or from 2X to 6X, or from 2X to 4X, or from 4X to 30X, or from 4X to 20X, or from 4X to 15X, or from 4X to 10X, or from 4X to 6X, or from 6X to 30X, or from 6X to 20X, or from 6X to 15X, or from 6X to 10X, or from 10X to 30X, or from 10X to 20X, or from 10X to 15X.

In some embodiments the fiber cross-section shape is changed by changing the spinneret orifice shapes. In some embodiments, the fiber diameter or linear density is increased or decreased by increasing or decreasing the spinneret orifice diameter. The softness of a fiber is highly influenced by the diameter or linear density, and in some embodiments, the spinneret diameter can also be used to tune the softness of the fiber by decreasing the fineness of the fibers. In some embodiments, the linear density of the fiber can be tuned from less than 10 dtex, or less than 5 dtex, or less than 1 dtex, or from 1 to 20 dtex, or from 1 to 10 dtex by using a draw ratio during spinning of greater than 1.5X, or greater than 2X, or greater than 3X, or greater than 4X, or greater than 5X, or greater than 6X, or greater than 8X, or greater than 10X, or greater than 15X, or greater than 20X, or greater than 25X, or greater than 30X, or from 1.5X to 30X, or from 1.5X to 20X, or from 1.5X to 15X, or from 1.5X to 10X, or from 1.5X to 6X, or 1.5X to 4X, or from 2X to 30X, or from 2X to 20X, or from 2X to 15X, or from 2X to 10X, or from 2X to 6X, or from 2X to 4X, or from 4X to 30X, or from 4X to 20X, or from 4X to 15X, or from 4X to 10X, or from 4X to 6X, or from 6X to 30X, or from 6X to 20X, or from 6X to 15X, or from 6X to 10X, or from 10X to 30X, or from 10X to 20X, or from 10X to 15X. In some embodiments, a textile with good softness contains filament yarn, or spun yarn, or blended yarn comprising fibers with fiber linear density less than 10 dtex, or less than 5 dtex, or less than 1 dtex, or from 1 to 20 dtex, or from 1 to 10 dtex. The drape of a fabric is highly influenced by the linear density or diameter of the fibers comprising the fabric, and in some embodiments, the spinneret diameter or the draw ratio can also be used to tune the drape of a fabric by increasing or decreasing the fineness of the fibers comprising the fabric. In some embodiments, a textile with desirable drape contains filament yarn, or spun yarn, or blended yarn comprising fibers with fiber linear density less than 10 dtex, or less than 5 dtex, or less than 1 dtex, or from 1 to 20 dtex, or from 1 to 10 dtex.

In some embodiments, the RPF cross-section shape can be changed by changing the residence time in the coagulation bath, or by changing the ratio of protein solvent to protein non-solvent in the coagulation bath. The RPFs of the present disclosure processed with residence times in coagulation baths at the longer end of the disclosed range produce corrugated cross sections. That is, each RPF has a plurality of corrugations (or alternatively "grooves") disposed at an outer surface of a fiber. Each of these corrugations is parallel to a longitudinal axis of the corresponding fiber on which the corrugations are disposed. The luster of a fiber is also highly influenced by the smoothness of the surface. A RPF with a smoother surface has a higher luster, and in some embodiments, the luster of the fiber can also be tuned by changing the coagulation bath residence time or chemistry. A filament yarn, or spun yarn, or blended yarn can contain RPFs with tailored cross-sections to create a yarn with low or high luster.

RECOMBINANT PROTEIN FIBER PROTEIN DESIGN

Embodiments of the present disclosure include fibers synthesized from synthetic proteinaceous copolymers based on recombinant spider silk protein fragment sequences derived from MaSp2, such as from the species *Argiope*

*bruennichi.* Each synthesized fiber contains protein molecules that include two to twenty repeat units, in which a molecular weight of each repeat unit is greater than about 20 kDal. Within each repeat unit of the copolymer are more than about 60 amino acid residues that are organized into a number of "quasi-repeat units." In some embodiments, the repeat unit of a polypeptide described in this disclosure has at least 95% sequence identity to a MaSp2 dragline silk protein sequence.

Utilizing long polypeptides with fewer long exact repeat units has many advantages over utilizing polypeptides with a greater number of shorter exact repeat units to create a recombinant spider silk fiber. An important distinction is that a "long exact repeat" is defined as an amino acid sequence without shorter exact repeats concatenated within it. Long polypeptides with long exact repeats are more easily processed than long polypeptides with a greater number of short repeats because they suffer less from homologous recombination causing DNA fragmentation, they provide more control over the composition of amorphous versus crystalline domains, as well as the average size and size distribution of the nano-crystalline domains, and they do not suffer from unwanted crystallization during intermediate processing steps prior to fiber formation. Throughout this disclosure the term "repeat unit" refers to a subsequence that is exactly repeated within a larger sequence.

Throughout this disclosure, wherever a range of values is recited, that range includes every value falling within the range, as if written out explicitly, and further includes the values bounding the range. Thus, a range of "from X to Y" includes every value falling between X and Y, and includes X and Y.

The term percent "identity," in the context of two or more nucleic acid or polypeptide sequences, refer to two or more sequences or subsequences that have a specified percentage of nucleotides or amino acid residues that are the same, when compared and aligned for maximum correspondence, as measured using one of the sequence comparison algorithms described below (e.g., BLASTP and BLASTN or other algorithms available to persons of skill) or by visual inspection. Depending on the application, the percent "identity" can exist over a region of the sequence being compared (i.e., subsequence), e.g., over a functional domain, or, alternatively, exist over the full length of the two sequences to be compared. Within this disclosure, a "region" is considered to be 6 or more amino acids in a continuous stretch within a polypeptide.

For sequence comparison, typically one sequence acts as a reference sequence to which test sequences are compared. When using a sequence comparison algorithm, test and reference sequences are input into a computer, subsequence coordinates are designated, if necessary, and sequence algorithm program parameters are designated. The sequence comparison algorithm then calculates the percent sequence identity for the test sequence(s) relative to the reference sequence, based on the designated program parameters.

Optimal alignment of sequences for comparison can be conducted, e.g., by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Nat'l. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, Wis.), or by visual inspection (*see* generally Ausubel et al., infra).

One example of an algorithm that is suitable for determining percent sequence identity and sequence similarity is the BLAST algorithm, which is described in Altschul et al., J. Mol. Biol. 215:403-410 (1990). Software for performing BLAST analyses is publicly available through the National Center for Biotechnology Information. Such software also can be used to determine the mole percentage of any specified amino acid found within a polypeptide sequence or within a domain of such a sequence. As the person of ordinary skill will recognize such percentages also can be determined through inspection and manual calculation.

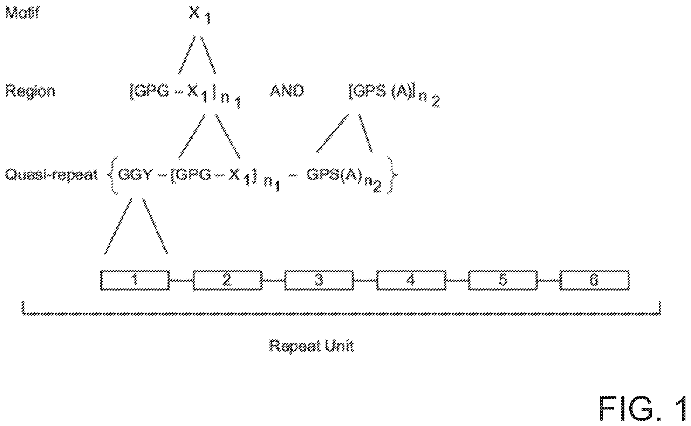

FIG. 1 schematically illustrates an example copolymer molecule of the present disclosure, in an embodiment. A block copolymer molecule of the present disclosure includes in each repeat unit more than 60, or more than 100, or more than 150, or more than 200, or more than 250, or more than 300, or more than 350, or more than 400, or more than 450, or more than 500, or more than 600, or more than 700, or more than 800, or more than 900, or more than 1000 amino acid residues, or from 60 to 1000, or from 100 to 1000, or from 200 to 1000, or from 300 to 1000, or from 400 to 1000, or from 500 to 1000, or from 150 to 1000, or from 150 to 400, or from 150 to 500, or from 150 to 750, or from 200 to 400, or from 200 to 500, or from 200 to 750, or from 250 to 350, or from 250 to 400, or from 250 to 500, or from 250 to 750, or from 250 to 1000, or from 300 to 500, or from 300 to 750 amino acid residues. Each repeat unit of the polypeptide molecules of this disclosure can have a molecular weight from 20 kDal to 100 kDal, or greater than 20 kDal, or greater than 10 kDal, or greater than 5 kDal, or from 5 to 60 kDal, or from 5 to 40 kDal, or from 5 to 20 kDal, or from 5 to 100 kDal, or from 5 to 50 kDal, or from 10 to 20 kDal, or from 10 to 40 kDal, or from 10 to 60 kDal, or from 10 to 100 kDal, or from 10 to 50 kDal, or from 20 to 100 kDal, or from 20 to 80 kDal, or from 20 to 60 kDal, or from 20 to 40 kDal, or from 20 to 30 kDal. A copolymer molecule of the present disclosure can include in each repeat unit more than 300 amino acid residues. A copolymer molecule of the present disclosure can include in each repeat unit about 315 amino acid residues. These amino acid residues are organized within the molecule at several different levels. A copolymer molecule of the present disclosure includes from 2 to 20 occurrences of a repeat unit. After concatenating the repeat unit, the polypeptide molecules of this disclosure can be from 20 kDal to 2000 kDal, or greater than 20 kDal, or greater than 10 kDal, or greater than 5 kDal, or from 5 to 400 kDal, or from 5 to 300 kDal, or from 5 to 200 kDal, or from 5 to 100 kDal, or from 5 to 50 kDal, or from 5 to 500 kDal, or from 5 to 1000 kDal, or from 5 to 2000 kDal, or from 10 to 400 kDal, or from 10 to 300 kDal, or from 10 to 200 kDal, or from 10 to 100 kDal, or from 10 to 50 kDal, or from 10 to 500 kDal, or from 10 to 1000 kDal, or from 10 to 2000 kDal, or from 20 to 400 kDal, or from 20 to 300 kDal, or from 20 to 200 kDal, or from 40 to 300 kDal, or from 40 to 500 kDal, or from 20 to 100 kDal, or from 20 to 50 kDal, or from 20 to 500 kDal, or from 20 to 1000 kDal, or from 20 to 2000 kDal. As shown in FIG. 1, each "repeat unit" of a copolymer fiber comprises from two to twenty "quasi-repeat" units (i.e., n3 is from 2 to 20). Quasi-repeats do not have to be exact repeats. Each repeat can be made up of concatenated quasi-repeats. Equation 1 shows the composition of a quasi-repeat unit according the present disclosure.

$$\{GGY\text{-}[\mathbf{GPG}\text{-}X_1]_{n1}\text{-}\mathbf{GPS}\text{-}(A)_{n2}\}_{n3}. \qquad \text{(Equation 1)}$$

The variable compositional element $X_1$ (termed a "motif") is according to any one of the following amino acid sequences shown in Equation 2 and $X_1$ varies randomly within each quasi-repeat unit.

$$X_1 = SGGQQ \text{ or } GAGQQ \text{ or } GQGPY \text{ or } AGQQ \text{ or } SQ \qquad \text{(Equation 2)}$$

Referring again to Equation 1, the compositional element of a quasi-repeat unit represented by "GGY-[$\mathbf{GPG}$-$X_1$]$_{n1}$-GPS" in Equation 1 is referred to a "first region." A quasi-repeat unit is formed, in part by repeating from 4 to 8 times the first region within the quasi-repeat unit. That is, the value of $n_1$ indicates the number of first region units that are repeated within a single quasi-repeat unit, the value of $n_1$ being any one of 4, 5, 6, 7 or 8. The compositional element represented by "$(A)_{n2}$" is referred to a "second region" and is formed by repeating within each quasi-repeat unit the amino acid sequence "A" $n_2$ times. That is, the value of $n_2$ indicates the number of second region units that are repeated within a single quasi-repeat unit, the value of $n_2$ being any one of 6, 7, 8, 9, 10, 11, 12, 13, 14, 15, 16, 17, 18, 19, or 20. In some embodiments, the repeat unit of a polypeptide of this disclosure has at least 95% sequence identity to a sequence containing quasi-repeats described by Equations 1 and 2. In some embodiments, the repeat unit of a polypeptide of this disclosure has at least 80%, or at least 90%, or at least 95%, or at least 99% sequence identity to a sequence containing quasi-repeats described by Equations 1 and 2.

The first region described in Equation 1 is considered a glycine-rich region. A region can be glycine-rich if 6 or more consecutive amino acids within a sequence are more than 45% glycine. A region can be glycine-rich if 12 or more consecutive amino acids within a sequence are more than 45% glycine. A region can be glycine-rich if 18 or more consecutive amino acids within a sequence are more than 45% glycine. A region can be glycine-rich if 4 or more, or 6 or more, or 10 or more, or 12 or more, or 15 or more, or 20 or more, or 25 or more, or 30 or more, or 40 or more, or 50 or more, or 60 or more, or 70 or more, or 80 or more, or 100 or more, or 150 or more consecutive amino acids within a sequence are more than 30%, or more than 40%, or more than 45%, or more than 50%, or more than 55% glycine, or more than 60% glycine, or more than 70% glycine, or more than 80% glycine, or from 30% to 80%, or from 40% to 80%, or from 45% to 80%, or from 30% to 55%, or from 30% to 50%, or from 30% to 45%, or from 30% to 40%, or from 40% to 50%, or 40% to 55%, or 40% to 60% glycine. A region can be glycine-rich if from 5 to 150, or from 10 to 150, or from 12 to 150, or from 12 to 100, or from 12 to 80, or from 12 to 60, or from 20 to 60 consecutive amino acids within a sequence are more than 30%, or more than 40%, or more than 45%, or more than 50%, or more than 55% glycine, or more than 60% glycine, or more than 70% glycine, or more than 80% glycine, or from 30% to 80%, or from 40% to 80%, or from 45% to 80%, or from 30% to 55%, or from 30% to 50%, or from 30% to 45%, or from 30% to 40%, or from 40% to 50%, or 40% to 55%, or 40% to 60% glycine. In addition, a glycine-rich region can have less than 10%, or less than 20%, or less than 30%, or less than 40% alanine, or from about 0% to 10%, or from about 0% to 20%, or from about 0% to 30%, or from about 0% to 40%, or alanine. A region can be alanine-rich if 4 or more, or 6 or more, or 8 or more, or 10 or more consecutive amino acids within a sequence are more than 70%, or more than 75%, or more than 80%, or more than 85%, or more than 90% alanine, or from 70% to about 100%, or from 75% to about 100%, or from 80% to about 100%, or from 85% to about 100%, or from 90% to about 100% alanine. A region can be alanine-rich if from 4 to 10, or from 4 to 12, or from 4 to 15, or from 6 to 10, or from 6 to 12, or from 6 to 15, or from 4 to 20, or from 6 to 20 consecutive amino acids within a sequence are more than 70%, or more than 75%, or more than 80%, or more than 85%, or more than 90% alanine, or from 70% to about 100%, or from 75% to about 100%, or from 80% to about 100%, or from 85% to about 100%, or from 90% to about 100% alanine. The repeats described in this disclosure can have 6, or more than 2, or more than 4 or more than 6, or more than 8, or more than 10, or more than 15, or more than 20, or from 2 to 25, or from 2 to 10, or from 4 to 10, or from 2 to 8, or from 4 to 8 alanine-rich regions. The repeats described in this disclosure can have 6, or more than 2, or more than 4 or more than 6, or more than 8, or more than 10, or more than 15, or more than 20, or from 2 to 25, or from 2 to 10, or from 4 to 10, or from 2 to 8, or from 4 to 8 glycine-rich regions.

In some embodiments, a filament yarn, or spun yarn, or blended yarn contains RPFs with proteins containing SEQ described by Equation 1 and Equation 2. In some embodiments, a filament yarn, or spun yarn, or blended yarn contains recombinant protein fibers with repeat units, where each repeat unit has at least 95% sequence identity to a sequence that comprises from 2 to 20 quasi-repeat units, and each quasi-repeat unit has a composition of {GGY-[GPG-X1]$_{n1}$-GPS-(A)$_{n2}$}, and for each quasi-repeat unit X1 is independently selected from the group consisting of SGGQQ, GAGQQ, GQGPY, AGQQ, and SQ, and is from 4 to 8, and n2 is from 6 to 10.

As further described below, one example of a copolymer molecule includes three "long" quasi-repeats followed by three "short" quasi-repeat units. A "long" quasi-repeat unit is comprised of quasi-repeat units that do not use the same $X_1$ constituent (as shown in Equation 2) more than twice in a row, or more than two times in a repeat unit. Each "short" quasi-repeat unit includes any of the amino acid sequences identified in Equation 2, but regardless of the amino acid sequences used, the same sequences are in the same location within the molecule. Furthermore, in this example copolymer molecule, no more than 3 quasi-repeats out of 6 share the same $X_1$. "Short" quasi-repeat units are those in which n1=4 or 5 (as shown in Equation 1). Long quasi-repeat units are defined as those in which n1=6, 7 or 8 (as shown in Equation 1).

In some embodiments, the repeat unit of the copolymer is composed of $X_{qr}$ quasi-repeat units, where $X_{qr}$ is a number from 2 to 20, and the number of short quasi-repeat units is $X_{sqr}$ and the number of long quasi-repeat units is $X_{lqr}$, where

$$X_{sqr} + X_{lqr} = X_{qr} \qquad \text{(Equation 3)}$$

and $X_{sqr}$ is a number from 1 to ($X_{qr}$-1) and $X_{lqr}$ is a number from 1 to ($X_{qr}$-1).

In another embodiment, is from 4 to 5 for at least half of the quasi-repeat units. In yet another embodiment, n2 is from 5 to 8 for at least half of the quasi-repeat units.

One feature of copolymer molecules of the present disclosure is the formation of nano-crystalline regions that, while not wishing to be bound by theory, are believed to form from the stacking of beta-sheet regions, and amorphous regions composed of alpha-helix structures, beta-turn structures, or both. Poly-alanine regions (or in some species (GA)$_n$ regions) in a molecule form crystalline beta-sheets within major ampullate (MA) fibers. Other regions within a repeat unit of major ampullate and flagelliform spider silks (for example containing GPGGX, GPGQQ, GGX where X = A, S or Y, GPG, SGGQQ, GAGQQ, GQGPY, AGQQ, and SQ, may form amorphous rubber-like structures that include alpha-helices and beta-turn containing structures. Furthermore, secondary, tertiary and quaternary structure is imparted to the morphology of the fibers via amino acid sequence and length, as well as the conditions by which the fibers are formed, processed and post-processed. Materials characterization techniques (such as NMR, FTIR and x-ray diffraction) have suggested that the poly-alanine crystalline domains within natural MA spider silks and recombinant silk derived from MA spider silk sequences are typically very small (<10 nm). Fibers can be highly crystalline or highly amorphous, or a blend of both crystalline and amorphous regions, but fibers with optimal mechanical properties have been speculated to be composed of 10~40% crystalline material by volume. In some embodiments, the repeat unit of a polypeptide described in this disclosure has at least 80%, or at least 90%, or at least 95%, or at least 99% sequence identity to a MA dragline silk protein sequence. In some embodiments, the repeat unit of a polypeptide described in this disclosure has at least 80%, or at least 90%, or at least 95%, or at least 99% sequence identity to a MaSp2 dragline silk protein sequence. In some embodiments, the repeat unit of a polypeptide described in this disclosure has at least 80%, or at least 90%, or at least 95%, or at least 99% sequence identity to a spider dragline silk protein sequence. In some embodiments, a quasi-repeat unit of a polypeptide described in this disclosure has at least 80%, or at least 90%, or at least 95%, or at least 99% sequence identity to a MA dragline silk protein sequence. In some embodiments, a quasi-repeat unit of a polypeptide described in this disclosure has at least 80%, or at least 90%, or at least 95%, or at least 99% sequence identity to a MaSp2 dragline silk protein sequence. In some embodiments, a quasi-repeat unit of a polypeptide described in this disclosure has at least 80%, or at least 90%, or at least 95%, or at least 99% sequence identity to a spider dragline silk protein sequence.

While not wishing to be bound by theory, the structural properties of the proteins within the spider silk are theorized to be related to fiber mechanical properties. Crystalline regions in a fiber have been linked with the tensile strength of a fiber, while the amorphous regions have been linked to the extensibility of a fiber. The major ampullate (MA) silks tend to have higher strengths and less extensibility than the flagelliform silks, and likewise the MA silks have higher volume fraction of crystalline regions compared with flagelliform silks. Furthermore, theoretical models based on the molecular dynamics of crystalline and amorphous regions of spider silk proteins, support the assertion that the crystalline regions have been linked with the tensile strength of a fiber, while the amorphous regions have been linked to the extensibility of a fiber. Additionally, the theoretical modeling supports the importance of the secondary, tertiary and quaternary structure on the mechanical properties of recombinant protein fibers. For instance, both the assembly of nano-crystal domains in a random, parallel and serial spatial distributions, and the strength of the interaction forces between entangled chains within the amorphous regions, and between the amorphous regions and the nano-crystalline regions, influenced the theoretical mechanical properties of the resulting fibers.

The repeat unit of the proteinaceous block copolymer that forms fibers with good mechanical properties can be synthesized using a portion of a silk polypeptide. Some exemplary sequences that can be used as repeats in the proteinaceous block copolymers of this disclosure are shown in Table 1. These polypeptide repeat units contain alanine-rich regions and glycine-rich regions, and are 150 amino acids in length or longer. These exemplary sequences were demonstrated to express using a *Pichia* expression system as taught in co-owned PCT Publication WO 2015042164.

Table 1: Exemplary sequences that can be used as repeat units

| Seq. ID No. | AA |
|---|---|
| 1 | GGYGPGAGQQGPGSGGQQGPGGQGPYGSGQQGPGGAGQQGPGGQGPYGPGAAAAAAAA AGGYGPGAGQQGPGGAGQQGPGSQGPGGQGPYGPGAGQQGPGSQGPGSGGQQGPGGQ GPYGPSAAAAAAAAAGGYGPGAGQRSQGPGGQGPYGPGAGQQGPGSQGPGSGGQQGPGG QGPYGPSAAAAAAAAGGYGPGAGQQGPGSQGPGSGGQQGPGGQGPYGPGAAAAAAAVGG YGPGAGQQGPGSQGPGSGGQQGPGGQGPYGPSAAAAAAAAGGYGPGAGQQGPGSQGPGS GGQQGPGGQGPYGPSAAAAAAAA |
| 2 | GGQGGRGGFGGLGSQGAGGAGQGGAGAAAAAAAAGGDGGSGLGGYGAGRGHGVGLGGA GGAGAASAAAAAGGQGGRGGFGGLGSQGAGGAGQGGAGAAAAAAAAGGDGGSGLGGYG AGRGHGAGLGGAGGAGAASAAAAAGGQGGRGGFGGLGSQGSGGAGQGGSGAAAAAAAA GGDGGSGLGGYGAGRGYGAGLGGAGGAGAASAAAAAGGQGGRGGFGGLGSQGAGGAGQ GGSGAAAAAAAAVADGGSGLGGYGAGRGYGAGLGGAGGAGAASAAAAT |
| 3 | GSAPQGAGGPAPQGPSQQGPVSQGPYGPGAAAAAAAAGGYGPGAGQQGPGSQGPGSGGQ QGPGSQGPGSGGQQGPGGQGPYGPSAAAAAAAAAGGYGPGAGQQGPGSQGPGSGGQQG PGGQGPYGPGAAAAAAAVGGYGPGAGQQGPGSQGPGSGGQQGPGGQGPYGPSAAAAAAAA AGGYGPGAGQQGPGSQGPGSGGQQGPGGQGPYGPSAAAAAAAAGGYGPGAGQQGPGSG GQQGPGGQGPYGSGQQGPGGAGQQGPGGQGPYGPGAAAAAAAAA |

| Seq. ID No. | AA |
|---|---|
| 4 | GGYGPGAGQQGPGSGGQQGPGGQGPYGSGQQGPGGAGQQGPGGQGPYGPGAAAAAAAA AGGYGPGAGQQGPGGAGQQGPGSQGPGGQGPYGPGAGQQGPGSQGPGSGGQQGPGGQ GPYGPSAAAAAAAAGGYGPGAGQQGPGSQGPGSGGQQGPGGQGPYGPSAAAAAAAAGGY GPGAGQQGPGSGGQQGPGGQGPYGSGQQGPGGAGQQGPGGQGPYGGGYGPGAGQQGP GSQGPGSGGQQGPGGQGPYGPSAAAAAAAA |
| 5 | GPGARRQGPGSQGPGSGGQQGPGGQGPYGSGQQGPGGAGQQGPGGQGPYGPGAAAAAA AAAGGYGPGAGQQGPGGAGQQGPGSQGPGGQGPYGPGAGQQGPGSQGPGSGGQQGPG GQGPYGPSAAAAAAAAAGGYGPGAGQQGPGSQGPGSGGQQGPGGQGPYGPGAAAAAAAV GGYGPGAGQQGPGSQGPGSGGQQGPGGQGPYGPSAAAAAAAAGGYGPGAGQQGPGSQG PGSGGQQGPGGQGPYGPSAAAAAAAA |
| 6 | GPGARRQGPGSQGPGSGGQQGPGGQGPYGSGQQGPGGAGQQGPGGQGPYGPGAAAAAA AAAGGYGPGAGQQGPGGAGQQGPGSQGPGGQGPYGPGAGQQGPGSQGPGSGGQQGPG GQGPYGPSAAAAAAAAGGYGPGAGQQGPGSQGPGSGGQQGPGGQGPYGPGAAAAAAAVG GYGPGAGQQGPGSQGPGSGGQQGPGGQGPYGPSAAAAAAAAGGYGPGAGQQGPGSQGP GSGGQQGPGGQGPYGPSAAAAAAAA |
| 7 | GGYGPGAGQQGPGSGGQQGPGGQGPYGSGQQGPGGAGQQGPGGQGPYGPGAAAAAAAA AGGYGPGAGQQGPGGAGQQGPEGPGSQGPGSGGQQGPGGQGPYGPGAAAAAAAVGGYG PGAGQQGPGSQGPGSGGQQGPGGQGPYGPSAAAAAAAAGGYGPGAGQQGPGSQGPGSG GQQGPGGQGPYGPSAAAAAAAAGGYGPGAGQQGPGSGGQQGPGGQGPYGSGQQGPGGA GQQGPGGQGPYGPGAAAAAAAA |

(continued)

| Seq. ID No. | AA |
|---|---|
| 8 | GVFSAGQGATPWENSQLAESFISRFLRFIGQSGAFSPNQLDDMSSIGDTLKTAIEKMAQSRKSSK SKLQALNMAFASSMAEIAVAEQGGLSLEAKTNAIASALSAAFLETTGYVNQQFVNEIKTLIFMIA QASSNEISGSAAAAGGSSGGGGGSGQGGYGQGAYASASAAAAYGSAPQGTGGPASQGPSQQ GPVSQPSYGPSATVAVTAVGGRPQGPSAPRQQGPSQQGPGQQGPGGRGPYGPSAAAAAAA A |
| 9 | GAGAGAGAGAGAGAGSGASTSVSTSSSSGSGAGAGAGSGAGSGAGAGSGAGAGAGG AGAGFGSGLGLGYGVGLSSAQAQAQAQAAAQAQAQAQAQAYAAAQAQAQAQAQAAA AAAAAAAGAGAGAGAGAGAGAGSGASTSVSTSSSSGSGAGAGAGSGAGSGAGAGSGA GAGAGAGGAGAGFGSGLGLGYGVGLSSAQAQAQAQAAAQAQAQAQAYAAAQAQAQA QAQAQAAAAAAAAAAA |
| 10 | GAGAGAGAGAGAGAGSGASTSVSTSSSSGSGAGAGAGSGAGSGAGAGSGAGAGAGG AGAAFGSGLGLGYGVGLSSAQAQAQAQAAAQAQADAQAQAYAAAQAQAQAQAQAAAA AAAAAAGAGAGAGAGSGAGAGAGSGASTSVSTSSSSGSGAGAGAGSGAGSGAGAGSGAGA GAGAGGAGAGFGSGLGLGYGVGLSSAQAQAQAQAAAQAQADAQAQAYAAAQAQAQAQA QAQAAAAAAAAAAA |
| 11 | GAGAGAGSGAGAGAGSGASTSVSTSSSSGSGAGAGAGSGAGSGAGAGSGAGAGAGG AGAGFGSGLGLGYGVGLSSAQAQAQSAAAARAQADAQAQAYAAAQAQAQAQAQAAAA AAAAAAAGAGAGAGAGAGAGAGSGASTSVSTSSSSASGAGAGAGSGAGSGAGAGSGAGA GAGAGGAGAGFGSGLGLGYGVGLSSAQAQAQAQAAAQAQAQAQAQALAAAQAQAQAQA QAQAAAATAAAAAA |

(continued)

| Seq. ID No. | AA |
|---|---|
| 12 | GGYGPGAGQQGPGGAGQQGPGSQGPGGQGPYGPGAGQQGPGSQGPGSGGQQGPGGQG PYGPSAAAAAAAAGGYGPGAGQQGPGSQGPGSGGQQGPGSQGPGSGGQQGPGGQGPYGP SAAAAAAAAAGGYGPGAGQQGPGSQGPGSGGQQGPGGQGPYGPGAAAAAAAVGGYGPGA GQQGPGSQGPGSGGQQGPGGQGPYGPSAAAAAAAAGGYGPGAGQQGPGSQGPGSGGQQ GPGGQGPYGPSAAAAAAAA |
| 13 | GGYGPGAGQQGPGGAGQQGPGSQGPGGQGPYGPGAGQQGPGSQGPGSGGQQGPGGQG PYGPSAAAAAAAAGGYGPGAGQQGPGSQGPGSGGQQGPGSQGPGSGGQQGPGGQGPYGP SAAAAAAAAGGYGPGAGQQGPGSQGPGSGGQQGPGGQGPYGPGAAAAAAAVGGYGPGAG QQGPGSQGPGSGGQQGPGGQGPYGPSAAAAAAAAGGYGPGAGQQGPGSQGPGSGGQQG PGGQGPYGPSAAAAAAAA |
| 14 | GHQGPHRKTPWETPEMAENFMNNVRENLEASRIFPDELMKDMEAITNTMIAAVDGLEAQHR SSYASLQAMNTAFASSMAQLFATEQDYVDTEVIAGAIGKAYQQITGYENPHLASEVTRLIQLFRE EDDLENEVEISFADTDNAIARAAAGAAAGSAAASSSADASATAEGASGDSGFLFSTGTFGRGGA GAGAGAAAASAAAASAAAAGAEGDRGLFFSTGDFGRGGAGAGAGAAAASAAAASAAAA |
| 15 | GGAQKHPSGEYSVATASAAATSVTSGGAPVGKPGVPAPIFYPQGPLQQGPAPGPSNVQPGTSQ QGPIGGVGESNTFSSSFASALGGNRGFSGVISSASATAVASAFQKGLAPYGTAFALSAASAAADA YNSIGSGASASAYAQAFARVLYPLLQQYGLSSSADASAFASAIASSFSTGVAGQGPSVPYVGQQQ PSIMVSAASASAAASAAAVGGGPVVQGPYDGGQPQQPNIAASAAAAATATSS |

(continued)

| Seq. ID No. | AA |
|---|---|
| 16 | GGQGGRGGFGGLGSQGEGGAGQGGAGAAAAAAAAGADGGFGLGGYGAGRGYGAGLGGAGGAGAASAAAAAGGQGGRSGFGGLGSQGAGGAGQGGAGAAAAAAAAGADGGSGLGGYGAGRGYGASLGGADGAGAASAAAAAGGQGGRGGFGGLGSQGAGGAGQGGAGAAAAAAAASGDGGSGLGGYGAGRGYGAGLGGAGGAGAASAAAAAGGEGGRGGFGGLGSQGAGGAGQGGSLAAAAAAAA |
| 17 | GPGGYGGPGQPGPGQGQYGPGPGQQGPRQGGQQGPASAAAAAAAGPGGYGGPGQQGPRQGQQQGPASAAAAAAAAAGPRGYGGPGQQGPVQGGQQGPASAAAAAAAAGVGGYGGPGQQGPGQGQYGPGTGQQGQGPSGQQGPAGAAAAAAGGAAGPGGYGGPGQQGPGQGQYGPGTGQQGQGPSGQQGPAGAAAAAAAAAGPGGYGGPGQQGPGQGQYGPGAGQQGQGPGSQQGPASAAAAAA |
| 18 | GSGAGQGTGAGAGAAAAAGAAGSGAGQGAGSGAGAAAAAAAASAAGAGQGAGSGSGAGAAAAAAAAAGAGQGAGSGSGAGAAAAAAAAAAAAQQQQQQQAAAAAAAAAAAAAAAGSGQGASFGVTQQFGAPSGAASSAAAAAAAAAAAAAAGSGAGQEAGTGAGAAAAAAAAGAAGSGAGQGAGSGAGAAAAAAAAASAAGAGQGAGSGSGAGAAAAAAAAAAAAQQQQQQQAAAAAAAAAAA |
| 19 | GGAQKQPSGESSVATASAAATSVTSAGAPVGKPGVPAPIFYPQGPLQQGPAPGPSYVQPATSQQGPIGGAGRSNAFSSSFASALSGNRGFSEVISSASATAVASAFQKGLAPYGTAFALSAASAAADAYNSIGSGANAFAYAQAFARVLYPLVQQYGLSSSAKASAFASAIASSFSSGAAGQGQSIPYGGQQQPPMTISAASASAGASAAAVKGGQVGQGPYGGQQQSTAASASAAATTATA |

(continued)

| Seq. ID No. | AA |
|---|---|
| 20 | GADGGSGLGGYGAGRGYGAGLGGADGAGAASAAAAAGGQGGRGGFGRLGSQGAGGAGQG GAGAAAAVAAAGGDGGSGLGGYGAGRGYGAGLGGAGGAGAASAAAAAGGQGGRGGFGGL GSQGAGGAGQGGAGAAASGDGGSGLGGYGAGRGYGAGLGGADGAGAASAASAAGGQGGR GGFGGLGSQGAGGAGQGGAGAAAAATAGGDGGSGLGGYGAGRGYGAGLGGAGGAGAAS AAAAA |
| 21 | GAGAGQGGRGGYGQGGFGGQGSGAGAGASAAAGAGAGQGGRGGYGQGGFGGQGSGAG AGASAAAGAGAGQGGRGGYGQGGFGGQGSGAGAGASAAAAAGAGQGGRGGYGQGGLGG SGSGAGAGAGAAAAAAAGAGGYGQGGLGGYGQGAGAGQGGLGGYGSGAGAGASAAAAAG AGGAGQGGLGGYGQGAGAGQGGLGGYGSGAGAGAAAAAAAGAGGSGQGGLGGYGSGGG AGGASAAAA |
| 22 | GAYAYAYAIANAFASILANTGLLSVSSAASVASSVASAIATSVSSSSAAAAASASAAAAASAGASA ASSASASSSASAAAGAGAGAGAGASGASGAAGGSGGFGLSSGFGAGIGGLGGYPSGALGGLGI PSGLLSSGLLSPAANQRIASLIPLILSAISPNGVNFGVIGSNIASLASQISQSGGGIAASQAFTQALLE LVAAFIQVLSSAQIGAVSSSSASAGATANAFAQSLSSAFAG |
| 23 | GAAQKQPSGESSVATASAAATSVTSGGAPVGKPGVPAPIFYPQGPLQQGPAPGPSNVQPGTSQ QGPIGGVGGSNAFSSSFASALSLNRGFTEVISSASATAVASAFQKGLAPYGTAFALSAASAAADA YNSIGSGANAFAYAQAFARVLYPLVRQYGLSSSGKASAFASAIASSFSSGTSGQGPSIGQQQPPV TISAASASAGASAAAVGGGQVGQGPYGGQQQSTAASASAAAATATS |

(continued)

| Seq. ID No. | AA |
|---|---|
| 24 | GAAQKQPSGESSVATASAAATSVTSGGAPVGKPGVPAPIFYPQGPLQQGPAPGPSNVQPGTSQ QGPIGGVGGSNAFSSSFASALSLNRGFTEVISSASATAVASAFQKGLAPYGTAFALSAASAAADA YNSIGSGANAFAYAQAFARVLYPLVRQYGLSSSGKASAFASAIASSFSSGTSGQGPSIGQQQPPV TISAASASAGASAAAVGGGQVGQGPYGGQQQSTAASASAAAATATS |
| 25 | GAAQKQPSGESSVATASAAATSVTSGGAPVGKPGVPAPIFYPQGPLQQGPAPGPSNVQPGTSQ QGPIGGVGGSNAFSSSFASALSLNRGFTEVISSASATAVASAFQKGLAPYGTAFALSAASAAADA YNSIGSGANAFAYAQAFARVLYPLVQQYGLSSSAKASAFASAIASSFSSGTSGQGPSIGQQQPPV TISAASASAGASAAAVGGGQVGQGPYGGQQQSTAASASAAAATATS |
| 26 | GGAQKQPSGESSVATASAAATSVTSAGAPVGKPGVPAPIFYPQGPLQQGPAPGPSNVQPGTSQ QGPIGGVGGSNAFSSSFASALSLNRGFTEVISSASATAVASAFQKGLAPYGTAFALSAASAAADA YNSIGSGANAFAYAQAFARVLYPLVQQYGLSSSAKASAFASAIASSFSSGTSGQGPSNGQQQPP VTISAASASAGASAAAVGGGQVSQGPYGGQQQSTAASASAAAATATS |
| 27 | GGAQKQPSGESSVATASAAATSVTSAGAPGGKPGVPAPIFYPQGPLQQGPAPGPSNVQPGTS QQGPIGGVGGSNAFSSSFASALSLNRGFTEVISSASATAVASAFQKGLAPYGTAFALSAASAAAD AYNSIGSGANAFAYAQAFARVLYPLVQQYGLSSSAKASAFASAIASSFSSGTSGQGPSIGQQQPP VTISAASASAGASAAAVGGGQVGQGPYGGQQQSTAASASAAAATATS |

(continued)

| Seq. ID No. | AA |
|---|---|
| 28 | GPGGYGGPGQQGPGQGQQQGPASAAAAAAAAGPGGYGGPGQQGPGQGQQQGPASAAA AAAAAAGPGGYGGPGQQRPGQAQYGRGTGQQGQGPGAQQGPASAAAAAAAGAGLYGGP GQQGPGQGQQQGPASAAAAAAAAAAGPGGYGGPGQQGPGQAQQQGPASAAAAAAAGP GGYSGPGQQGPGQAQQQGPASAAAAAAAAAGPGGYGGPGQQGPGQGQQQGPASAAAA AATAA |
| 29 | GAGGDGGLFLSSGDFGRGGAGAGAGAAAASAAAASSAAAGARGGSGFGVGTGGFGRGGAG DGASAAAASAAAASAAAAGAGGDSGLFLSSGDFGRGGAGAGAGAAAASAAAASAAAAGTGG VGGLFLSSGDFGRGGAGAGAGAAAASAAAASSAAAGARGGSGFGVGTGGFGRGGPGAGTGA AAASAAAASAAAAGAGGDSGLFLSSEDFGRGGAGAGTGAAAASAAAASAAAA |
| 30 | GAGRGYGGGYGGGAAAGAGAGAGAGRGYGGGYGGGAGSGAGSGAGAGGGSGYGRGAGA GAGAGAAAAAGAGAGGAGGYGGGAGAGAGASAAAGAGAGAGGAGGYGGGYGGGAGAGA GAGAAAAAGAGAGAGAGRGYGGGFGGGAGSGAGAGAGAGGGSGYGRGAGGYGGGYGGG AGTGAGAAAATGAGAGAGAGRGYGGGYGGGAGAGAGAGAGAGGGSGYGRGAGAGASVA A |
| 31 | GALGQGASVWSSPQMAENFMNGFSMALSQAGAFSGQEMKDFDDVRDIMNSAMDKMIRSG KSGRGAMRAMNAAFGSAIAEIVAANGGKEYQIGAVLDAVTNTLLQLTGNADNGFLNEISRLITL FSSVEANDVSASAGADASGSSGPVGGYSSGAGAAVGQGTAQAVGYGGGAQGVASSAAAGAT NYAQGVSTGSTQNVATSTVTTTTNVAGSTATGYNTGYGIGAAAGAAA |

| Seq. ID No. | AA |
|---|---|
| 32 | GGQGGQGGYDGLGSQGAGQGGYGQGGAAAAAAAASGAGSAQRGGLGAGGAGQGYGAGS GGQGGAGQGGAAAATAAAAGGQGGQGGYGGLGSQGSGQGGYGQGGAAAAAAAASGDG GAGQEGLGAGGAGQGYGAGLGGQGGAGQGGAAAAAAAAAGGQGGQGGYGGLGSQGAG QGGYGQGGAAAAAAAASGAGGAGQGGLGAAGAGQGYGAGSGGQGGAGQGGAAAAAAA A |
| 33 | GGQGGQGGYGGLGSQGAGQGGYGQGGVAAAAAAASGAGGAGRGGLGAGGAGQEYGAVS GGQGGAGQGGEAAAAAAAAGGQGGQGGYGGLGSQGAGQGGYGQGGAAAAAAAASGAG GARRGGLGAGGAGQGYGAGLGGQGGAGQGSASAAAAAAAGGQGGQGGYGGLGSQGSGQ GGYGQGGAAAAAAAASGAGGAGRGSLGAGGAGQGYGAGLGGQGGAGQGGAAAAASAAA |
| 34 | GPGGYGGPGQQGPGQGQYGPGTGQQGQGPGGQQGPVGAAAAAAAAVSSGGYGSQGAGQ GGQQGSGQRGPAAAGPGGYSGPGQQGPGQGGQQGPASAAAAAAAAAAGPGGYGGSGQQG PGQGRGTGQQGQGPGGQQGPASAAAAAAAGPGGYGGPGQQGPGQGQYGPGTGQQGQG PASAAAAAAAGPGGYGGPGQQGPGQGQYGPGTGQQGQGPGGQQGPGGASAAAAAAA |
| 35 | GGYGPGAGQQGPGSGGQQGPGGQGPYGSGQQGPGGAGQQGPGGQGPYGPGAAAAAAAAA AGGYGPGAGQQGPGGAGQQGPGSQGPGGQGPYGPGAGQQGPGSQGPGSGGQQGPGGQ GPYGPSAAAAAAAAAGGYGPGAGQRSQGPGGQGPYGPGAGQQGPGSQGPGSGGQQGPGG QGPYGPSAAAAAAAAGPGAGRQGPGSQGPGSGGQQGPGGQGPYGPSAAAAAAAA |

(continued)

| Seq. ID No. | AA |
|---|---|
| 36 | GQGGQGGQGGLGQGGYGQGAGSSAAAAAAAAAAAAAAAGRGQGGYGQGSGGNAAAAAAA AAAAASGQGSQGGQGGQGQGGYGQGAGSSAAAAAAAAAAAAASGRGQGGYGQGAGGNA AAAAAAAAAAAAAGQGGQGGYGGLGQGGYGQGAGSSAAAAAAAAAAAAGGQGGQGQGG YGQGSGGSAAAAAAAAAAAAAAAGRGQGGYGQGSGGNAAAAAAAAAAAAAA |
| 37 | GRGPGGYGPGQQGPGGPGAAAAAAGPGGYGPGGYGPGQQGPGGPGAAAAAAGRGPGG YGPGQQGPGQQGPGGSGAAAAAAGRGPGGYGPGQQGPGGPGAAAAAAGPGGYGPGQQG PGAAAAAAAAGRGPGGYGPGQQGPGGPGAAAAAAGRGPGGYGPGQQGPGQQGPGGSG AAAAAAGRGPGGYGPGQQGPGGPGAAAAAAGPGGYGPGQQGPGAAAAAAAA |
| 38 | GRGPGGYGPGQQGPGGSGAAAAAAGRGPGGYGPGQQGPGGPGAAAAAAGPGGYGPGQQ GTGAAAAAAAGSGAGGYGPGQQGPGGPGAAAAAAGPGGYGPGQQGPGAAAAAAAGSGPG GYGPGQQGPGGSSAAAAAAGPGRYGPGQQGPGAAAAASAGRGPGGYGPGQQGPGGPGAA AAAAGPGGYGPGQQGPGAAAAAAAGSGPGGYGPGQQGPGGPGAAAAAAA |
| 39 | GAAATAGAGASVAGGYGGGAGAAAGAGAGGYGGGYGAVAGSGAGAAAAASSGAGGAAGY GRGYGAGSGAGAGAGTVAAYGGAGGVATSSSSATASGSRIVTSGGYGYGTSAAAGAGVAAGS YAGAVNRLSSAEAASRVSSNIAAIASGGASALPSVISNIYSGVVASGVSSNEALIQALLELLSALVHV LSSASIGNVSSVGVDSTLNVVQDSVGQYVG |
| 40 | GGQGGFSGQGQGGFGPGAGSSAAAAAAAAAAAARQGGQGQGGFGQGAGGNAAAAAAAAA AAAAAQQGGQGGFSGRGQGGFGPGAGSSAAAAAAGQGGQGQGGFGQGAGGNAAAAAAA AAAAAAAAGQGGQGRGGFGQGAGGNAAAAAAAAAAAAAAAAQQGGQGGFGGRGQGGFG PGAGSSAAAAAAGQGGQGRGGFGQGAGGNAAAASAAAAASAAAAGQ |

(continued)

| Seq. ID No. | AA |
| --- | --- |
| 41 | GGYGPGAGQQGPGGAGQQGPGSQGPGGAGQQGPGGQGPYGPGAAAAAAAVGGYGPGAG QQGPGSQGPGSGGQQGPGGQGPYGPSAAAAAAAAGGYGPGAGQQGPGSQGPGSGGQQG PGGLGPYGPSAAAAAAAAGGYGPGAGQQGPGSQGPGSGGQQRPGGLGPYGPSAAAAAAA GGYGPGAGQQGPGSQGPGSGGQQRPGGLGPYGPSAAAAAAA |
| 42 | GAGAGGGYGGGYSAGGGAGAGSGAAAGAGAGRGGAGGYSAGAGTGAGAAAGAGTAGGYS GGYGAGASSSAGSSFISSSSMSSSQATGYSSSSGYGGGAASAAAGAGAAAGGYGGGYGAGAG AGAAAASGATGRVANSLGAMASGGINALPGVFSNIFSQVSAASGGASGGAVLVQALTEVIALLL HILSSASIGNVSSQGLEGSMAIAQQAIGAYAG |
| 43 | GAGAGGAGGYAQGYGAGAGAGAGAGTGAGGAGGYGQGYGAGSGAGAGGAGGYGAGAGA GAGAGDASGYGQGYGDGAGAGAGAAAAAGAAAGARGAGGYGGGAGAGAGAGAGAAGGY GQGYGAGAGEGAGAGAGAGAVAGAGAAAAAGAGAGAGGAEGYGAGAGAGGAGGYGQSY GDGAAAAAGSGAGAGGSGGYGAGAGAGSGAGAAGGYGGGAGA |
| 44 | GPGGYGPGQQGPGGYGPGQQGPGRYGPGQQGPSGPGSAAAAAAGSGQQGPGGYGPRQQ GPGGYGQGQQGPSGPGSAAAASAAASAESGQQGPGGYGPGQQGPGGYGPGQQGPGGYGP GQQGPSGPGSAAAAAAAASGPGQQGPGGYGPGQQGPGGYGPGQQGPSGPGSAAAAAAAA SGPGQQGPGGYGPGQQGPGGYGPGQQGLSGPGSAAAAAAA |
| 45 | GRGPGGYGQGQQGPGGPGAAAAAAGPGGYGPGQQGPGAAAAAAAGSGPGGYGPGQQGP GRSGAAAAAAAAGRGPGGYGPGQQGPGGPGAAAAAAGPGGYGPGQQGPGAAAAASAGRG PGGYGPGQQGPGGSGAAAAAAGRGPGGYGPGQQGPGGPGAAAAAAAGRGPGGYGPGQQ GPGQQGPGGSGAAAAAAGRGPGGYGPGQQGPGGPGAAAAAA |

(continued)

| Seq. ID No. | AA |
|---|---|
| 46 | GVGAGGEGGYDQGYGAGAGAGSGGGAGGAGGYGGGAGAGSGGGAGGAGGYGGGAGAG AGAGAGGAGGYGGGAGAGTGARAGAGGVGGYGQSYGAGASAAAGAGVGAGGAGAGGAG GYGQGYGAGAGIGAGDAGGYGGGAGAGASAGAGGYGGGAGAGAGGVGGYGKGYGAGSGA GAAAAAGAGAGSAGGYGRGDGAGAGGASGYGQGYGAGAAA |
| 47 | GYGAGAGRGYGAGAGAGAGAVAASGAGAGAGYGAGAGAGAGAGYGAGAGRGYGAGAGA GAGSGAASGAGAGAGYGAGAGAGAGYGAGAGSGYGTGAGAGAGAAAAGGAGAGAGYGAG AGRGYGAGAGAGAASGAGAGAGAGAASGAGAGSGYGAGAAAAGGAGAGAGGGYGAGAG RGYGAGAGAGAGSGSGSAAGYGQGYGSGSGAGAAA |
| 48 | GQGTDSSASSVSTSTSVSSSATGPDTGYPVGYYGAGQAEAAASAAAAAAASAAEAATIAGLGYG RQGQGTDSSASSVSTSTSVSSSATGPDMGYPVGNYGAGQAEAAASAAAAAAASAAEAATIASL GYGRQGQGTDSSASSVSTSTSVSSSATGPGSRYPVRDYGADQAEAAASAAAAAAAAASAAEEIA SLGYGRQ |
| 49 | GQGTDSVASSASSSASASSSATGPDTGYPVGYYGAGQAEAAASAAAAAAASAAEAATIAGLGY GRQGQGTDSSASSVSTSTSVSSSATGPGSRYPVRDYGADQAEAAASATAAAAAAASAAEEIASL GYGRQGQGTDSVASSASSSASASSSATGPDTGYPVGYYGAGQAEAAASAAAAAAASAAEAATI AGLGYGRQ |
| 50 | GQGGQGGYGGLGQGGYGQGAGSSAAAAAAAAAAAAAGGQGGQGQGRYGQGAGSSAAAA AAAAAAAAGRGQGGYGQGSGGNAAAAAAAAAAAAASGQGSQGGQGGQGQGGYGQGA GSSAAAAAAAAAAAAASGRGQGGYGQGAGGNAAAAAAAAAAAAAAGQGGQGGYGGLGQ GGYGQGAGSSAAAAAAAAAAA |

| Seq. ID No. | AA |
|---|---|
| 51 | GGLGGQGGLGGLGSQGAGLGGYGQGGAGQGGAAAAAAAAGGLGGQGGRGGLGSQGAGQ GGYGQGGAGQGGAAAAAAAAGGLGGQGGLGALGSQGAGQGGAGQGGYGQGGAAAAAA GGLGGQGGLGGLGSQGAGQGGYGQGGAGQGGAAAAAAAAGGLGGQGGLGGLGSQGAGP GGYGQGGAGQGGAAAAAAAA |
| 52 | GGQGRGGFGQGAGGNAAAAAAAAAAAAAAAQQVGQFGFGGRGQGGFGPFAGSSAAAAAAAA SAAAGQGGQGQGGFGQGAGGNAAAAAAAAAAAAARQGGQGQGGFSQGAGGNAAAAAAAA AAAAAAAAQQGGQGGFGGRGQGGFGPGAGSSAAAAAAATAAAGQGGQGRGGFGQGAGS NAAAAAAAAAAAAAAAGQ |
| 53 | GGQGGQGGYGGLGSQGAGQGGYGAGQGAAAAAAAAGGAGGAGRGGLGAGGAGQGYGA GLGGQGGAGQAAAAAAAGGAGGARQGGLGAGGAGQGYGAGLGGQGGAGQGGAAAAAA AAGGQGGQGGYGGLGSQGAGQGGYGAGQGGAAAAAAAAGGQGGQGGYGGLGSQGAGQ GGYGGRQGGAGAAAAAAAA |
| 54 | GGAGQRGYGGLGNQGAGRGGLGGQGAGAAAAAAAAGGAGQGGYGGLGNQGAGRGGQGA AAAAGGAGQGGYGGLGSQGAGRGGQGAGAAAAAAVGAGQEGIRGQGAGQGGYGGLGSQ GSGRGGLGGQGAGAAAAAAGGAGQGGLGGQGAGQGAGAAAAAAGGVRQGGYGGLGSQG AGRGGQGAGAAAAAA |
| 55 | GGAGQGGLGGQGAGQGAGASAAAAGGAGQGGYGGLGSQGAGRGGEGAGAAAAAAAGGAG QGGYGGLGGQGAGQGGYGGLGSQGAGRGGLGGQGAGAAAAGGAGQGGLGGQGAGQGA GAAAAAAGGAGQGGYGGLGSQGAGRGGLGGQGAGAVAAAAGGAGQGGYGGLGSQGAG RGGQGAGAAAAAA |

(continued)

| Seq. ID No. | AA |
|---|---|
| 56 | GAGAGAGAGSGAGAAGGYGGGAGAGVGAGGAGGYDQGYGAGAGAGSGAGAGGAGGYGG GAGAGADAGAGGAGGYGGGAGAGAGARAGAGGVGGYGQSYGAGAGAGAGVGAGGAGA GGADGYGQGYGAGAGTGAGDAGGYGGGAGAGASAGAGGYGGGAGAGGVGVYGKGYGSG SGAGAAAAA |
| 57 | GGAGGYGVGQGYGAGAGAGAAAGAGAGGAGGYGAGQGYGAGAGVGAAAAAGAGAGVG GAGGYGRGAGAGAGAGAGAAAGAGAGAAAGAGAGGAGGYGAGQGYGAGAGVGAAAAAG AGAGVGGAGGYGRGAGAGAGAGAGGAGGYGRGAGAGAGAGAGGAGGYGAGQGYGA GAGAGAAAAA |
| 58 | GEAFSASSASSAVVFESAGPGEEAGSSGDGASAAASAAAAAGAGSGRRGPGGARSRGGAGAG AGAGSGVGGYGSGSGAGAGAGAGAGAGGEGGFGEGQGYGAGAGAGFGSGAGAGAGAGSG AGAGEGVGSGAGAGAGAGFGVGAGAGAGAGAGFGSGAGAGSGAGAGYGAGRAGGRGRGG RG |
| 59 | GEAFSASSASSAVVFESAGPGEEAGSSGGGASAAASAAAAAGAGSGRRGPGGARSRGGAGAG AGAGSGVGGYGSGSGAGAGAGAGAGAGGEGGFGEGQGYGAGAGAGFGSGAGAGAGAGSG AGAGEGVGSGAGAGAGAGFGVGAGAGAGAGAGFGSGAGAGSGAGAGYGAGRAGGRGRGG RG |
| 60 | GNGLGQALLANGVLNSGNYLQLANSLAYSFGSSLSQYSSSAAGASAAGAASGAAGAGAGAASS GGSSGSASSSTTTTTTTSTSAAAAAAAAAAAAASAAASTSASASASASASASASAFSQTFVQTVLQSA AFGSYFGGNLSLQSAQAAASAAAQAAAQQIGLGSYGYALANAVASAFASAGANA |

| Seq. ID No. | AA |
|---|---|
| 61 | GNGLGQALLANGVLNSGNYLQLANSLAYSFGSSLSQYSSSAAGASAAGAASGAAGAGAGAASS GGSSGSASSSTTTTTTTSTSAAAAAAAAAAAAASAAASTSASASASASASASASAFSQTFVQTVLQSA AFGSYFGGNLSLQSAQAAASAAAQAAAQQIGLGSYGYALANAVASAFASAGANA |
| 62 | GNGLGQALLANGVLNSGNYLQLANSLAYSFGSSLSQYSSSAAGASAAGAASGAAGAGAGAASS GGSSGSASSSTTTTTTTSTSAAAAAAAAAAAAASAAASTSASASASASASASASAFSQTFVQTVLQSA AFGSYFGGNLSLQSAQAAASAAAQAAAQQIGLGSYGYALANAVASAFASAGANA |
| 63 | GASGAGQGQGYGQQGQGGSSAAAAAAAAAAAAAAAAQGQGQGYGQQGQGSAAAAAAAAA AGASGAGQGQGYGQQGQGSAAAAAAAAAAGASGAGQGQGYGQQGQGGSSAAAAAAAAAA AAAAAAAQGQGYGQQGQGSAAAAAAAAAGASGAGQGQGYGQQGQGGSSAAAAAAAAAA AAAAAA |
| 64 | GRGQGGYGQGSGGNAAAAAAAGQGGFGGQEGNGQGAGSAAAAAAAAAAAAGGSGQGRY GGRGQGGYGQGAGAAASAAAAAAAAAAGQGGFGGQEGNGQGAGSAAAAAAAAAAAAGG SGQGGYGGRGQGGYGQGAGAAAAAAAAAAAAAAGQGGQGGFGSQGGNGQGAGSAAAAA AAAAA |
| 65 | GQNTPWSSTELADAFINAFMNEAGRTGAFTADQLDDMSTIGDTIKTAMDKMARSNKSSKGKL QALNMAFASSMAEIAAVEQGGLSVDAKTNAIADSLNSAFYQTTGAANPQFVNEIRSLINMFAQ SSANEVSYGGGYGGQSAGAAASAAAAGGGGQGGYGNLGGQGAGAAAAAAASAA |
| 66 | GQNTPWSSTELADAFINAFLNEAGRTGAFTADQLDDMSTIGDTLKTAMDKMARSNKSSQSKL QALNMAFASSMAEIAAVEQGGLSVAEKTNAIADSLNSAFYQTTGAVNVQFVNEIRSLISMFAQA SANEVSYGGGYGGGQGGQSAGAAAAAASAGAGQGGYGGLGGQGAGSAAAAAA |

(continued)

| Seq. ID No. | AA |
| --- | --- |
| 67 | GGQGGQGGYGGLGSQGAGQGGYGQGGAAAAAASAGGQGGQGGYGGLGSQGAGQGGYG GGAFSGQQGGAASVATASAAASRLSSPGAASRVSSAVTSLVSSGGPTNSAALSNTISNVVSQISS SNPGLSGCDVLVQALLEIVSALVHILGSANIGQVNSSGVGRSASIVGQSINQAFS |
| 68 | GGAGQGGYGGLGGQGAGAAAAAAGGAGQGGYGGQGAGQGAAAAAASGAGQGGYEGPG AGQGAGAAAAAAGGAGQGGYGGLGGQGAGQGAGAAAAAAGGAGQGGYGGLGGQGAGQ GAGAAAAAAGGAGQGGYGGQGAGQGAAAAAAGGAGQGGYGGLGSGQGGYGRQGAGAA AAAAAA |
| 69 | GASSAAAAAAATATSGGAPGGYGGYGPGIGGAFVPASTTGTGSGSGSGAGAAGSGGLGGLGS SGGSGGLGGGNGGSGASAAASAAAASSSPGSGGYGPGQGVGSGSGSGAAGGSGTGSGAGGP GSGGYGGPQFFASAYGGQGLLGTSGYGNGQGGASGTGSGGVGGSGSGAGSNS |
| 70 | GQPIWTNPNAAMTMTNNLVQCASRSGVLTADQMDDMGMMADSVNSQMQKMGPNPPQ HRLRAMNTAMAAEVAEVVATSPPQSYSAVLNTIGACLRESMMQATGSVDNAFTNEVMQLVK MLSADSANEVSTASASGASYATSTSSAVSSSQATGYSTAAGYGNAAGAGAGAAAAVS |
| 71 | GQKIWTNPDAAMAMTNNLVQCAGRSGALTADQMDDLGMVSDSVNSQVRKMGANAPPHKI KAMSTAVAAGVAEVVASSPPQSYSAVLNTIGGCLRESMMQVTGSVDNTFTTEMMQMVNMF AADNANEVSASASGSGASYATGTSSAVSTSQATGYSTAGGYGTAAGAGAGAAAAA |
| 72 | GSGYGAGAGAGAGSGYGAGAGAGSGYGAGAGAGAGSGYVAGAGAGAGAGSGYGAGAGAG AGSSYSAGAGAGAGSGYGAGSSASAGSAVSTQTVSSSATTSSQSAAAATGAAYGTRASTGSGAS AGAAASGAGAGYGGQAGYGQGGGAAAYRAGAGSQAAYGQGASGSSGAAAAA |

| Seq. ID No. | AA |
|---|---|
| 73 | GGQGGRGGFGGLSSQGAGGAGQGGSGAAAAAAAAGGDGGSGLGDYGAGRGYGAGLGGAG GAGVASAAASAAASRLSSPSAASRVSSAVTSLISGGGPTNPAALSNTFSNVVYQISVSSPGLSGCD VLIQALLELVSALVHILGSAIIGQVNSSAAGESASLVGQSVYQAFS |
| 74 | GVGQAATPWENSQLAEDFINSFLRFIAQSGAFSPNQLDDMSSIGDTLKTAIEKMAQSRKSSKSKL QALNMAFASSMAEIAVAEQGGLSLEAKTNAIANALASAFLETTGFVNQQFVSEIKSLIYMIAQAS SNEISGSAAAAGGGSGGGGGSGQGGYGQGASASASAAAA |
| 75 | GGGDGYGQGGYGNQRGVGSYGQGAGAGAAATSAAGGAGSGRGGYGEQGGLGGYGQGAG AGAASTAAGGGDGYGQGGYGNQGGRGSYGQGSGAGAGAAVAAAAGGAVSGQGGYDGEG GQGGYGQGSGAGAAVAAASGGTGAGQGGYGSQGSQAGYGQGAGFRAAAATAAA |
| 76 | GAGAGYGGQVGYGQGAGASAGAAAAGAGAGYGGQAGYGQGAGGSAGAAAAGAGAGRQA GYGQGAGASARAAAAGAGTGYGQGAGASAGAAAAGAGAGSQVGYGQGAGASSGAAAAAG AGAGYGGQVGYEQGAGASAGAEAAASSAGAGYGGQAGYGQGAGASAGAAAA |
| 77 | GGAGQGGYGGLGGQGAGQGGLGGQRAGAAAAAAGGAGQGGYGGLGSQGAGRGGYGGVG SGASAASAAASRLSSPEASSRVSSAVSNLVSSGPTNSAALSSTISNVVSQISASNPGLSGCDVLVQ ALLEVVSALIQILGSSSIGQVNYGTAGQAAQIVGQSVYQALG |
| 78 | GGYGPGSGQQGPGGAGQQGPGGQGPYGPGSSSAAAVGGYGPSSGLQGPAGQGPYGPGAA ASAAAAAGASRLSSPQASSRVSSAVSSLVSSGPTNSAALTNTISSVVSQISASNPGLSGCDVLIQAL LEIVSALVHILGYSSIGQINYDAAAQYASLVGQSVAQALA |

| Seq. ID No. | AA |
|---|---|
| 79 | GGAGAGQGSYGGQGGYGQGGAGAATATAAAAGGAGSGQGGYGGQGGLGGYGQGAGAGA AAAAAAAAGGAGAGQGGYGGQGGQGGYGQGAGAGAAAAAGGAGAGQGGYGGQGGYG QGGGAGAAAAAAASGGSGSGQGGYGGQGGLGGYGQGAGAGAGAAASAAAA |
| 80 | GQGGQGGYGRQSQGAGSAAAAAAAAAAAAAAAGSGQGGYGGQGQGGYGQSSASASAAASA ASTVANSVSRLSSPSAVSRVSSAVSSLVSNGQVNMAALPNIISNISSSVSASAPGASGCEVIVQALL EVITALVQIVSSSSVGYINPSAVNQITNVVANAMAQVMG |
| 81 | GGAGQGGYGGLGGQGSGAAAAGTGQGGYGSLGGQGAGAAGAAAAAVGGAGQGGYGGVG SAAASAAASRLSSPEASSRVSSAVSNLVSSGPTNSAALSNTISNVVSQISSSNPGLSGCDVLVQALL EVVSALIHILGSSSIGQVNYGSAGQATQIVGQSVYQALG |
| 82 | GAGAGGAGGYGAGQGYGAGAGAGAAAGAGAGGARGYGARQGYGSGAGAGAGARAGGAG GYGRGAGAGAAAASGAGAGGYGAGQGYGAGAGAVASAAAGAGSGAGGAGGYGRGAGAVA GAGAGGAGGYGAGAGAAAGVGAGGSGGYGGRQGGYSAGAGAGAAAAA |
| 83 | GQGGQGGYGGLGQGGYGQGAGSSAAAAAAAAAAAGRGQGGYGQGSGGNAAAAAAAAAA AASGQGGQGGQGGQGQGGYGQGAGSSAAAAAAAAAAAAAAAGRGQGGYGQGAGGNAA AAAAAAAAASGQGGQGGQGGQGQGGYGQGAGSSAAAAAAAAAAAAAA |
| 84 | GGYGPGSGQQGPGQQGPGQQGPGQQGPYGAGASAAAAAAGGYGPGSGQQGPGVRVAAP VASAAASRLSSSAASSRVSSAVSSLVSSGPTTPAALSNTISSAVSQISASNPGLSGCDVLVQALLEV VSALVHILGSSSVGQINYGASAQYAQMVGQSVTQALV |

EP 3 307 765 B1

(continued)

| Seq. ID No. | AA |
|---|---|
| 85 | GAGAGGAGYGRGAGAGAGAAAGAGAGAAAGAGAGAGGYGGQGGYGAGAGAGAAAAAGA GAGGAAGYSRGGRAGAAGAGAGAAAGAGAGAGGYGGQGGYGAGAGAGAAAAAGAGSGG AGGYGRGAGAGAAAGAGAAAGAGAGAGGYGGQGGYGAGAGAAAAA |
| 86 | GAGAGRGGYGRGAGAGGYGGQGGYGAGAGAGAAAAAGAGAGGYGDKEIACWSRCRYTVA STTSRLSSAEASSRISSAASTLVSGGYLNTAALPSVISDLFAQVGASSPGVSDSEVLIQVLLEIVSSLIH ILSSSSVGQVDFSSVGSSAAAVGQSMQVVMG |
| 87 | GAGAGAGGAGGYGRGAGAGAGAGAGAAAGQGYGSGAGAGAGASAGGAGSYGRGAGAGA AAASGAGAGGYGAGQGYGAGAGAVASAAAGAGSGAGGAGGYGRGAVAGSGAGAGAGAGG AGGYGAGAGAGAAAGAVAGGSGGYGGRQGGYSAGAGAGAAAAA |
| 88 | GPGGYGPVQQGPSGPGSAAGPGGYGPAQQGPARYGPGSAAAAAAAAGSAGYGPGPQASAA ASRLASPDSGARVASAVSNLVSSGPTSSAALSSVISNAVSQIGASNPGLSGCDVLIQALLEIVSACV TILSSSSIGQVNYGAASQFAQVVGQSVLSAFS |
| 89 | GTGGVGGLFLSSGDFGRGGAGAGAGAAAASAAAASSAAAGARGGSGFGVGTGGFGRGGAGA GTGAAAASAAAASAAAAGAGGDGGLFLSSGDFGRGGAGAGAGAAAASAAAASSAAAGARGG SGFGVGTGGFGRGGAGDGASAAAASAAAASAAAA |
| 90 | GGYGPGAGQQGPGGAGQQGPGGQGPYGPSVAAAASAAGGYGPGAGQQGPVASAAVSRLSS PQASSRVSSAVSSLVSSGPTNPAALSNAMSSVVSQVSASNPGLSGCDVLVQALLEIVSALVHILGS SSIGQINYAASSQYAQMVGQSVAQALA |

| Seq. ID No. | AA |
|---|---|
| 91 | GGAGQGGYGGLGSQGAGRGGYGGQGAGAAAAATGGAGQGGYGGVGSGASAASAAASRLS SPQASSRVSSAVSNLVASGPTNSAALSSTISNAVSQIGASNPGLSGCDVLIQALLEVVSALIHILGSS SIGQVNYGSAGQATQIVGQSVYQALG |
| 92 | GGAGQGGYGGLGSQGAGRGGYGGQGAGAAVAAIGGVGQGGYGGVGSGASAASAAASRLSS PEASSRVSSAVSNLVSSGPTNSAALSSTISNVVSQIGASNPGLSGCDVLIQALLEVVSALVHILGSSS IGQVNYGSAGQATQIVGQSVYQALG |
| 93 | GASGGYGGGAGEGAGAAAAAGAGAGGAGGYGGGAGSGAGAVARAGAGGAGGYGSGIGGG YGSGAGAAAGAGAGGAGAYGGGYGTGAGAGARGADSAGAAAGYGGGVGTGTGSSAGYGR GAGAGAGAGAAAGSGAGAAGGYGGGYGAGAGAGA |
| 94 | GAGSGQGGYGGQGGLGGYGQGAGAGAAAGASGSGSGGAGQGGLGGYGQGAGAGAAAAA AGASGAGQGGFGPYGSSYQSSTSYSVTSQGAAGGLGGYGQGSGAGAAAAGAAGQGGQGGY GQGAGAGAGAGQGGLGGYGQGAGSSAASAAAA |
| 95 | GGAGQGGYGGLGGQGVGRGGLGGQGAGAAAAGGAGQGGYGGVGSGASAASAAASRLSSP QASSRLSSAVSNLVATGPTNSAALSSTISNVVSQIGASNPGLSGCDVLIQALLEVVSALIQILGSSSI GQVNYGSAGQATQIVGQSVYQALG |
| 96 | GAGSGGAGGYGRGAGAGAGAAAGAGAGAGSYGGQGGYGAGAGAGAAAAAGAGAGAGGY GRGAGAGAGAGAGAAARAGAGAGGAGYGGQGGYGAGAGAGAAAAAGAGAGGAGGYGRG AGAGAAAGAGAGAGGYGGQSGYGAGAGAAAAA |

(continued)

| Seq. ID No. | AA |
|---|---|
| 97 | GASGAGQGQGYGQQGQGGSSAAAAAAAAQGQGQGYGQQGQGYGQQGQGGSSAAA<br>AAAAAAAAQGQGQGYGQQGQGYGQQGQGGSSAAAAAGASGAGQGQGYGQQGQGGSSAAAAA<br>AAAAAAAAAAQGQGYGQQGQGSAAAAAAAAAA |

In an embodiment a block copolymer polypeptide repeat unit that forms fibers with good mechanical properties is synthesized using SEQ ID NO. 1. This repeat unit contains 6 quasi-repeats, each of which includes motifs that vary in composition, as described herein. This repeat unit can be concatenated 1, 2, 3, 4, 5, 6, 7, 8, 9, 10, 11, 12, 13, 14, 15, 16, 17, 18, 19 or 20 times to form polypeptide molecules from 20 kDal to 535 kDal, or greater than 20 kDal, or greater than 10 kDal, or greater than 5 kDal, or from 5 to 400 kDal, or from 5 to 300 kDal, or from 5 to 200 kDal, or from 5 to 100 kDal, or from 5 to 50 kDal, or from 5 to 600 kDal, or from 5 to 800 kDal, or from 5 to 1000 kDal, or from 10 to 400 kDal, or from 10 to 300 kDal, or from 10 to 200 kDal, or from 10 to 100 kDal, or from 10 to 50 kDal, or from 10 to 600 kDal, or from 10 to 800 kDal, or from 10 to 1000 kDal, or from 20 to 400 kDal, or from 20 to 300 kDal, or from 20 to 200 kDal, or from 20 to 100 kDal, or from 20 to 50 kDal, or from 40 to 300 kDal, or from 40 to 500 kDal, or from 20 to 600 kDal, or from 20 to 800 kDal, or from 20 to 1000 kDal. This polypeptide repeat unit also contains poly-alanine regions related to nanocrystalline regions, and glycine-rich regions related to beta-turn containing less-crystalline regions. In other embodiments the repeat is selected from any of the sequences listed as Seq ID Nos: 2-97.

In some embodiments, a filament yarn, or spun yarn, or blended yarn contains RPFs with proteins containing the SEQ ID Nos: 1-97.

In some embodiments, the quasi-repeat unit of the polypeptide can be described by the formula {GGY-[GPG-$X_1]_{n1}$-GPS-$(A)_{n2}$}, where $X_1$ is independently selected from the group consisting of SGGQQ, GAGQQ, GQGPY, AGQQ and SQ, is a number from 4 to 8, and n2 is a number from 6 to 20. The repeat unit is composed of multiple quasi-repeat units. In additional embodiments, 3 "long" quasi repeats are followed by 3 "short" quasi-repeat units. As mentioned above, short quasi- repeat units are those in which n1=4 or 5. Long quasi-repeat units are defined as those in which n1=6, 7 or 8. In some embodiments, all of the short quasi-repeats have the same $X_1$ motifs in the same positions within each quasi-repeat unit of a repeat unit. In some embodiments, no more than 3 quasi-repeat units out of 6 share the same $X_1$ motifs.

In additional embodiments, a repeat unit is composed of quasi-repeat units that do not use the same $X_1$ more than two occurrences in a row within a repeat unit. In additional embodiments, a repeat unit is composed of quasi-repeat units where at least 1, 2, 3, 4, 5, 6, 7, 8, 9, 10, 11, 12, 13, 14, 15, 16, 17, 18, 19 or 20 of the quasi-repeats do not use the same $X_1$ more than 2 times in a single quasi-repeat unit of the repeat unit.

In some embodiments, the structure of fibers formed from the described polypeptides form beta-sheet structures, beta-turn structures, or alpha-helix structures. In some embodiments, the secondary, tertiary and quaternary protein structures of the formed fibers are described as having nanocrystalline beta-sheet regions, amorphous beta-turn regions, amorphous alpha helix regions, randomly spatially distributed nanocrystalline regions embedded in a non-crystalline matrix, or randomly oriented nanocrystalline regions embedded in a non-crystalline matrix.

In some embodiments, the polypeptides utilized to form fibers with mechanical properties as described herein include glycine-rich regions from 20 to 100 amino acids long concatenated with poly-alanine regions from 4 to 20 amino acids long. In some embodiments, polypeptides utilized to form fibers with good mechanical properties comprise 5-25% poly-alanine regions (from 4 to 20 poly-alanine residues). In some embodiments, polypeptides utilized to form fibers with good mechanical properties comprise 25-50% glycine. In some embodiments, polypeptides utilized to form fibers with good mechanical properties comprise 15-35% GGX, where X is any amino acid. In some embodiments, polypeptides utilized to form fibers with good mechanical properties comprise 15-60% GPG. In some embodiments, polypeptides utilized to form fibers with good mechanical properties comprise 10-40% alanine. In some embodiments, polypeptides utilized to form fibers with good mechanical properties comprise 0-20% proline. In some embodiments, polypeptides utilized to form fibers with good mechanical properties comprise 10-50% beta-turns. In some embodiments, polypeptides utilized to form fibers with good mechanical properties comprise 10-50% alpha-helix composition. In some embodiments all of these compositional ranges will apply to the same polypeptide. In some embodiments two or more of these compositional ranges will apply to the same polypeptide.

RECOMBINANT PROTEIN FIBER SPIN DOPE AND SPINNING PARAMETERS

In some embodiments, a spin dope is synthesized containing proteins expressed from any of the polypeptides of the present disclosure. The spin dope is prepared using published techniques such as those found in WO2015042164 A2, especially at paragraphs 114-134. In some embodiments, a fiber spinning solution was prepared by dissolving the purified and dried block copolymer polypeptide in a formic acid-based spinning solution, using standard mixing techniques. Spin dopes were mixed until the polypeptide was completely dissolved as determined by visual inspection. Spin dopes were degassed and undissolved particulates were removed by centrifugation.

In an embodiment the fraction of protein that is at least some percentage (e.g., 80%) of the intended length is determined through quantitative analysis of the results of a size-separation process. In an embodiment, the size-separation process can include size-exclusion chromatography. In an embodiment, the size-separation process can include gel electrophoresis. The quantitative analysis can include determining the fraction of total protein falling within a designated size range by integrating the area of a chromatogram or densitometric scan peak. For example, if a sample is run through a size-separation process, and the relative areas under the peaks corresponding to full-length, 60% full-length and 20% full length are 3:2:1, then the fraction that is full length corresponds to 3 parts out of a total of 6 parts by mass = 50% mass ratio.

In some embodiments, the proteins of the spin dope, expressed from any of the polypeptides of the present disclosure, are substantially monodisperse. In some embodiments, the proteins of the spin dope, expressed from any of the polypeptides of the present disclosure, have from 5% to 99%, or from 5% to 50%, or from 50% to 99%, or from 20% to 80%, or from 40% to 60%, or from 5% to 30%, or from 70% to 99%, or from 5% to 20%, or from 5% to 10%, or from 80% to 99%, or from 90% to 99% of the protein in the spin dope having molecular weight from 5% to 99%, or from 5% to 50%, or from 50% to 99%, or from 20% to 80%, or from 40% to 60%, or from 5% to 30%, or from 70% to 99%, or from 5% to 20%, or from 5% to 10%, or from 80% to 99%, or from 90% to 99% of the molecular weight of the encoded proteins. The "encoded proteins" are defined as the polypeptide amino acid sequences that are encoded by the DNA utilized in protein expression. In other words, the "encoded proteins" are the polypeptides that would be produced if there were no imperfect processes (e.g., transcription errors, protein degradation, homologous recombination, truncation, protein fragmentation, protein agglomeration) at any stage during protein production. A higher monodispersity of proteins in the spin dopes, in other words a higher purity, can have the advantage of producing fibers with better mechanical properties, such as higher initial modulus, higher extensibility, higher ultimate tensile strength, and higher maximum tensile strength.

In other embodiments, fibers with low monodispersity, <10%, or <15%, or <20%, or <25%, or <30%, or <35%, or <40%, or <45%, or <50% of the protein in the spin dope having molecular weight >50%, or >55%, or >60%, or >65%, or >70%, or >75%, or >80%, or >85%, or >90%, or >95%, or >99% of the molecular weight of the proteins encoded by the DNA utilized in protein expression, were still able to create fibers with good mechanical properties. The mechanical properties described herein (e.g., high initial modulus and/or extensibility), from fibers formed from low purity spin dopes was achieved through the use of the long polypeptide repeat units, suitable polypeptide compositions and spin dope and fiber spinning parameters described elsewhere in the present disclosure.

In other embodiments, the proteins are produced via secretion from a microorganism such as *Pichia pastoris, Escherichia coli, Bacillus subtilis, or mammalian cells.* Optionally, the secretion rate is at least 20 mg /g DCW / hr (DCW = dry cell weight). Optionally, the proteins are then recovered, separated, and spun into fibers using spin dopes containing solvents. Some examples of the classes of solvents that can be used in spin dopes are aqueous, inorganic or organic, including but not limited to ethanol, methanol, isopropanol, t-butyl alcohol, ethyl acetate, and ethylene glycol. Various methods for synthesizing recombinant proteinaceous block copolymers have been published such as those found in WO2015042164 A2, especially at paragraphs 114-134.

In some embodiments, the fibers are extruded through a spinneret to form long uniform RPFs, for example greater than 20 m long. Continuous fiber manufacturing includes the following processes: pumping, filtration, fiber forming, and optionally, fiber treatment. The spin dope is pumped through a filter and subsequently through the spinneret, which contains small holes. Resistance in the fluid paths through the filter and the spinneret produces a pressure drop across each of these elements. The pumping pressure and type of pump required is dictated by the system elements' intrinsic fluid dynamic properties, the pathways used to interconnect them, and the viscosity of the spin dope liquid. Filtration is used to screen out particles that would lead to defects in the fiber, or lead to an obstruction of one of the spinneret holes. In some embodiments, screen filtration or deep bed type filtration systems is used. Recombinant protein fibers are formed using wet spinning, and the spin dope coagulates in a coagulation bath upon leaving the spinneret holes. Due to the friction between the coagulated fiber and the coagulant, continuous fiber manufacture employs lower spinning speeds than those used for other spinning processes (such as melt spinning or dry spinning). In some embodiments post-spinning fiber treatments, such as cold drawing or hot drawing are used. Drawing imparts a higher degree of polymer orientation in the fiber, which leads to improved mechanical properties.

In some embodiments, a solution of polypeptide is spun into fibers using elements of processes known in the art. These processes include, for example, wet spinning, dry-jet wet spinning, and dry spinning. In preferred wet-spinning embodiments, the filament is extruded through an orifice into a liquid coagulation bath. In one embodiment, the filament can be extruded through an air gap prior to contacting the coagulation bath. In a dry-jet wet spinning process, the spinning solution is attenuated and stretched in an inert, non-coagulating fluid, e.g., air, before entering the coagulating bath. Suitable coagulating fluids are the same as those used in a wet-spinning process.

In other embodiments, the coagulation bath conditions for wet spinning are chosen to promote fiber formation with certain mechanical properties. Optionally, the coagulation bath is maintained at temperatures of 0-90° C, more preferably 20-60° C. Optionally, the coagulation bath comprises about 60%, 70%, 80%, 90%, or even 100% alcohol, preferably isopropanol, ethanol, or methanol. Optionally, the coagulation bath is 95:5, 90:10, 85:15, 80:20, 75:25, 70:30, 65:35, 60:40, 55:45 or 50:50 by volume methanol:water. Optionally, the coagulation bath contains additives to enhance the fiber mechanical properties, such as additives comprising ammonium sulfate, sodium chloride, sodium sulfate, or other protein precipitating salts at temperature from 20 to 60 °C.

In some embodiments, the extruded filament or fiber is passed through more than one bath. For embodiments in which more than one bath is used, the different baths have either different or same chemical compositions. In some embodiments, the extruded filament or fiber is passed through more than one coagulation bath. For embodiments in which more than one coagulation bath is used, the different coagulation baths have either different or same chemical compositions. The residence time can be tuned to improve mechanical properties, such as from 2 seconds to 100 minutes in the coagulant bath. The reeling/drawing rate can be tuned to improve fiber mechanical properties, such as a rate from 0.1 to 100 meters/minute.

Optionally, the filament or fiber is also passed through one or more rinse baths to remove residual solvent and/or coagulant. Rinse baths of decreasing salt or alcohol concentration up to, preferably, an ultimate water bath, preferably follow salt or alcohol baths.

Following extrusion, the filament or fiber can be drawn. Drawing can improve the consistency, axial orientation and toughness of the filament. Drawing can be enhanced by the composition of a coagulation bath. Drawing may also be performed in a drawing bath containing a plasticizer such as water, glycerol or a salt solution. Drawing can also be performed in a drawing bath containing a crosslinker such as gluteraldehyde or formaldehyde. Drawing can be performed at temperature from 25-100 °C to alter fiber properties, preferably at 60 °C. As is common in a continuous process, drawing can be performed simultaneously during the coagulation, wash, plasticizing, and/or crosslinking procedures described previously. Drawing ratio depends on the filament being processed. In some embodiments, the drawing rate is about 4×, or 5×, or 6×, or 7×, or 8×, or 9×, or 10×, or 11×, or 12×, or 13×, or 14×, or 15× the rate of reeling from the coagulation bath.

In certain embodiments of the invention, the filament is wound onto a spool after extrusion or after drawing. Winding rates are generally 1 to 500 m/min, preferably 10 to 50 m/min.

In some embodiments, the extruded filament or fiber is passed through more than one coagulation bath. For embodiments in which more than one coagulation bath is used, the different coagulation baths have either different or same chemical compositions. The residence time can be tuned to improve mechanical properties, such as from 2 to 100 seconds in the coagulant bath. The reeling/drawing rate can be tuned to improve fiber mechanical properties, such as a rate from 2 to 10 meters/minute.

The draw ratio can also be tuned to improve fiber mechanical properties. In different embodiments the draw ratio was 1.5X to 6X. In one embodiment, lower draw ratios improved the fiber extensibility. In one embodiment, higher draw ratios improved the fiber maximum tensile strength. Drawing can also be done in different environments, such as in solution, in humid air, or at elevated temperatures.

The fibers of the present disclosure processed with residence times in coagulation baths at the longer end of the disclosed range produce corrugated cross sections. That is, each fiber has a plurality of corrugations (or alternatively "grooves") disposed at an outer surface of a fiber. Each of these corrugations is parallel to a longitudinal axis of the corresponding fiber on which the corrugations are disposed. The fibers of the present disclosure processed with higher ethanol content in the coagulation bath produce hollow core fibers. That is, the fiber includes an inner surface and an outer surface. The inner surface defines a hollow core parallel to the longitudinal axis of the fiber.

In some embodiments a coagulation bath or the first coagulation bath is prepared using combinations of one or more of water, acids, solvents and salts, including but not limited to the following classes of chemicals of Bronsted-Lowry acids, Lewis acids, binary hydride acids, organic acids, metal cation acids, organic solvents, inorganic solvents, alkali metal salts, and alkaline earth metal salts. Some examples of acids used in the preparation of a coagulation bath or the first coagulation bath are dilute hydrochloric acid, dilute sulfuric acid, formic acid and acetic acid. Some examples of solvents that are used in the preparation of the first coagulation bath are ethanol, methanol, isopropanol, t-butyl alcohol, ethyl acetate, and ethylene glycol. Examples of salts used in the preparation of a coagulation bath or the first coagulation bath include LiCl, KCl, BeCl2, MgCl2, CaCl2, NaCl, ammonium sulfate, sodium sulfate, and other salts of nitrates, sulfates or phosphates.

In some embodiments, the chemical composition and extrusion parameters of a coagulation bath or the first coagulation bath are chosen so that the fiber remains translucent in a coagulation bath or the first coagulation bath. In some embodiments the chemical composition and extrusion parameters of a coagulation bath or the first coagulation bath are chosen to slow down the rate of coagulation of the fiber in a coagulation bath or the first coagulation bath, which improves the ability to draw the resulting fiber in subsequent drawing steps. In various embodiments, these subsequent drawing steps are done in different environments, including wet, dry, and humid air environments. Examples of wet environments include one or more additional baths or coagulation baths. In some embodiments, the fiber travels through one or more baths after the first coagulation bath. The one or more additional baths, or coagulation baths, are prepared, in embodiments, using combinations of one or more of water, acids, solvents and salts, including but not limited to the following classes of chemicals of Bronsted-Lowry acids, Lewis acids, binary hydride acids, organic acids, metal cation acids, organic solvents, inorganic solvents, alkali metal salts, and alkaline earth metal salts. Some examples of acids that are used in the preparation of the second baths or coagulant baths are dilute hydrochloric acid, dilute sulfuric acid, formic acid and acetic acid. Some examples of solvents that are used in the preparation of the second coagulant baths are ethanol, methanol, isopropanol, t-butyl alcohol, ethyl acetate, and ethylene glycol. Some examples of salts used in the preparation of a second bath or coagulation bath include LiCl, KCl, MgCl2, CaCl2, NaCl, ammonium sulfate, sodium sulfate, and other salts of nitrates, sulfates, or phosphates. In some embodiments, there are two coagulation baths, where the first coagulation bath has a different chemical composition than the second coagulation bath, and the second coagulation bath has a higher concentration of solvents than the first coagulation bath. In some embodiments, there are more than two coagulation baths, and the first coagulation bath has a different chemical composition than the second coagulation bath, and the second coagulation bath has a lower concentration of solvents than the first coagulation bath. In some embodiments, there are two baths, the first being a coagulation bath and the second being a wash bath. In some embodiments, the first coagulation bath has a different chemical composition than the second wash bath, and the second wash bath has a higher concentration of solvents than the first bath. In some embodiments, there are more than two baths, and the first bath has a different chemical composition than the second bath, and the second bath has a lower concentration of solvents than the first bath.

In some embodiments a spin dope is further prepared using combinations of one or more of water, acids, solvents and salts, including but not limited to the following classes of chemicals of Bronsted-Lowry acids, Lewis acids, binary hydride acids, organic acids, metal cation acids, organic solvents, inorganic solvents, alkali metal salts, and alkaline earth metal salts. Some examples of acids that are used in the preparation of spin dopes are dilute hydrochloric acid, dilute sulfuric acid, formic acid and acetic acid. Some examples of solvents that are used in the preparation of spin dopes are ethanol, methanol, isopropanol, t-butyl alcohol, ethyl acetate, and ethylene glycol. Some examples of salts that are used in the preparation of spin dopes are LiCl, KCl, MgCl2, CaCl2, NaCl, ammonium sulfate, sodium sulfate, and other salts of nitrates, sulfates or phosphates.

In some embodiments, a spinneret is chosen to enhance the fiber mechanical properties. The dimensions of the spinneret can be from 0.001 cm to 5 cm long, and from 25 to 200 um in diameter. In some embodiments, a spinneret includes multiple orifices to spin multiple fibers simultaneously. In some embodiments, the cross-section of a spinneret gradually tapers to the smallest diameter at the orifice, is straight-walled and then quickly tapers to the orifice, or includes multiple constrictions. An extrusion pressure of a spin dope from a spinneret can also be varied to affect the fiber mechanical properties in a range from 10 to 1000 psi. The interaction between fiber properties and extrusion pressure can be affected by spin dope viscosity, drawing/reeling rate, and coagulation bath chemistry.

The concentration of protein to solvent in the spin dope is also an important parameter. In some embodiments, the concentration of protein weight for weight is 20%, or 25%, or 30%, or 35%, or 40%, or 45% or 50%, or 55%, or from 20% to 55%, or from 20% to 40%, or from 30% to 40%, or from 30% to 55%, or from 30% to 50% in solution with solvents and other additives making up the remainder.

RECOMBINANT PROTEIN FIBER YARNS

In some embodiments, yarns comprising recombinant protein fibers are manufactured into filament yarns, spun yarns, or blended yarns. In some embodiments, the filament yarns, spun yarns, or blended yarns contain recombinant protein fibers with mechanical properties such as high initial modulus, high extensibility, high tenacity, and high toughness. The filament yarns, spun yarns, or blended yarns can also contain recombinant protein fibers with structural properties such as high fineness (e.g., small diameter, low linear density, low denier), high softness, smoothness, engineered cross-section shapes and porosity. The filament yarns, spun yarns, or blended yarns can also contain recombinant protein fibers with chemical properties such as hydrophilicity. The filament yarns, spun yarns, or blended yarns can also contain recombinant protein fibers with biological properties such as being antimicrobial.

Engineering Yarn RPF Linear Density

In some embodiments, the synthesized fibers making up the filament yarn, or spun yarn, or blended yarn exhibit linear density less than 5 dtex, or less than 3 dtex, or less than 2 dtex, or less than 1.5 dtex, or greater than 1.5 dtex, or greater than 1.7 dtex, or greater than 2 dtex, or from 1 to 5 dtex, or from 1 to 3 dtex, or from 1.5 to 2 dtex, or from 1.5 to 2.5 dtex.

In some embodiments, the median or mean denier of the recombinant protein fibers comprising the filament yarn, or spun yarn, or blended yarn is less than 1 denier (about 15 microns in diameter). In some embodiments, the median or mean denier of the recombinant protein fibers comprising the filament yarn, or spun yarn, or blended yarn is less than 0.5 denier (about 10 microns in diameter). Microfibers are a classification of fibers having a fineness of less than 1 decitex (dtex), approximately 10 μm in diameter. H.K., Kaynak and O. Babaarslan, Woven Fabrics, Croatia: InTech, 2012. The small diameter of microfibers imparts a range of qualities and characteristics to microfiber yarns and fabrics that are desirable to consumers. Microfibers are inherently more flexible (bending is inversely proportional to fiber diameter) and thus have a soft feel, low stiffness, and high drapeability. Microfibers can also be formed into filament yarns having high fiber density (greater fibers per yarn cross-sectional area), giving microfiber yarns a higher strength compared to other yarns of similar dimensions. Microfibers also contribute to discrete stress relief within the yarn, resulting in anti-wrinkle fabrics. Furthermore, microfibers have high compaction efficiency within the yarn, which improves fabric waterproofness and windproofness while maintaining breathability compared to other waterproofing and windproofing techniques (such as polyvinyl coatings). The high density of fibers within microfiber fabrics results in microchannel structures between fibers, which promotes the capillary effect and imparts a wicking and quick drying characteristic. The high surface area to volume of microfiber yarns allows for brighter and sharper dyeing, and printed fabrics have clearer and sharper pattern retention as well. Currently, recombinant silk fibers do not have a fineness that is small enough to result in silks having microfiber type characteristics. U.S. Pat. App. Pub. No. 2014/0058066 generally discloses fiber diameters between 5-100 $\mu$m, but does not actually disclose any working examples of any fiber having a diameter as small as 5 $\mu$m.

In some embodiments, the median or mean linear density of the recombinant protein fibers comprising the filament yarn, or spun yarn, or blended yarn is less than 5 denier, or is less than 10 denier, or is less than 15 denier, or is less than 20 denier, or is from 1 to 30 denier, or from 1 to 20 denier, or from 1 to 10 denier, or from 1 to 5 denier, or from 0.1 to 5 denier, or from 0.1 to 30 denier.

Engineering Yarn RPF Mechanical Properties

Filament yarns, spun yarns, and blended yarns can be formed using many different techniques and constituent fibers. In some embodiments, a filament yarn, or spun yarn, or blended yarn comprises recombinant protein fibers, wherein the median or mean properties of the fibers comprise an initial modulus greater than 115 cN/tex, a maximum tensile strength greater than 7.7 cN/tex, and an extensibility of at least 3%. In some embodiments, a filament yarn, or spun yarn, or blended yarn comprises recombinant protein fibers, wherein the median or mean properties of the fibers comprise an initial modulus greater than 115 cN/tex a maximum tensile strength greater than 7.7 cN/tex, and an extensibility of at least 3%, or an extensibility of greater than 10%, or an extensibility of greater than 30%, or an extensibility of greater than 50%, or an extensibility of greater than 100%, or an extensibility of greater than 200%, or an extensibility of greater than 300%. In some embodiments, a filament yarn comprises recombinant protein fibers, wherein the median or mean properties of the fibers comprise an initial modulus from 10 to 1000 cN/tex, a maximum tensile strength from 0.5 to 100 cN/tex, and an extensibility from 1% to 300%. In some embodiments, a filament yarn, or spun yarn, or blended yarn comprises recombinant protein fibers, wherein the median or mean properties of the fibers comprise an initial modulus from 10 to 1000 cN/tex. In some embodiments, a filament yarn, or spun yarn, or blended yarn comprises recombinant protein fibers, wherein the median or mean properties of the fibers comprise a maximum tensile strength from 0.5 to 100 cN/tex. In some embodiments, a filament yarn, or spun yarn, or blended yarn comprises recombinant protein fibers, wherein the median or mean properties of the fibers comprise an extensibility from 1 to 300%. In some embodiments, a filament yarn, or spun yarn, or blended yarn comprises recombinant protein fibers, wherein the median or mean properties of the fibers comprise an extensibility of at least 3%, or an extensibility of greater than 10%, or an extensibility of greater than 30%, or an extensibility of greater than 50%, or an extensibility of greater than 100%, or an extensibility of greater than 200%, or an extensibility of greater than 300%. In some embodiments, a filament yarn, or spun yarn, or blended yarn comprises recombinant protein fibers, wherein the median or mean properties of the fibers comprise an initial modulus greater than 50 cN/tex, or greater than 115 cN/tex, or greater than 200 cN/tex, or greater than 400 cN/tex, or greater than 550 cN/tex, or greater than 600 cN/tex, or greater than 800 cN/tex, or greater than 1000 cN/tex, or greater than 2000 cN/tex, or greater than 3000 cN/tex, or greater than 4000 cN/tex, or greater than 5000 cN/tex, or from 200 to 900 cN/tex, or from 100 to 7000 cN/tex, or from 500 to 7000 cN/tex, or from 50 to 7000 cN/tex, or from 100 to 5000 cN/tex, or from 500 to 5000 cN/tex, or from 50 to 5000 cN/tex, or from 100 to 2000 cN/tex, or from 500 to 2000 cN/tex, or from 50 to 2000 cN/tex, or from 100 to 1000 cN/tex, or from 500 to 1000 cN/tex, or from 50 to 1000 cN/tex, or from 50 to 500 cN/tex, or from 100 to 1000 cN/tex, or from 500 to 1000 cN/tex, or from 100 to 700 cN/tex. In some embodiments, a filament yarn, or spun yarn, or blended yarn comprises recombinant protein fibers, wherein the median or mean properties of the fibers comprise a maximum tensile strength greater than 0.5 cN/tex, or a maximum tensile strength greater than 1 cN/tex, or a maximum tensile strength greater than 2 cN/tex, or a maximum tensile strength greater than 4 cN/tex, or a maximum tensile strength greater than 6 cN/tex, or a maximum tensile strength greater than 7.7 cN/tex, or a maximum tensile strength greater than 8 cN/tex, or a maximum tensile strength greater than 10 cN/tex, or a maximum tensile strength greater than 15 cN/tex, or a maximum tensile strength greater than 20 cN/tex, or a maximum tensile strength greater than 25 cN/tex, or a maximum tensile strength greater than 30 cN/tex, or a maximum tensile strength greater than 40 cN/tex, or a maximum tensile strength greater than 50 cN/tex, or a maximum tensile strength greater than 60 cN/tex, or a maximum tensile strength greater than 70 cN/tex, or a maximum tensile strength greater than 80 cN/tex, or a maximum tensile strength greater than 90 cN/tex, or a maximum tensile strength greater than 100 cN/tex.

In some embodiments, a filament yarn, or spun yarn, or blended yarn comprises recombinant protein fibers, wherein the median or mean properties of the fibers comprise an initial modulus greater than 50 cN/tex, or greater than 115 cN/tex, or greater than 200 cN/tex, or greater than 400 cN/tex, or greater than 550 cN/tex, or greater than 600 cN/tex, or greater than 800 cN/tex, or greater than 1000 cN/tex, or greater than 2000 cN/tex, or greater than 3000 cN/tex, or greater than 4000 cN/tex, or greater than 5000 cN/tex, and a maximum tensile strength greater than 0.5 cN/tex, or greater than 1 cN/tex, or greater than 2 cN/tex, or greater than 4 cN/tex, or greater than 6 cN/tex, or greater than 7.7 cN/tex, or greater than 10 cN/tex, or greater than 15 cN/tex, or greater than 20 cN/tex, or greater than 25 cN/tex, or greater than 30 cN/tex, or greater than 40 cN/tex, or greater than 50 cN/tex, or greater than 60 cN/tex, or greater than 70 cN/tex, or greater than 80 cN/tex, or greater than 90 cN/tex, or greater than 100 cN/tex, and an extensibility of at least 10%, or greater than 20%, or greater than 30%, or greater than 50%, or greater than 100%, or greater than 200%, or greater than 300%. In some embodiments, a filament yarn, or spun yarn, or blended yarn comprises recombinant protein fibers, wherein the median or mean properties of the fibers comprise an initial modulus from 100 to 7000 cN/tex, or from 500 to 7000 cN/tex, or from 50 to 7000 cN/tex, or from 100 to 5000 cN/tex, or from 500 to 5000 cN/tex, or from 50 to 5000 cN/tex, or from 100 to 2000 cN/tex, or from 500 to 2000 cN/tex, or from 50 to 2000 cN/tex, or from 100 to 1000 cN/tex, or from 500 to 1000 cN/tex, or from 50 to 1000 cN/tex, or from 50 to 500 cN/tex, or from 100 to 1000 cN/tex, or from 500 to 1000 cN/tex, or from 100 to 700 cN/tex, and a maximum tensile strength from 0.5 to 100 cN/tex, or from 5 to 100 cN/tex, or from 5 to 50 cN/tex, and an extensibility from 10 to 300 %, or from 10 to 100%, or from 10 to 50%. The standard test method for measuring tensile properties of yarns by the single-strand method is ASTM D2256-10. These fiber mechanical properties enable use of the fibers in industrial fiber drawing and yarn forming methods. Such yarns are also useful in a myriad of applications, such as construction into ropes, textiles and garments, upholstery or linens.

In embodiments, the synthesized fibers making up the filament yarn, or spun yarn, or blended yarn exhibit a high extensibility (i.e., a strain to fracture). Specifically, in embodiments, the synthesized fibers making up the filament yarn, or spun yarn, or blended yarn exhibit an extensibility (i.e., a strain to fracture) greater than 1%, or greater than 5%, or greater than 10%, or greater than 20%, or greater than 100%, or greater than 200%, or greater than 300%, or greater than 400%. In embodiments, the synthesized fibers making up the filament yarn, or spun yarn, or blended yarn exhibit an extensibility (i.e., strain to fracture) of from 1% to 400%, or from 1 to 200%, or from 1 to 100%, or from 1 to 20%, or from 10 to 200%, or from 10 to 100%, or from 10 to 50%, or from 10 to 20%, or from 50% to 150%, or from 100% to 150%, or from 300% to 400%.

In embodiments, the synthesized fibers making up the filament yarn, or spun yarn, or blended yarn exhibit a high elastic modulus. Specifically, in embodiments, the synthesized fibers making up the filament yarn, or spun yarn, or blended yarn exhibit an elastic modulus greater than 1500 MPa, or greater than 2000 MPa, or greater than 3000 MPa, or greater than 5000 MPa, or greater than 6000 MPa, or greater than 7000 MPa. In embodiments, the synthesized fibers making up the filament yarn, or spun yarn, or blended yarn exhibit an elastic modulus from 5200 to 7000 MPa, or from 1500 to 10000 MPa, or from 1500 to 8000 MPa, or from 2000 to 8000 MPa, or from 3000 to 8000 MPa, or from 5000 to 8000 MPa, or from 5000 to 6000 MPa, or from 6000 to 8000 MPa. In embodiments, the synthesized fibers making up the filament yarn, or spun yarn, or blended yarn exhibit an elastic modulus greater than 100 cN/tex, or greater than 200 cN/tex, or greater than 300 cN/tex, or greater than 400 cN/tex, or greater than 500 cN/tex, or greater than 550 cN/tex, or greater than 600 cN/tex. In embodiments, the synthesized fibers making up the filament yarn, or spun yarn, or blended yarn exhibit an elastic modulus from 100 to 600 cN/tex, or from 200 to 600 cN/tex, or from 300 to 600 cN/tex, or from 400 to 600 cN/tex, or from 500 to 600 cN/tex, or from 550 to 600 cN/tex, or from 550 to 575 cN/tex, or from 500 to 750 cN/tex, or from 500 to 1000 cN/tex, or from 500 to 1500 cN/tex.

In embodiments, the synthesized fibers making up the filament yarn, or spun yarn, or blended yarn exhibit a high maximum tensile strength. In embodiments, the synthesized fibers making up the filament yarn, or spun yarn, or blended yarn exhibit a maximum tensile strength greater than 100 MPa, or greater than 120 MPa, or greater than 140 MPa, or greater than 160 MPa, or greater than 180 MPa, or greater than 200 MPa, or greater than 220 MPa, or greater than 240 MPa, or greater than 260 MPa, or greater than 280 MPa, or greater than 300 MPa, or greater than 400 MPa, or greater than 600 MPa, or greater than 1000 MPa. In embodiments, the synthesized fibers making up the filament yarn, or spun yarn, or blended yarn exhibit a maximum tensile strength from 100 to 1000 MPa, or from 100 to 500 MPa, or from 100 to 300 MPa, or from 100 to 250 MPa, or from 100 to 200 MPa, or from 100 to 150 MPa. In embodiments, the synthesized fibers making up the filament yarn, or spun yarn, or blended yarn exhibit an ultimate tensile strength greater than 100 MPa, or greater than 120 MPa, or greater than 140 MPa, or greater than 160 MPa, or greater than 180 MPa, or greater than 200 MPa, or greater than 220 MPa, or greater than 240 MPa, or greater than 260 MPa, or greater than 260 MPa, or greater than 280 MPa, or greater than 300 MPa, or greater than 400 MPa, or greater than 600 MPa, or greater than 1000 MPa. In embodiments, the synthesized fibers making up the filament yarn, or spun yarn, or blended yarn exhibit an ultimate tensile strength from 100 to 1000 MPa, or from 100 to 500 MPa, or from 100 to 300 MPa, or from 100 to 250 MPa, or from 100 to 200 MPa, or from 100 to 150 MPa. In embodiments, the synthesized fibers making up the filament yarn, or spun yarn, or blended yarn exhibit a maximum tensile strength greater than 0.5 cN/tex, or greater than 1 cN/tex, or greater than 2 cN/tex, or greater than 5 cN/tex, or greater than 10 cN/tex, or greater than 15 cN/tex, or greater than 20 cN/tex, or greater than 25 cN/tex. In embodiments, the synthesized fibers making up the filament yarn, or spun yarn, or blended yarn exhibit a maximum tensile strength from 0.5 to 30 cN/tex, or from 0.5 to 25 cN/tex, or from 0.5 to 20 cN/tex, or from 0.5 to 10 cN/tex, or from 5 to 30 cN/tex, or from 5 to 25 cN/tex, or from 10 to 30 cN/tex, or from 10 to 20 cN/tex, or from 15 to 20 cN/tex, or from 15 to 50 cN/tex, or from 15 to 75 cN/tex, or from 15 to 100 cN/tex. In embodiments, the synthesized fibers making up the filament yarn, or spun yarn, or blended yarn exhibit an ultimate tensile strength greater than 5 cN/tex, or greater than 10 cN/tex, or greater than 15 cN/tex, or greater than 20 cN/tex, or greater than 25 cN/tex. In embodiments, the synthesized fibers making up the filament yarn, or spun yarn, or blended yarn exhibit an ultimate tensile strength from 5 to 30 cN/tex, or from 5 to 25 cN/tex, or from 10 to 30 cN/tex, or from 10 to 20 cN/tex, or from 15 to 20 cN/tex, or from 15 to 50 cN/tex, or from 15 to 75 cN/tex, or from 15 to 100 cN/tex.

In some embodiments, the synthesized fibers making up the filament yarn, or spun yarn, or blended yarn exhibit a high work of rupture (as a measure of the fiber toughness). In some embodiments, the synthesized fibers making up the filament yarn, or spun yarn, or blended yarn exhibit a work of rupture greater than 0.1 cN*cm, or greater than 0.2 cN*cm, or greater than 0.3 cN*cm, or greater than 0.4 cN*cm, or greater than 0.5 cN*cm, or greater than 0.6 cN*cm, or greater than 0.7 cN*cm, or greater than 0.8 cN*cm, or greater than 0.9 cN*cm, or greater than 1 cN*cm, or greater than 1.3 cN*cm, or greater than 2 cN*cm, or greater than 5 cN*cm, or greater than 10 cN*cm, or from 0.1 to 10 cN*cm, or from 0.1 to 5 cN*cm, or from 0.1 to 2 cN*cm, or from 0.2 to 5 cN*cm, or from 0.2 to 10 cN*cm, or from 0.2 to 2 cN*cm, or from 0.3 to 2 cN*cm, or from 0.4 to 10 cN*cm, or from 0.4 to 5 cN*cm, or from 0.4 to 2 cN*cm, or from 0.4 to 1 cN*cm, or from 0.5 to 2 cN*cm, or from 0.5 to 1.3 cN*cm, 0.6 to 2 cN*cm, or from 0.7 to 1.1 cN*cm.

Toughness (defined as the area under the stress-strain curve) is an important characteristic of textile fibers. In some embodiments, the recombinant protein fibers comprising the filament yarn, or spun yarn, or blended yarn have a median or mean toughness greater than 2 cN/tex, or from 0.5 to 70 cN/tex, or greater than 3 cN/tex, or greater than 4 cN/tex, or greater than 5 cN/tex, or greater than 7.5 cN/tex, or greater than 10 cN/tex, or greater than 20 cN/tex, or greater than 30 cN/tex, or greater than 40 cN/tex, or greater than 50 cN/tex, greater than 60 cN/tex, or greater than 70 cN/tex, or from 2 to 3 cN/tex, or from 3 to 4 cN/tex, or from 4 to 5 cN/tex, or from 5 to 7.5 cN/tex, or from 7.5 to 10 cN/tex, or from 10 to 20 cN/tex, or from 20 to 30 cN/tex, or from 30 to 40 cN/tex, or from 40 to 50 cN/tex, or from 50 to 60 cN/tex, or from 60 to 70 cN/tex. Filament yarns, or spun yarns, or blended yarns comprising fibers with high toughness can be used in many applications, including: carpeting and carpet backing, industrial textile products (such as tire cord and tire fabric, seat belts, industrial webbing and tape, tents, fishing line and nets, rope, and tape reinforcement), apparel fabrics (such as women's sheer hosiery, underwear, nightwear, anklets and socks, and a variety of apparel fabrics), interior and household products (such as bed ticking, furniture upholstery, curtains, bedspreads, sheets, and draperies).

Engineering Yarn RPF Moisture Properties

There are many different metrics by which to characterize the interaction between a fiber and water. One such method is measuring the hydrophilicity of the surface of the fiber, characterized by the contact angle with water. In some embodiments, the recombinant protein fibers, comprising the filament yarn, or spun yarn, or blended yarn, when measured with a fiber tensiometer, have a median or mean tensiometer contact angle of less than 90 degrees, or less than 80 degrees, or less than 70 degrees, or less than 60 degrees, or between 60 and 90 degrees or 60 and 80 degrees, or from 60 and 70 degrees, or from 70 and 90 degrees, or from 70 and 80 degrees, or from 80 and 90 degrees when tested using a standard assay with a water-filled tensiometer. Such yarns are useful in textiles which use fiber properties and yarn constructions used to pull moisture away from the skin in order to create more comfort for the wearer. In some embodiments, these filament yarns, or spun yarns, or blended yarns can be constructed into plaited yarn or textile, or double knit textiles. In some embodiments, these textiles can be located in a position towards the outer surface of a textile and/or garment to allow the absorbed moisture to easily evaporate.

Another moisture-related characteristic of a fiber is the degree of swelling when submerged in water. In some embodiments, the recombinant protein fibers comprising the filament yarn, or spun yarn, or blended yarn have high moisture absorption properties. In some embodiments, the recombinant protein fibers comprising the filament yarn, or spun yarn, or blended yarn have high moisture absorption properties, with median or mean of greater than 5% diameter change upon being submerged in water at a temperature of 21 °C +/- 1 °C. In some embodiments, the recombinant protein fibers comprising the filament yarn, or spun yarn, or blended yarn have high moisture absorption properties, with median or mean diameter change upon being submerged in water at a temperature of 21 °C +/- 1 °C from 0.1% to 100%. In some embodiments, the recombinant protein fibers comprising the filament yarn, or spun yarn, or blended yarn have high moisture absorption properties upon being submerged in water at a temperature of 21 °C +/- 1 °C, with median or mean diameter change greater than 1%, or greater than 2%, or greater than 4%, or greater than 6%, or greater than 8%, or greater than 10%, or greater than 15%, or greater than 20%, or greater than 25%, or greater than 30%, or greater than 35%, or greater than 40%, or greater than 45%, or greater than 50%, or greater than 60%, or greater than 70%, or greater than 80%, or greater than 90%, or from 5% to 10%, or from 10% to 20%, or from 20% to 30%, or from 30% to 40%, or from 40% to 50%, or from 50% to 60%, or from 60% to 70%, or from 70% to 80%, or from 80% and 90%, or from 90% to 100%, or from 20% to 35%, or from 15% to 40%, or from 15% to 35%. Such a filament yarn, or spun yarn, or blended yarn is useful in textiles and garments such as skin knits or woven fabrics where transfer of moisture away from the skin is desired, such as active wear apparel. In some embodiments, these filament yarn, or spun yarn, or blended yarn can be constructed into plaited yarn or textile, or double knit textiles. In some embodiments, these textiles can be located in a position towards the outer surface of a textile and/or garment to allow the absorbed moisture to easily evaporate. Fiber diameter change can be directly measured using optical microscopy.

Two other moisture-related characteristics of fibers are moisture regain and moisture content, which measure the uptake of water vapor from the environment. In one type of measurement a sample is allowed to equilibrate in an environment with a known relative humidity (e.g., 60-70% relative humidity) and temperature (e.g., 20-25 °C), and then heated to drive out the water (e.g., at a temperature slightly above 100 °C). Using a tool, such as a thermogravimetric analysis (TGA) system, the initial conditioned mass (containing some water), the final dry mass, and the mass change can be measured over time. The moisture regain of the fiber is defined as the lost water mass divided by the dry mass. The moisture content of the RPF is defined as the lost water mass divided by the conditioned mass. In some embodiments, the recombinant protein fibers comprising the filament yarn, or spun yarn, or blended yarn have high moisture absorption properties, have median or mean moisture regain or moisture content, when measured from equilibrium conditioned mass at 65% relative humidity environment at 22 °C and heated at 110 °C until approximately equilibrium dry mass is achieved, of greater than 1%, or greater than 2%, or greater than 3% or greater than 4%, or greater than 5%, or greater than 6%, or greater than 7%, or greater than 8%, or greater than 9%, or greater than 10%, or greater than 12%, or greater than 14%, or greater than 16%, or greater than 18%, or greater than 20%, or from 1% to 30%, or from 1% to 30%, or from 1% to 20%, or from 1% to 15%, or from 1% to 10%, or from 5% to 15%, or from 5% to 10%.

In some embodiments, the recombinant protein fibers comprising the filament yarn, or spun yarn, or blended yarn have high moisture wicking properties. A standard method of measuring wicking rate is the AATCC test method 197-2011 for vertical wicking of textiles, and AATCC test method 198-2011 for horizontal wicking of textiles. In some embodiments, a plain weave 1/1 textile with warp density of 72 warps/cm and pick density of 40 picks/cm, comprising filament yarn, or spun yarn, or blended yarn, comprising recombinant protein fibers, is tested using AATCC test method 197-2011, and has a median or mean horizontal wicking rate greater than 1 mm/s, or a median or mean horizontal wicking rate from 0.1 to 100 mm/s, or a median or mean horizontal wicking rate greater than 0.1 mm/s, or has a median or mean horizontal wicking rate greater than 0.2 mm/s, or has a median or mean horizontal wicking rate greater than 0.4 mm/s, or has a median or mean horizontal wicking rate greater than 0.6 mm/s, or has a median or mean horizontal wicking rate greater than 0.8 mm/s, or has a median or mean horizontal wicking rate greater than 2 mm/s, or has a median or mean horizontal wicking rate greater than 4 mm/s, or has a median or mean horizontal wicking rate greater than 6 mm/s, or has a median or mean horizontal wicking rate greater than 8 mm/s, or has a median or mean horizontal wicking rate greater than 10 mm/s, or has a median or mean horizontal wicking rate greater than 15 mm/s, or has a median or mean horizontal wicking rate greater than 20 mm/s, or has a median or mean horizontal wicking rate greater than 40 mm/s, or has a median or mean horizontal wicking rate greater than 60 mm/s, or has a median or mean horizontal wicking rate greater than 80 mm/s, or has a median or mean horizontal wicking rate greater than 100 mm/s. In some embodiments, a plain weave 1/1 textile with warp density of 72 warps/cm and pick density of 40 picks/cm, comprising filament yarn, or spun yarn, or blended yarn, comprising recombinant protein fibers, is tested using AATCC test method 197-2011, and has a median or mean horizontal wicking rate from 0.1 mm/s to 1 mm/s, or has a median or mean horizontal wicking rate from 1 mm/s to 10 mm/s, or has a median or mean horizontal wicking rate from 10 mm/s to 20 mm/s, or has a median or mean horizontal wicking rate from 20 mm/s to 30 mm/s, or has a median or mean horizontal wicking rate from 30 mm/s to 40 mm/s, or has a median or mean horizontal wicking rate from 40 mm/s to 50 mm/s, or has a median or mean horizontal wicking rate from 50 mm/s to 60 mm/s, or has a median or mean horizontal wicking rate from 60 mm/s to 70 mm/s, or has a median or mean horizontal wicking rate from 70 mm/s to 80 mm/s, or has a median or mean horizontal wicking rate from 80 mm/s to 90 mm/s, or has a median or mean horizontal wicking rate from 90 mm/s to 100 mm/s. Such filament yarns, or spun yarns, or blended yarns are useful in textiles and garments such as skin knits or woven fabrics where wicking of moisture away from the skin is desired, such as active wear apparel. In some embodiments, these filament yarns, or spun yarns, or blended yarns can be constructed into plaited yarn or textile, or double knit textiles. In some embodiments, these textiles are located in a position towards the outer surface of a textile and/or garment to allow the absorbed moisture to easily evaporate.

Engineering Yarn RPF Antimicrobial Properties

In some embodiments, the recombinant protein fibers comprising the filament yarn, or spun yarn, or blended yarn are antimicrobial. AATCC test method 100-2012 can be used to evaluate the antimicrobial properties of a textile material. In this test method, textile samples are inoculated with bacteria, incubated for a specified amount of time under specified conditions, and then the cultures are counted. The results are typically reported as a CFU number (colony forming units) per sample. In some embodiments, a textile, comprising filament yarn, or spun yarn, or blended yarn, comprising recombinant protein fibers, is tested using AATCC test method 100-2012, and has an increase in colony forming units less than 100 times in 24 hours, or has a change in colony forming units from a 100 times reduction to a 10000 times increase in 24 hours, or has a decrease in colony forming unit greater than or equal to 10 times in 24 hours, or has a decrease in colony forming units greater than or equal to 50 times in 24 hours, or has a decrease in colony forming units greater than or equal to 100 times in 24 hours, or has an increase in colony forming units less than 500 times in 24 hours, or has an increase in colony forming units less than 1000 times in 24 hours, or has an increase in colony forming units less than 5000 times in 24 hours, or has an increase in colony forming units less than 10000 times in 24 hours. In some embodiments, a textile, comprising filament yarn, or spun yarn, or blended yarn, comprising recombinant protein fibers, is tested using AATCC test method 100-2012, and has a decrease in colony forming units from 1 times to 10 times in 24 hours, or has an increase in colony forming units from 10 times to 100 times in 24 hours, or, or has an increase in colony forming units from 100 times to 500 times in 24 hours, or has an increase in colony forming units from 500 times to 1000 times in 24 hours, or has an increase in colony forming units from 1000 times to 5000 times in 24 hours, or has an increase in colony forming units from 5000 times to 10000 times in 24 hours. Such filament yarns, or spun yarns, or blended yarns are useful in many textiles and garments, such as active wear apparel which tend to retain odors after wearing during exercise.

Engineering Yarn RPF Cross-Section

In some embodiments, the recombinant protein fibers comprising the filament yarn, or spun yarn, or blended yarn have a longitudinal axis, an inner surface and an outer surface, the inner surface defining a hollow core parallel to the longitudinal axis of the fiber. In some embodiments, the recombinant protein fibers comprising the filament yarn, or spun yarn, or blended yarn have a longitudinal axis and an outer surface, the outer surface including a plurality of corrugations, each corrugation of the plurality parallel or substantially parallel to the longitudinal axis of the fiber. By substantially parallel, we mean an angular deviation between a line defining the longitudinal fiber axis and a line defining the axis of corrugation of less than 25° or less than 20° or less than 15° or less than 10° or less than 5°. In some embodiments, the recombinant protein fibers comprising the filament yarn, or spun yarn, or blended yarn have a longitudinal axis and cross-sectional shape transverse to the longitudinal axis that is substantially circular. In some embodiments, the recombinant protein fibers comprising the filament yarn, or spun yarn, or blended yarn have a longitudinal axis and cross-sectional shape transverse to the longitudinal axis that is substantially triangular. In some embodiments, the recombinant protein fibers comprising the filament yarn, or spun yarn, or blended yarn have a longitudinal axis and cross-sectional shape transverse to the longitudinal axis that is substantially bilobal. In some embodiments, the recombinant protein fibers comprising the filament yarn, or spun yarn, or blended yarn have a longitudinal axis and cross-sectional shape transverse to the longitudinal axis that is substantially trilobal. In some embodiments, the recombinant protein fibers comprising the filament yarn, or spun yarn, or blended yarn have a longitudinal axis and cross-sectional shape transverse to the longitudinal axis that is substantially ovular. In some embodiments, the recombinant protein fibers comprising the filament yarn, or spun yarn, or blended yarn have a longitudinal axis and cross-sectional shape transverse to the longitudinal axis that is substantially c-shaped.

Surface area to volume ratios are relatively small when the fiber has a smooth surface and a circular cross-section. In some embodiments, the recombinant protein fibers comprising the filament yarn, or spun yarn, or blended yarn have a surface area to volume ratio greater than 1000 $cm^{-1}$, or from 1000 to $3\times10^5$ $cm^{-1}$, or greater than $1\times10^4$ $cm^{-1}$, or greater than $1\times10^5$ $cm^{-1}$. Surface area to volume ratios can be substantially larger when the fiber has a rough surface and/or a non-circular cross-section, for instance if the fiber is striated. In some embodiments, the recombinant protein fibers comprising the filament yarn, or spun yarn, or blended yarn have a surface area to volume ratio from 1000 to $3\times10^7$ $cm^{-1}$, or greater than $1\times10^6$ $cm^{-1}$, or greater than $1\times10^7$ $cm^{-1}$. Fibers with high surface area to volume ratios could be useful in biomedical applications, filters, and garments.

Engineering Yarn Linear Density and Mechanical Properties

In some embodiments, a filament yarn, or spun yarn, or blended yarn comprises recombinant protein fibers, wherein the median or mean properties of the filament yarn, or spun yarn, or blended yarn comprise a linear density of less than 10000 denier, or is less than 8000 denier, or is less than 6000 denier, or is less than 4000 denier, or is less than 3000 denier, or is less than 2000 denier, or is less than 1000 denier, or is less than 500 denier, or is less than 100, or is less than 75, or is less than 50, or is less than 35, or is less than 20, or is less than 10, or is from 10 to 50 denier, or is from 10 to 100 denier, or is from 10 to 500 denier, or is from 10 to 1000 denier, or is from 10 to 10000 denier, or is from 50 to 10000 denier, or is from 100 to 10000 denier, or from 100 to 5000 denier, or from 500 to 5000 denier, or from 700 to 4300 denier, or from 500 to 4500 denier, or from 2000 to 4500 denier.

In some embodiments, a filament yarn, or spun yarn, or blended yarn comprises recombinant protein fibers, wherein the median or mean properties of the filament yarn, or spun yarn, or blended yarn comprise an initial modulus greater than 50 cN/tex, or greater than 115 cN/tex, or greater than 200 cN/tex, or greater than 400 cN/tex, or greater than 550 cN/tex, or greater than 600 cN/tex, or greater than 800 cN/tex, or greater than 1000 cN/tex, or greater than 2000 cN/tex, or greater than 3000 cN/tex, or greater than 4000 cN/tex, or greater than 5000 cN/tex, and a maximum tensile strength greater than 0.5 cN/tex, or greater than 1 cN/tex, or greater than 2 cN/tex, or greater than 4 cN/tex, or greater than 6 cN/tex, or greater than 7.7 cN/tex, or greater than 10 cN/tex, or greater than 15 cN/tex, or greater than 20 cN/tex, or greater than 25 cN/tex, or greater than 30 cN/tex, or greater than 40 cN/tex, or greater than 50 cN/tex, or greater than 60 cN/tex, or greater than 70 cN/tex, or greater than 80 cN/tex, or greater than 90 cN/tex, or greater than 100 cN/tex, or from 0.5 to 30 cN/tex, or from 0.5 to 25 cN/tex, or from 0.5 to 20 cN/tex, or from 0.5 to 10 cN/tex, or from 5 to 30 cN/tex, or from 5 to 25 cN/tex, or from 10 to 30 cN/tex, or from 10 to 20 cN/tex, or from 15 to 20 cN/tex, or from 15 to 50 cN/tex, or from 15 to 75 cN/tex, or from 15 to 100 cN/tex, and an extensibility of at least 1%, or greater than 2%, or greater than 5%, or greater than 10%, or greater than 20%, or greater than 30%, or greater than 50%, or greater than 100%, or greater than 200%, or greater than 300%, or from 0.5% to 50%, or from 0.5% to 35%, or from 0.5% to 30%, or from 0.5% to 25%, or from 0.5% to 20%, or from 0.5% to 15%, or from 0.5% to 5%, or from 0.5% to 3%. In some embodiments, a filament yarn, or spun yarn, or blended yarn comprises recombinant protein fibers, wherein the median or mean properties of the filament yarn, or spun yarn, or blended yarn comprise an initial modulus from 100 to 7000 cN/tex, or from 500 to 7000 cN/tex, or from 50 to 7000 cN/tex, or from 100 to 5000 cN/tex, or from 500 to 5000 cN/tex, or from 50 to 5000 cN/tex, or from 100 to 2000 cN/tex, or from 500 to 2000 cN/tex, or from 50 to 2000 cN/tex, or from 100 to 1000 cN/tex, or from 500 to 1000 cN/tex, or from 50 to 1000 cN/tex, or from 50 to 500 cN/tex, or from 100 to 1000 cN/tex, or from 500 to 1000 cN/tex, or from 100 to 700 cN/tex, and a maximum tensile strength from 0.5 to 100 cN/tex, or from 5 to 100 cN/tex, or from 5 to 50 cN/tex, and an extensibility from 10 to 300 %, or from 10 to 100%, or from 10 to 50%. Such filament yarns, or spun yarns, or blended yarns are useful in a myriad of applications, such as construction into ropes, textiles and garments, upholstery or linens.

In some embodiments, a filament yarn, or spun yarn, or blended yarn comprises recombinant protein fibers, wherein the filament yarn, or spun yarn, or blended yarn comprise a mean or median extensibility greater than 1%, or greater than 5%, or greater than 10%, or greater than 20%, or greater than 100%, or greater than 200%, or greater than 300%, or greater than 400%. In embodiments, the synthesized fibers making up the filament yarn, or spun yarn, or blended yarn exhibit an extensibility (i.e., strain to fracture) of from 1% to 400%, or from 1 to 200%, or from 1 to 100%, or from 1 to 20%, or from 10 to 200%, or from 10 to 100%, or from 10 to 50%, or from 10 to 20%, or from 50% to 150%, or from 100% to 150%, or from 300% to 400%.

In some embodiments, a filament yarn, or spun yarn, or blended yarn comprises recombinant protein fibers, wherein the filament yarn, or spun yarn, or blended yarn comprise a mean or median maximum tensile strength greater than 0.5 cN/tex, or greater than 1 cN/tex, or greater than 2 cN/tex, or greater than 5 cN/tex, or greater than 10 cN/tex, or greater than 15 cN/tex, or greater than 20 cN/tex, or greater than 25 cN/tex, or from 0.5 to 30 cN/tex, or from 0.5 to 25 cN/tex, or from 0.5 to 20 cN/tex, or from 0.5 to 10 cN/tex, or from 5 to 30 cN/tex, or from 5 to 25 cN/tex, or from 10 to 30 cN/tex, or from 10 to 20 cN/tex, or from 15 to 20 cN/tex, or from 15 to 50 cN/tex, or from 15 to 75 cN/tex, or from 15 to 100 cN/tex, or from 1 to 100 cN/tex.

In some embodiments, a filament yarn, or spun yarn, or blended yarn comprises recombinant protein fibers, wherein the filament yarn, or spun yarn, or blended yarn comprise a mean or median initial modulus greater than 1500 MPa, or greater than 2000 MPa, or greater than 3000 MPa, or greater than 5000 MPa, or greater than 6000 MPa, or greater than 7000 MPa, or from 5200 to 7000 MPa, or from 1500 to 10000 MPa, or from 1500 to 8000 MPa, or from 2000 to 8000 MPa, or from 3000 to 8000 MPa, or from 5000 to 8000 MPa, or from 5000 to 6000 MPa, or from 6000 to 8000 MPa.

In some embodiments, a filament yarn, or spun yarn, or blended yarn comprises recombinant protein fibers, wherein the filament yarn, or spun yarn, or blended yarn comprise a mean or median toughness greater than 2 cN/tex, or from 0.5 to 70 cN/tex, or greater than 3 cN/tex, or greater than 4 cN/tex, or greater than 5 cN/tex, or greater than 7.5 cN/tex, or greater than 10 cN/tex, or greater than 20 cN/tex, or greater than 30 cN/tex, or greater than 40 cN/tex, or greater than 50 cN/tex, greater than 60 cN/tex, or greater than 70 cN/tex, or from 2 to 3 cN/tex, or from 3 to 4 cN/tex, or from 4 to 5 cN/tex, or from 5 to 7.5 cN/tex, or from 7.5 to 10 cN/tex, or from 10 to 20 cN/tex, or from 20 to 30 cN/tex, or from 30 to 40 cN/tex, or from 40 to 50 cN/tex, or from 50 to 60 cN/tex, or from 60 to 70 cN/tex.

Different degrees of twist can be applied to yarns, which will give different mechanical properties to the yarn. Generally, the higher the twist angle (or higher number of turns per centimeter) of a spun yarn, the higher the fiber strength but the lower the fiber modulus. However, above a certain degree of twist the fiber strength can decrease. The degree of twist in spun yarns ranges from about 5 turns per centimeter (TPC) for low twist up to about 200 TPC for very high twist. In some embodiments, the filament, spun, or blended yarn comprising recombinant protein fibers has a number of turns per centimeter greater than 30. In some embodiments, the filament, spun, or blended yarn comprising recombinant protein fibers has a number of turns per centimeter from 15 to 200. In some embodiments, the filament, spun, or blended yarn comprising recombinant protein fibers has a number of turns per centimeter greater than 15, or greater than 50, or greater than 100, or greater than 150, or greater than 200. In some embodiments, the filament, spun, or blended yarn comprising recombinant protein fibers has greater than 2 cN/tex strength, and greater than 30 cN/tex modulus, and a number of turns per centimeter greater than 15, or a number of turns per centimeter greater than 30, or a number of turns per centimeter greater than 50, or a number of turns per centimeter greater than 100, or a number of turns per centimeter greater than 150, or a number of turns per centimeter greater than 200. In some embodiments, the filament, spun, or blended yarn comprising recombinant protein fibers has greater than 2 cN/tex strength, and a number of turns per centimeter greater than 30, and greater than 30 cN/tex modulus, or greater than 50 cN/tex modulus, or greater than 100 cN/tex modulus, or greater than 150 cN/tex modulus. In some embodiments, the filament, spun, or blended yarn comprising recombinant protein fibers has a number of turns per centimeter greater than 30, and greater than 30 cN/tex modulus, and greater than 2 cN/tex strength, or greater than 4 cN/tex strength, or greater than 8 cN/tex strength, or greater than 10 cN/tex strength, or greater than 15 cN/tex strength, or greater than 20 cN/tex strength, or greater than 25 cN/tex strength, or greater than 30 cN/tex strength, or greater than 40 cN/tex strength, or greater than 60 cN/tex strength. In an embodiment, a spun, or blended that is ring spun comprising recombinant protein fibers and a twist of about 150 per centimeter would be very strong. All of the disclosed twists can be either of the "S" type or "Z" type.

Engineering Yarn Twist

In some embodiments, filament yarns containing recombinant protein fibers are twisted. Filament yarns containing recombinant protein fibers can be twisted to form either a Z-twist (twisted in the counterclockwise direction), or an S-twist (twisted in the clockwise direction).

Recombinant protein fiber filament yarns fall into two main classes, flat and textured. Textured yarns comprising recombinant protein fibers have noticeably greater apparent volume than a conventional flat yarn of the same fiber, count and linear density.

In some embodiments, two or more filament yarns, spun yarns or blended yarns (e.g., blended spun yarns) comprising recombinant protein fibers can be twisted with each other to form a plied or multiple or folded filament yarn, spun yarn or blended yarn. The filament yarns, spun yarns or blended yarns making up the plied or multiple filament yarn, spun yarn or blended yarn may be twisted in an "S" (twisted in a clockwise direction) or "Z" (twisted in a counterclockwise direction) direction in order to produce different yarn characters. In some cases, the direction of the twist in such a plied or multiple filament yarn, spun yarn or blended yarn is the opposite of the direction of the twist of the filament yarns, spun yarns or blended yarns that make up the plied or multiple filament yarn, spun yarn or blended yarn. Two or more plied yarns, when twisted together, are sometimes referred to as a cabled yarn. In this disclosure "filament yarns," "spun yarns" or "blended yarns" can refer to single yarns, plied yarns, multiple yarns, or cabled yarns.

In some embodiments, the filament yarn, or spun yarn, or blended yarn comprises recombinant protein fibers, and has a twist of more than 5 turns per centimeter, or more than 10 turns per centimeter, or more than 15 turns per centimeter, or more than 20 turns per centimeter, or more than 25 turns per centimeter, or more than 30 turns per centimeter, or more than 35 turns per centimeter, or more than 40 turns per centimeter, or more than 50 turns per centimeter, or more than 60 turns per centimeter, or more than 70 turns per centimeter, or more than 80 turns per centimeter, or more than 90 turns per centimeter, or more than 100 turns per centimeter, or from 5 to 200 turns per centimeter, or from 5 to 100 turns per centimeter, or from 5 to 50 turns per centimeter, or from 10 to 100 turns per centimeter, or from 10 to 50 turns per centimeter, or from 20 to 100 turns per centimeter.

RECOMBINANT PROTEIN FIBER BLENDED YARN EMBODIMENTS

In some embodiments, the blended yarn comprises at least 10% RPFs, or at least 20% RPFs, or at least 30% RPFs, or at least 40% RPFs, or at least 50% RPFs, or at least 60% RPFs, or at least 70% RPFs, or at least 80% RPFs, or at least 90% RPFs, or from 10% to 90% RPFs, or from 20% to 80% RPFs, or from 30% to 70% RPFs, or from 40 to 60% RPFs. All blended yarns described in this disclosure can have any of the above fractions of RPFs. Such blended RPF yarns are also useful in a myriad of applications, such as construction into ropes, textiles and garments, upholstery or linens.

In some embodiments, the RPFs and non-RPFs are dyed different colors to create a blended yarn with different colored fibers. In some embodiments the RPFs are dyed, and the non-recombinant protein fibers are not dyed. In some embodiments, the RPFs are not dyed and the non-recombinant protein fibers are dyed. In some embodiments, the RPFs and non-RPFs uptake dye to a different degree of depth.

In some embodiments, RPFs are blended with cotton fibers. In some embodiments, the RPF/cotton blended yarn comprises at least 10% RPFs, or at least 20% RPFs, or at least 30% RPFs, or at least 40% RPFs, or at least 50% RPFs, or at least 60% RPFs, or at least 70% RPFs, or at least 80% RPFs, or at least 90% RPFs, or from 10% to 90% RPFs, or from 20% to 80% RPFs, or from 30% to 70% RPFs, or from 40 to 60% RPFs. The cotton contributes a soft feel, comfort next to skin and/or dulls the luster of the textile. As described in this disclosure, RPFs can be engineered to have smooth surfaces, and therefore brings increased luster to the blended RPF/cotton yarn. As described in this disclosure, RPFs can be engineered to have small diameters, which increases the softness, and therefore brings improved softness to the blended RPF/cotton yarn. Applications for this blended yarn include sportswear garments, to give the cotton a more luxurious hand and appeal. In some embodiments, the color of the yarn is also heathered if only one of the fibers is dyed the RPFs and cotton fibers uptake dye to a different degree of depth. The ratio of RPFs to other types of fibers in this embodiment is at least 10% RPFs by weight, or at least 20% RPFs weight, or at least 30% RPFs weight, or at least 40% RPFs weight, or at least 50% RPFs by weight, or at least 60% RPFs by weight, or at least 70% RPFs, or at least 80% RPFs weight, or at least 90% RPFs weight, or from 1 to 99% by weight, or from 30 to 70% by weight, or from 1 to 10% by weight, or from 1 to 20% by weight, or from 1 to 30% by weight, or from 40 to 60% by weight, or from 70 to 99% by weight, or from 80 to 99% by weight, or from 90 to 99% by weight.

In some embodiments, RPFs are blended with wool fibers. In some embodiments, the RPF/wool blended yarn comprises at least 10% RPFs, or at least 20% RPFs, or at least 30% RPFs, or at least 40% RPFs, or at least 50% RPFs, or at least 60% RPFs, or at least 70% RPFs, or at least 80% RPFs, or at least 90% RPFs, or from 10% to 90% RPFs, or from 20% to 80% RPFs, or from 30% to 70% RPFs, or from 40 to 60% RPFs. The wool fibers in this embodiment can be obtained from sheep as well as other animals, including but not limited to cashmere from goats, mohair from goats, qiviut from muskoxen, angora from rabbits, and other types of wool from camelids. The wool imparts warmth to the yarn. When formed into fabric and a garment, the wool moderates the body temperature, keeping the wearer warm in cold conditions and cooler in hot conditions. Not to be limited by theory, this is due to the hollow core structure of the wool fiber providing dead air space, which acts as thermal insulation. As described in this disclosure, RPFs can be engineered to have small diameters, which increases the drape of a fabric comprising the RPFs, and therefore brings improved drape to the blended RPF/wool fabric. As described in this disclosure, RPFs can be engineered to have smooth surfaces, and therefore brings increased luster to the blended RPF/wool yarn. As described in this disclosure, RPFs can be engineered to have small diameters, which increases the softness, and therefore brings improved softness to the blended RPF/wool yarn. As described in this disclosure, RPFs can be engineered to be hydrophilic, and therefore brings wickability to the yarn. The softness imparted by the RPF would make a resulting fabric and/or garment made from this blended yarn more comfortable, which would make this kind of blend particularly useful for garments worn next to the skin. The ratio of RPFs to other types of fibers in this embodiment is at least 10% RPFs by weight, or at least 20% RPFs weight, or at least 30% RPFs weight, or at least 40% RPFs weight, or at least 50% RPFs by weight, or at least 60% RPFs by weight, or at least 70% RPFs, or at least 80% RPFs weight, or at least 90% RPFs weight, or from 1 to 99% by weight, or from 30 to 70% by weight, or from 1 to 10% by weight, or from 1 to 20% by weight, or from 1 to 30% by weight, or from 40 to 60% by weight, or from 70 to 99% by weight, or from 80 to 99% by weight, or from 90 to 99% by weight.

In some embodiments, RPFs are blended with polyamide fibers. In some embodiments, the RPF/polyamide blended yarn comprises at least 10% RPFs, or at least 20% RPFs, or at least 30% RPFs, or at least 40% RPFs, or at least 50% RPFs, or at least 60% RPFs, or at least 70% RPFs, or at least 80% RPFs, or at least 90% RPFs, or from 10% to 90% RPFs, or from 20% to 80% RPFs, or from 30% to 70% RPFs, or from 40 to 60% RPFs. The polyamide fibers contribute strength and abrasion resistance to the yarn. As described in this disclosure, RPFs can be engineered to have improved hydrophilicity, moisture absorption and wickability, and therefore brings increased hydrophilicity, moisture absorption and wickability to the blended RPF/polyamide yarn. As described in this disclosure, RPFs can be engineered to have small diameters, which increases the softness, and therefore brings improved softness to the blended RPF/polyamide yarn. The color of the yarn could also be modified in an RPF/polyamide blend, and a melange dyeing effect could be created. One application where this blend is useful is in a sock because it would improve abrasion resistance of the heel and toe, while being comfortable next to the skin and possessing good moisture-related properties. The ratio of RPFs to other types of fibers in this embodiment is at least 10% RPFs by weight, or at least 20% RPFs weight, or at least 30% RPFs weight, or at least 40% RPFs weight, or at least 50% RPFs by weight, or at least 60% RPFs by weight, or at least 70% RPFs, or at least 80% RPFs weight, or at least 90% RPFs weight, or from 1 to 99% by weight, or from 30 to 70% by weight, or from 1 to 10% by weight, or from 1 to 20% by weight, or from 1 to 30% by weight, or from 40 to 60% by weight, or from 70 to 99% by weight, or from 80 to 99% by weight, or from 90 to 99% by weight.

In some embodiments, RPFs are blended with wool and acrylic fibers. In some embodiments, the RPF/wool/acrylic blended yarn comprises at least 10% RPFs, or at least 20% RPFs, or at least 30% RPFs, or at least 40% RPFs, or at least 50% RPFs, or at least 60% RPFs, or at least 70% RPFs, or at least 80% RPFs, or at least 90% RPFs, or from 10% to 90% RPFs, or from 20% to 80% RPFs, or from 30% to 70% RPFs, or from 40 to 60% RPFs. The RPF and the wool fibers in this blend contribute all of the attributes described in the RPF/wool blend in this disclosure, and additionally acrylic contributes additional softness and bulk. Furthermore, the acrylic would reduce the cost compared to using all wool as the other fiber blended with RPFs. The ratio of RPFs to other types of fibers in this embodiment is at least 10% RPFs by weight, or at least 20% RPFs weight, or at least 30% RPFs weight, or at least 40% RPFs weight, or at least 50% RPFs by weight, or at least 60% RPFs by weight, or at least 70% RPFs, or at least 80% RPFs weight, or at least 90% RPFs weight, or from 1 to 99% by weight, or from 30 to 70% by weight, or from 1 to 10% by weight, or from 1 to 20% by weight, or from 1 to 30% by weight, or from 40 to 60% by weight, or from 70 to 99% by weight, or from 80 to 99% by weight, or from 90 to 99% by weight.

In some embodiments, RPFs are blended with wool and nylon fibers. In some embodiments, the RPF/wool/nylon blended yarn comprises at least 10% RPFs, or at least 20% RPFs, or at least 30% RPFs, or at least 40% RPFs, or at least 50% RPFs, or at least 60% RPFs, or at least 70% RPFs, or at least 80% RPFs, or at least 90% RPFs, or from 10% to 90% RPFs, or from 20% to 80% RPFs, or from 30% to 70% RPFs, or from 40 to 60% RPFs. The RPF and the wool fibers in this blend contribute all of the attributes described in the RPF/wool blend in this disclosure, and additionally the nylon contributes strength to the yarn. One application where this blend is useful is in a sock because it would improve abrasion resistance of the heel and toe, while being comfortable next to the skin and possessing good moisture-related properties. The ratio of RPFs to other types of fibers in this embodiment is at least 10% RPFs by weight, or at least 20% RPFs weight, or at least 30% RPFs weight, or at least 40% RPFs weight, or at least 50% RPFs by weight, or at least 60% RPFs by weight, or at least 70% RPFs, or at least 80% RPFs weight, or at least 90% RPFs weight, or from 1 to 99% by weight, or from 30 to 70% by weight, or from 1 to 10% by weight, or from 1 to 20% by weight, or from 1 to 30% by weight, or from 40 to 60% by weight, or from 70 to 99% by weight, or from 80 to 99% by weight, or from 90 to 99% by weight.

In some embodiments, RPFs can be blended with linen fibers. In some embodiments, the RPF/linen blended yarn comprises at least 10% RPFs, or at least 20% RPFs, or at least 30% RPFs, or at least 40% RPFs, or at least 50% RPFs, or at least 60% RPFs, or at least 70% RPFs, or at least 80% RPFs, or at least 90% RPFs, or from 10% to 90% RPFs, or from 20% to 80% RPFs, or from 30% to 70% RPFs, or from 40 to 60% RPFs. As described in this disclosure, RPFs can be engineered to have small diameters, which increases the drape of a fabric comprising the RPFs, and therefore brings improved drape to the blended RPF/linen fabric. As described in this disclosure, RPFs can be engineered to have smooth surfaces, and therefore brings increased luster to the blended RPF/linen yarn. As described in this disclosure, RPFs can be engineered to have small diameters, which increases the softness, and therefore brings improved softness to the blended RPF/linen yarn. The inclusion of linen in this blended yarn would change the hand of the yarn for aesthetic purposes, and make it more comfortable next to skin. The ratio of RPFs to other types of fibers in this embodiment is at least 10% RPFs by weight, or at least 20% RPFs weight, or at least 30% RPFs weight, or at least 40% RPFs weight, or at least 50% RPFs by weight, or at least 60% RPFs by weight, or at least 70% RPFs, or at least 80% RPFs weight, or at least 90% RPFs weight, or from 1 to 99% by weight, or from 30 to 70% by weight, or from 1 to 10% by weight, or from 1 to 20% by weight, or from 1 to 30% by weight, or from 40 to 60% by weight, or from 70 to 99% by weight, or from 80 to 99% by weight, or from 90 to 99% by weight.

In some embodiments, RPFs can be blended with cotton and linen fibers. In some embodiments, the RPF/cotton/linen blended yarn comprises at least 10% RPFs, or at least 20% RPFs, or at least 30% RPFs, or at least 40% RPFs, or at least 50% RPFs, or at least 60% RPFs, or at least 70% RPFs, or at least 80% RPFs, or at least 90% RPFs, or from 10% to 90% RPFs, or from 20% to 80% RPFs, or from 30% to 70% RPFs, or from 40 to 60% RPFs. The RPF and the cotton fibers in this blend contribute all of the attributes described in the RPF/cotton blend in this disclosure, and additionally the linen contributes strength, soft feel, and/or comfort to the yarn. The inclusion of linen in this blended yarn would change the hand of the yarn for aesthetic purposes, and make it more comfortable next to skin. The ratio of RPFs to other types of fibers in this embodiment is at least 10% RPFs by weight, or at least 20% RPFs weight, or at least 30% RPFs weight, or at least 40% RPFs weight, or at least 50% RPFs by weight, or at least 60% RPFs by weight, or at least 70% RPFs, or at least 80% RPFs weight, or at least 90% RPFs weight, or from 1 to 99% by weight, or from 30 to 70% by weight, or from 1 to 10% by weight, or from 1 to 20% by weight, or from 1 to 30% by weight, or from 40 to 60% by weight, or from 70 to 99% by weight, or from 80 to 99% by weight, or from 90 to 99% by weight.

In some embodiments, RPFs are blended with cotton and nylon. In some embodiments, the RPF/cotton/nylon blended yarn comprises at least 10% RPFs, or at least 20% RPFs, or at least 30% RPFs, or at least 40% RPFs, or at least 50% RPFs, or at least 60% RPFs, or at least 70% RPFs, or at least 80% RPFs, or at least 90% RPFs, or from 10% to 90% RPFs, or from 20% to 80% RPFs, or from 30% to 70% RPFs, or from 40 to 60% RPFs. The RPF and the cotton fibers in this blend contribute all of the attributes described in the RPF/cotton blend in this disclosure, and additionally the nylon contributes strength to the yarn. One application where this blend is useful is in a sock because it would improve abrasion resistance of the heel and toe, while being comfortable next to the skin and possessing good moisture-related properties. The ratio of RPFs to other types of fibers in this embodiment is at least 10% RPFs by weight, or at least 20% RPFs weight, or at least 30% RPFs weight, or at least 40% RPFs weight, or at least 50% RPFs by weight, or at least 60% RPFs by weight, or at least 70% RPFs, or at least 80% RPFs weight, or at least 90% RPFs weight, or from 1 to 99% by weight, or from 30 to 70% by weight, or from 1 to 10% by weight, or from 1 to 20% by weight, or from 1 to 30% by weight, or from 40 to 60% by weight, or from 70 to 99% by weight, or from 80 to 99% by weight, or from 90 to 99% by weight.

In some embodiments, RPFs are blended with acrylic and polyamide fibers. In some embodiments, the RPF/acrylic/polyamide blended yarn comprises at least 10% RPFs, or at least 20% RPFs, or at least 30% RPFs, or at least 40% RPFs, or at least 50% RPFs, or at least 60% RPFs, or at least 70% RPFs, or at least 80% RPFs, or at least 90% RPFs, or from 10% to 90% RPFs, or from 20% to 80% RPFs, or from 30% to 70% RPFs, or from 40 to 60% RPFs. As described in this disclosure, RPFs can be engineered to have small diameters, which increases the softness, and therefore brings improved softness to the blended RPF/acrylic/polyamide yarn. As described in this disclosure, RPFs can be engineered to have improved hydrophilicity, moisture absorption and wickability, and therefore brings increased hydrophilicity, moisture absorption and wickability to the blended RPF/polyamide yarn. The polyamide fibers contribute strength and abrasion resistance to the yarn. The acrylic would give the yarn bulk and softness. One application where this blend is useful is in a sock because it would improve abrasion resistance of the heel and toe, while being comfortable next to the skin and possessing good moisture-related properties. The ratio of RPFs to other types of fibers in this embodiment is at least 10% RPFs by weight, or at least 20% RPFs weight, or at least 30% RPFs weight, or at least 40% RPFs weight, or at least 50% RPFs by weight, or at least 60% RPFs by weight, or at least 70% RPFs, or at least 80% RPFs weight, or at least 90% RPFs weight, or from 1 to 99% by weight, or from 30 to 70% by weight, or from 1 to 10% by weight, or from 1 to 20% by weight, or from 1 to 30% by weight, or from 40 to 60% by weight, or from 70 to 99% by weight, or from 80 to 99% by weight, or from 90 to 99% by weight.

In some embodiments, RPFs are blended with polyester fibers. In some embodiments, the RPF/polyester blended yarn comprises at least 10% RPFs, or at least 20% RPFs, or at least 30% RPFs, or at least 40% RPFs, or at least 50% RPFs, or at least 60% RPFs, or at least 70% RPFs, or at least 80% RPFs, or at least 90% RPFs, or from 10% to 90% RPFs, or from 20% to 80% RPFs, or from 30% to 70% RPFs, or from 40 to 60% RPFs. The polyester fibers contribute strength, improve drying time, and manage moisture in the yarn. As described in this disclosure, RPFs can be engineered to have improved hydrophilicity, moisture absorption and wickability, and therefore brings increased hydrophilicity, moisture absorption and wickability to the blended RPF/polyamide yarn. The color of the yarn could also be modified in an RPF/polyester blend, and a melange dyeing effect could be created. One application where this blend is useful is in a sock because it would improve abrasion resistance of the heel and toe, while being comfortable next to the skin and possessing good moisture-related properties. The ratio of RPFs to other types of fibers in this embodiment is at least 10% RPFs by weight, or at least 20% RPFs weight, or at least 30% RPFs weight, or at least 40% RPFs weight, or at least 50% RPFs by weight, or at least 60% RPFs by weight, or at least 70% RPFs, or at least 80% RPFs weight, or at least 90% RPFs weight, or from 1 to 99% by weight, or from 30 to 70% by weight, or from 1 to 10% by weight, or from 1 to 20% by weight, or from 1 to 30% by weight, or from 40 to 60% by weight, or from 70 to 99% by weight, or from 80 to 99% by weight, or from 90 to 99% by weight.

RECOMBINANT PROTEIN FIBER TEXTILE EMBODIMENTS

In some embodiments, a knitted, woven, or non-woven textile is constructed from filament yarn, or spun yarn, or blended yarn comprising recombinant protein fibers with properties described in the present disclosure. Knitted, woven, or non-woven textiles can be made from filament yarn, or spun yarn, or blended yarn containing recombinant protein fibers with one or more of the mechanical properties, physical properties, chemical properties and biological properties described in the present disclosure.

In some embodiments, a knitted textile is constructed comprising the filament yarn, or spun yarn, or blended yarn comprising recombinant protein fibers. Some examples of knitted textiles comprising yarns comprising recombinant protein fibers are circular-knitted textiles, flat-knitted textiles, and warp-knitted textiles. There are many more examples of knitted textiles comprising recombinant protein fibers within these major examples.

In some embodiments, a woven textile is constructed comprising the filament yarn, or spun yarn, or blended yarn comprising recombinant protein fibers. Some examples of woven textiles comprising yarns comprising recombinant protein fibers are plain weave textiles, dobby weave textiles, and jacquard weave textiles. There are many more examples of woven textiles comprising recombinant protein fibers within these major examples.

In some embodiments, a non-woven textile is constructed comprising the filament yarn, or spun yarn, or blended yarn comprising recombinant protein fibers. Some examples of non-woven textiles comprising yarns comprising recombinant protein fibers are needle punched textiles, spunlace textiles, wet-laid textiles, dry-laid textiles, melt-blown textiles, and 3-D printed non-woven textiles. There are many more examples of non-woven textiles comprising recombinant protein fibers within the major examples.

In some embodiments, the woven, knitted or non-woven textile contains filament, spun or blended yarns that contain recombinant protein fibers with mechanical properties such as high initial modulus, high extensibility, high tenacity, and high toughness. The woven textile can also contain filament yarns that can contain recombinant protein fibers with structural properties such as high fineness (e.g., small diameter, low linear density, low denier), high softness, smoothness, engineered cross-section shapes and porosity. The woven textile can also contain filament, spun or blended yarns that can contain recombinant protein fibers with chemical properties such as hydrophilicity. The woven textile can also contain filament, spun or blended yarns that can contain recombinant protein fibers with biological properties such as being antimicrobial.

Fabrics constructed from flat filament yarns comprising recombinant protein fibers will have larger interstices than fabrics constructed from textured yarns. Textiles constructed from textured filament yarns comprising recombinant protein fibers have better coverage since the bulk of the yarn fills the interstices between stitches or picks. Fabrics constructed from textured filament yarns comprising recombinant protein fibers therefore tend to have a lower luster, be more natural in hand, and be softer. Textiles constructed from filament yarns comprising recombinant protein fibers are used in many applications including carpeting and carpet backing, industrial textile products (such as tire cord and tire fabric, seat belts, industrial webbing and tape, tents, fishing line and nets, rope, and tape reinforcement), apparel fabrics (such as women's sheer hosiery, underwear, nightwear, sports apparel, anklets and socks), and interior and household products (such as bed ticking, furniture upholstery, curtains, bedspreads, sheets, and draperies).

Since the yarns produced from different types of recombinant protein fibers and different spinning methods have different properties, the textiles produced from these different yarns also have different properties. For instance, textiles produced from fully twisted ring-spun yarns formed from recombinant protein fibers, which have higher twist at yarn periphery, have higher tensile strength but lower abrasion resistance than textiles produced from open-end spun yarns formed from recombinant protein fibers. In contrast, textiles produced from open-end spun yarns formed from recombinant protein fibers, have higher twist at the yarn core than the periphery, have lower strength and higher abrasion resistance than textiles formed from ring-spun yarns formed from recombinant protein fibers. Air-jet spun yarns formed from recombinant protein fibers, which have genuine twist of the fibers at the yarn sheath, have very low hairiness, providing a textile with good resistance to wear, abrasion and piling.

In some embodiments, a textile is constructed comprising filament yarn, or spun yarn, or blended yarn comprising recombinant protein fibers, wherein the textile has a high maximum tenacity. The strength of textile samples is reported as the tenacity per yarn in the test sample. In some embodiments, a textile comprising filament yarn, or spun yarn, or blended yarn comprising recombinant protein fibers has a median or mean maximum tensile strength greater than 7.7 cN/tex per yarn, or from 0.5 to 150 cN/tex per yarn, or greater than 0.5 cN/tex per yarn, or a median or mean maximum tensile strength greater than 1 cN/tex per yarn, or a median or mean maximum tensile strength greater than 2 cN/tex per yarn, or a median or mean maximum tensile strength greater than 4 cN/tex per yarn, or a median or mean maximum tensile strength greater than 6 cN/tex per yarn, or a median or mean maximum tensile strength greater than 10 cN/tex per yarn, or a median or mean maximum tensile strength greater than 20 cN/tex per yarn, or a median or mean maximum tensile strength greater than 30 cN/tex per yarn, or a median or mean maximum tensile strength greater than 40 cN/tex per yarn, or a median or mean maximum tensile strength greater than 50 cN/tex per yarn, or a median or mean maximum tensile strength greater than 75 cN/tex per yarn, or a median or mean maximum tensile strength greater than 100 cN/tex per yarn, or a median or mean maximum tensile strength greater than 125 cN/tex per yarn, or a median or mean maximum tensile strength greater than 150 cN/tex per yarn. In some embodiments the above textile is knitted, or is woven.

In some embodiments, a lightweight textile can be constructed comprising filament yarn, or spun yarn, or blended yarn comprising recombinant protein fibers, wherein the recombinant protein fibers have a small denier, such as less than 5, as described in this disclosure.

In some embodiments, a highly comfortable textile can be constructed comprising filament yarn, or spun yarn, or blended yarn comprising recombinant protein fibers, wherein the recombinant protein fibers have good moisture absorption properties as discussed in this disclosure, good moisture wicking properties as discussed in this disclosure, and good softness as discussed in this disclosure. In some embodiments, a highly comfortable textile can be constructed comprising filament yarn, or spun yarn, or blended yarn comprising recombinant protein fibers: wherein the recombinant protein fibers have median or mean of greater than 5% diameter change upon immersion in water, the recombinant protein fibers, when constructed into a plain weave 1/1 textile with warp density of 72 warps/cm and pick density of 40 picks/cm, comprising filament yarn, or spun yarn, or blended yarn, comprising recombinant protein fibers, is tested using AATCC test method 197-2011, and has a median or mean horizontal wicking rate greater than 1 mm/s, and the recombinant protein fibers have a median or mean denier less than about 5.

In some embodiments, an ultra-soft textile can be constructed comprising filament yarn, or spun yarn, or blended yarn comprising recombinant protein fibers, wherein the recombinant protein fibers have a small denier, such as less than 5, as described in this disclosure, and the textile has very low flexural rigidity giving it the ability to form a very soft handed fabric.

In some embodiments, the fibers, yarns and/or textiles comprising recombinant protein fibers, achieve the properties described in this disclosure without the use of chemical finishes. In some embodiments, the fibers, yarns and/or textiles comprising recombinant protein fibers, do not comprise an antimicrobial finish, such as brominated phenols, quaternary ammonium compounds, zirconium peroxide, ethylene oxide, organo-silver and/or tin compounds. In some embodiments, the fibers, yarns and/or textiles comprising recombinant protein fibers, do not comprise a luster finish, such as calendaring, beetling and/or burning-out. In some embodiments, the fibers, yarns and/or textiles comprising recombinant protein fibers, do not comprise a drape finish, such as parchmentizing, acid designs, burning-out and/or sizing. In some embodiments, the fibers, yarns and/or textiles comprising recombinant protein fibers, do not comprise a texture finish, such as shearing, brushing, 3D or raised embossing, pleating, flocking, embroidery, expanded foam, and/or napping. In some embodiments, the fibers, yarns and/or textiles comprising recombinant protein fibers, do not comprise a softening finish, such as silicone compounds, emulsified oils, sulphonated oils, and/or waxes. In some embodiments, the fibers, yarns and/or textiles comprising recombinant protein fibers, do not comprise a wrinkle resistant finish, such as formaldehyde, di-methylol urea, di-methylol ethylene urea, di-methylol di-hydroxyl ethylene urea, and/or modified di-methylol di-hydoxyl ethylene urea. In some embodiments, the fibers, yarns and/or textiles comprising recombinant protein fibers, do not comprise a functional finish, such as waterproof finishes (such as with a resin, wax and/or oil), water repellant finishes (such as silicones, fluorocarbons, and/or paraffins), flame retardant finishes (such as tetrakis hydroxymethyl phosphonium chloride), moth proof finishes (such as fluorine compounds, naphthalene, DDT, paradichloro benzene), mildew fungus prevention finishes (such as boric acid), and/or antistatic finishes (such as moisture absorbing films).

In some embodiments, the fibers, yarns and/or textiles comprising recombinant protein fibers, include chemical finishes. In some embodiments, the fibers, yarns and/or textiles comprising recombinant protein fibers, comprise an antimicrobial finish, such as brominated phenols, quaternary ammonium compounds, zirconium peroxide, ethylene oxide, organo-silver and/or tin compounds. In some embodiments, the fibers, yarns and/or textiles comprising recombinant protein fibers, comprise a luster finish, such as calendaring, beetling and/or burning-out. In some embodiments, the fibers, yarns and/or textiles comprising recombinant protein fibers, do not comprise a drape finish, such as parchmentizing, acid designs, burning-out and/or sizing. In some embodiments, the fibers, yarns and/or textiles comprising recombinant protein fibers, comprise a texture finish, such as shearing, brushing, 3D or raised embossing, pleating, flocking, embroidery, expanded foam, and/or napping. In some embodiments, the fibers, yarns and/or textiles comprising recombinant protein fibers, comprise a softening finish, such as silicone compounds, emulsified oils, sulphonated oils, and/or waxes. In some embodiments, the fibers, yarns and/or textiles comprising recombinant protein fibers, comprise a wrinkle resistant finish, such as formaldehyde, di-methylol urea, di-methylol ethylene urea, di-methylol di-hydroxyl ethylene urea, and/or modified di-methylol di-hydoxyl ethylene urea. In some embodiments, the fibers, yarns and/or textiles comprising recombinant protein fibers, comprise a functional finish, such as waterproof finishes (such as with a resin, wax and/or oil), water repellant finishes (such as silicones, fluorocarbons, and/or paraffins), flame retardant finishes (such as tetrakis hydroxymethyl phosphonium chloride), moth proof finishes (such as fluorine compounds, naphthalene, DDT, paradichloro benzene), mildew fungus prevention finishes (such as boric acid), and/or antistatic finishes (such as moisture absorbing films).

In some embodiments, filament, spun or blended yarns containing RPFs can be incorporated into knit textiles with a push/pull construction. For example, textiles may be knit using a double knit construction either circular or warp knit where the hydrophobic RPFs or non-RPFs can be located in a layer next to the skin, and hydrophilic RPFs or non-RPFs can be located in a layer away from the skin, so that the moisture can be carried along the outside of the fiber through capillary action. Once the moisture reaches the outer hydrophilic fibers it is spread quickly across the outer surface of the fabric where it can evaporate and hence keep the wearer drier and more comfortable.

In some embodiments, filament, spun or blended yarns containing RPFs can be incorporated into woven textiles with a push/pull construction. For example, textiles may be woven using a double weave construction where the hydrophobic RPFs or non-RPFs can be located in a layer next to the skin, and hydrophilic RPFs or non-RPFs yarns can be located in a layer away from the skin, so that the moisture can be carried along the outside of the fiber through capillary action. Once the moisture reaches the outer hydrophilic fibers it is spread quickly across the outer surface of the fabric where it can evaporate and hence keep the wearer drier and more comfortable.

In different embodiments, some fibers, yarns and textiles characteristics can be grouped together. For example, fibers can be engineered to have high moisture absorption and have high extensibility. In fact, all of the fibers, yarns and textiles properties discussed in this disclosure can be combined with each other. However, in some cases the quantification of the fibers, yarns or textiles property and the method by which the property is obtained, are both important, and may change which properties can be combined. For example, moisture absorption can be imparted to the fibers by increasing the ratio of poly-alanine to glycine-rich regions in the protein sequence, however, increasing the ratio of poly-alanine regions in the protein sequence tends to the make the fiber less extensible. Table 2 illustrates combinations of fibers, yarns and textiles properties that are not mutually exclusive (Y), and fibers properties that are mutually exclusive (N).

## Table 2: Fibers, yarns and textiles properties, viable combinations

| | moisture absorption | wickability | antimicrobial | extensibility | tenacity | initial modulus | toughness | cross-section | linear density (or diameter) |
|---|---|---|---|---|---|---|---|---|---|
| moisture absorption | | Y | Y | Y | Y | Y | Y | Y | Y |
| wickability | | | Y | Y | Y | Y | Y | Y | Y |
| antimicrobial | | | | Y | Y | Y | Y | Y | Y |
| extensibility | | | | | Y | Y | Y | Y | Y |
| tenacity | | | | | | Y | Y | Y | Y |
| initial modulus | | | | | | | Y | Y | Y |
| toughness | | | | | | | | Y | Y |
| cross-section | | | | | | | | | Y |
| linear density (or diameter) | | | | | | | | | |

METHODS OF FORMING RECOMBINANT PROTEIN FIBER YARNS AND TEXTILES

Individual recombinant protein fibers are made into yarns to be used in textiles. There are different methods of forming yarns from RPFs and there are different methods of forming textiles from yarns comprising RPFs, which produce yarns and textiles with different structures and properties.

Depending on the type of yarn desired, several filament yarn forming methods can be used to make filament yarns containing recombinant protein fibers. These methods may include simple twisting of flat filament fibers using a silk throwing apparatus or continuous spinning. Textured filament yarns comprising recombinant protein fibers can be further subjected to processes that arrange the straight filaments into crimped, coiled or looped filaments to create bulk, texture or stretch. Some examples of methods used for processing textured filament yarns comprising recombinant protein fibers are air jet texturing, false twist texturing, or stuffer box texturing. Filament yarns may also be texturized during the spinning using false twist texturizing, air jet texturizing or stuffer box apparatus. Heating, chemically bonding or plying may also be employed.

In some embodiments, the yarns comprising recombinant protein fibers are manufactured using a ring spinning apparatus. In some embodiments, the yarns comprising recombinant protein fibers are manufactured using an open end spinning apparatus. In some embodiments, the yarns comprising recombinant protein fibers are manufactured using an air-jet spinning apparatus. In certain embodiments, twist is applied resulting in a twist angle optimized for desired mechanical, structural or other properties of the yarn. In certain embodiments, the twist applied to the inner core of the yarn has a different twist angle compared with the outer skin of the yarn. Throughout this disclosure "spun" yarns can refer to ring spun yarns, open end spun yarns, air-jet spun yarns, vortex spun yarns, or any other method of producing a yarn where the yarn comprises staple fibers.

In some embodiments, the blended yarn comprising RPFs and/or non-RPFs is manufactured by spinning. The structure of a spun yarn is influenced by the spinning methods parameters. The properties of the spun yarn are influenced by the structure of the yarn, as well as the constituent fibers. In embodiments, the blended yarn structure and the recombinant protein fibers (RPFs) properties and the type of non-recombinant protein fibers blended with the RPFs are all chosen to impart various characteristics to the resulting yarns. In some embodiments, the blended yarns are manufactured using a ring spinning apparatus. In some embodiments, the blended yarns are manufactured using an open end spinning apparatus. In some embodiments, the blended yarns are manufactured using an air-jet spinning apparatus. In many embodiments, twist is applied of a certain twist angle to optimize the mechanical properties of the blended yarn. In many embodiments, the twist applied to the inner core of the yarn has a different twist angle compared with the outer skin of the blended yarn.

In some embodiments, a method of making a spun yarn is employed, wherein a plurality of recombinant protein fibers is provided, the fiber are cut into staple, the fibers are conveyed the fibers to a spinning apparatus, and twist is provided to spin the fibers into a yarn. In some embodiments, the spinning apparatus is a ring spinning apparatus. In some embodiments, the spinning apparatus is an open end spinning apparatus. In some embodiments, the spinning apparatus is an air jet spinning apparatus. In some embodiments, the fibers are carded prior to spinning. In some embodiments, the fibers are combed prior to spinning.

In some embodiments, a method of making a blended spun yarn is employed, wherein a plurality of recombinant protein fibers and non-recombinant protein fibers is provided, the fibers are cut into staple, the fibers are loaded in to a spinning apparatus, and twist is provided to spin the fibers into a yarn. In some embodiments, the spinning apparatus is a ring spinning apparatus. In some embodiments, the spinning apparatus is an open end spinning apparatus. In some embodiments, the spinning apparatus is an air jet spinning apparatus. In some embodiments, the fibers are carded prior to spinning. In some embodiments, the fibers are combed prior to spinning.

In some embodiments, the yarns comprising recombinant protein fibers are manufactured into textiles, for example by weaving or knitting. In some embodiments, recombinant protein fibers are manufactured into textiles by knitting using a circular knitting apparatus, a warp knitting apparatus, a flat knitting apparatus, a one piece knitting apparatus, or a 3-D knitting apparatus. In some embodiments, recombinant protein fibers are manufactured into textiles by weaving using a plain weave loom, a dobby loom or a jacquard loom. In some embodiments, recombinant protein fibers are manufactured into textiles using a 3d printing method. In some embodiments, recombinant protein fibers are manufactured into non-woven textiles using techniques such as wet laying, spin bonding, stitch bonding, spunlacing (i.e., hydroentanglement), or needlepunching. In embodiments, the textile construction, the yarn structure and the recombinant protein fiber properties are chosen to impart various characteristics to the resulting yarns and textiles.

EXAMPLES

EXAMPLE 1: RECOMBINANT PROTEIN FIBER SPINNING

Copolymers in this example were secreted from *Pichia pastoris* commonly used for the expression of recombinant DNA using published techniques, such as those described in WO2015042164 A2, especially at paragraphs 114-134. In some embodiments, a secretion rate of at least 20 mg /g DCW / hr (DCW = dry cell weight) was observed. The secreted proteins were purified, dried, and dissolved in a formic acid-based spinning solvent, using standard techniques, to generate a homogenous spin dope.

The fibers in this example were produced using methods described in this disclosure, and extruding the spin dope through a 50-200 $\mu$m diameter orifice with 2:1 ratio of length to diameter into a room temperature alcohol-based coagulation bath comprising 20% formic acid with a residence time of 28 seconds. Fibers were pulled out of the coagulation bath under tension, strung through a wash bath consisting of 100% alcohol drawn to 4 times their length, and subsequently allowed to dry.

EXAMPLE 2: RECOMBINANT PROTEIN FIBER CROSS-SECTION

Using the synthesis methods in the fibers in Example 1, morphology of extruded fibers was varied by adjusting various parameters of a coagulation bath. For example, hollow core fibers were synthesized by having a higher ethanol content of the coagulation bath, as described below. In another example, corrugated morphologies were produced by increasing residence time in a coagulation bath, as described below.

The fibers of the present disclosure processed with residence times in coagulation baths greater than 60 seconds show corrugated cross-sections, as described above.

Fibers of the present disclosure processed with ethanol: water ratio in a coagulation bath of 80:20% by volume, or higher fraction of ethanol, include hollow cores, as described above.

EXAMPLE 3: RECOMBINANT PROTEIN FIBER MECHANICAL PROPERTIES

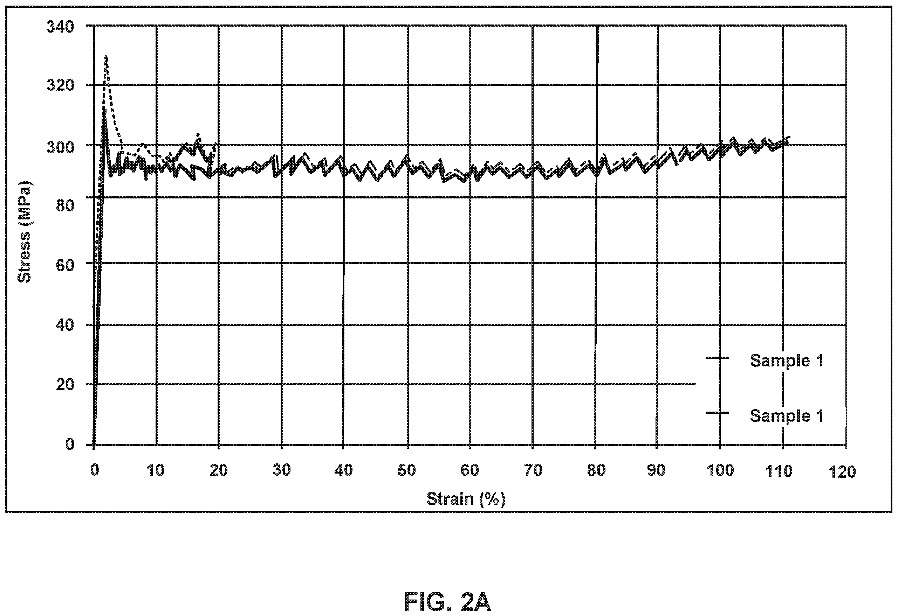

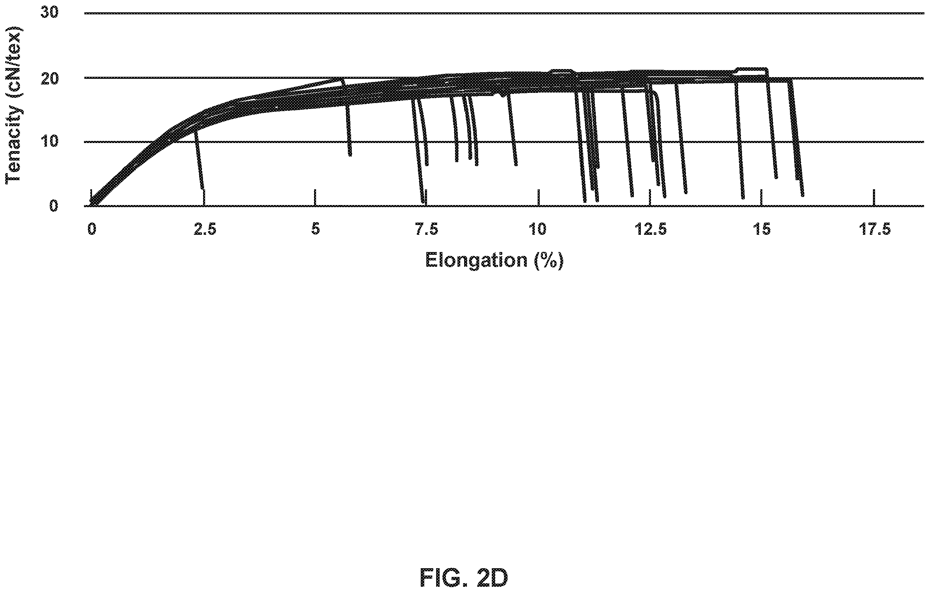

FIGS. 2A-2D show various mechanical properties of measured samples of the fibers, with the compositions described herein, and produced by the methods described in Example 1.

Some of the mechanical properties of the fibers in this disclosure are reported in units of MPa (i.e., $10^6$ N/m$^2$, or force per unit area), and some are reported in units of cN/tex (force per linear density). The measurements of fibers mechanical properties reported in MPa were obtained using a custom instrument, which includes a linear actuator and calibrated load cell, and the fiber diameter was measured by light microscopy. The measurements of fibers mechanical properties reported in cN/tex were obtained using FAVIMAT testing equipment, which includes a measurement of the fiber linear density using a vibration method (e.g., according to ASTM D1577). To accurately convert measurements from MPa to cN/tex, an estimate of the bulk density (e.g., in g/cm$^3$) of the fiber is used. An expression that can be used to convert a force per unit area in MPa, "FA", to a force per linear density in cN/tex, "FLD," using the bulk density in g/cm$^3$, "BD", is FLD = FA/(10*BD). Since the bulk density of recombinant silk can vary, a given value of fiber tenacity in MPa does not translate to a given value of fiber tenacity in cN/tex. However, if the bulk density of the recombinant silk is assumed to be from 1.1 to 1.4 g/cm$^3$, then mechanical property values can be converted from one set of units into the other within a certain range of error. For example, a maximum tensile stress of 100 MPa is equivalent to about 9.1 cN/tex if the mass density of the fiber is 1.1 g/cm$^3$, and a maximum tensile stress of 100 MPa is equivalent to about 7.1 cN/tex if the mass density of the fiber is 1.4 g/cm$^3$.

A set of 4 fibers was tested for tensile mechanical properties using an instrument including a linear actuator and calibrated load cell. Fibers were pulled at 1% per second strain rate until failure. Fiber diameters were measured with light microscopy at 20x magnification using image processing software. The mean diameter was 10.25 um, +/- 1 st.dev = 6.4 - 14.1 um. The mean max tensile stress was 97.9 MPa, +/- 1 st.dev = 68.1 - 127.6 MPa. The mean max strain was 37.2%, +/- 1 st.dev = -11.9 - 86.3%. The mean yield stress was 87.4 MPa, +/- 1 st.dev = 59.2 - 115.6 MPa. The mean initial modulus was 5.2 GPa, +/- 1 st.dev = 3.5 - 6.9 GPa.

A different set of 7 fibers was tested for tensile mechanical properties using an instrument including a linear actuator and calibrated load cell. Fibers were pulled at 1% per second strain rate until failure. Fiber diameters were measured with light microscopy at 20x magnification using image processing software. The mean diameter was 6.2 um, +/- 1 st.dev = 4.9 - 7.5 um. The mean max tensile stress was 127.9 MPa, +/- 1 st.dev = 106.4 - 149.3 MPa. The mean max strain was 105.5%, +/- 1 st.dev = 61.0 - 150.0%. The mean yield stress was 109.8 MPa, +/- 1 st.dev = 91.4 - 128.2 MPa. The mean initial modulus was 5.5 GPa, +/-1 st.dev = 4.4 - 6.6 GPa.

A different set of 4 fibers was tested for tensile mechanical properties using an instrument including a linear actuator and calibrated load cell. Fibers were pulled at 1% per second strain rate until failure. Fiber diameters were measured with light microscopy at 20x magnification using image processing software. The mean diameter was 8.9 um, +/- 1 st.dev = 6.9 - 11.0 um. The mean max tensile stress was 93.2 MPa, +/- 1 st.dev = 81.4 - 105.0 MPa. The mean max strain was 128.9%, +/- 1 st.dev = 84.0 - 173.8%. The mean yield stress was 83.3 MPa, +/- 1 st.dev = 64.9 - 101.7 MPa. The mean initial modulus was 2.6 GPa, +/- 1 st.dev = 1.5 - 3.8 GPa.

FIG. 2A shows a stress strain curve of fibers of the present disclosure in which maximum tensile stress is greater than 100 MPa, maximum tensile stress is from 111 MPa to 130 MPa, initial modulus is from 6 GPa to 7.1 GPa, maximum strain (i.e., extensibility) is from 18% to 111%, and the yield stress is from 107 MPa to 112 MPa. The ultimate tensile stress is also greater than 100 MPa for one of the fibers in this figure.

While not wishing to be bound by theory, the structural properties of the proteins within the spider silk are theorized to be related to fiber mechanical properties. Crystalline regions in a fiber have been linked with the tensile strength of a fiber, while the amorphous regions have been linked to the extensibility of a fiber. The major ampullate (MA) silks tend to have higher strengths and less extensibility than the flagelliform silks, and likewise the MA silks have higher volume fraction of crystalline regions compared with flagelliform silks. Furthermore, theoretical models based on the molecular dynamics of crystalline and amorphous regions of spider silk proteins, support the assertion that the crystalline regions have been linked with the tensile strength of a fiber, while the amorphous regions have been linked to the extensibility of a fiber. Additionally, the theoretical modeling supports the importance of the secondary, tertiary and quaternary structure on the mechanical properties of recombinant protein fibers. For instance, both the assembly of nano-crystal domains in a random, parallel and serial spatial distributions, and the strength of the interaction forces between entangled chains within the amorphous regions, and between the amorphous regions and the nano-crystalline regions, influenced the theoretical mechanical properties of the resulting fibers.

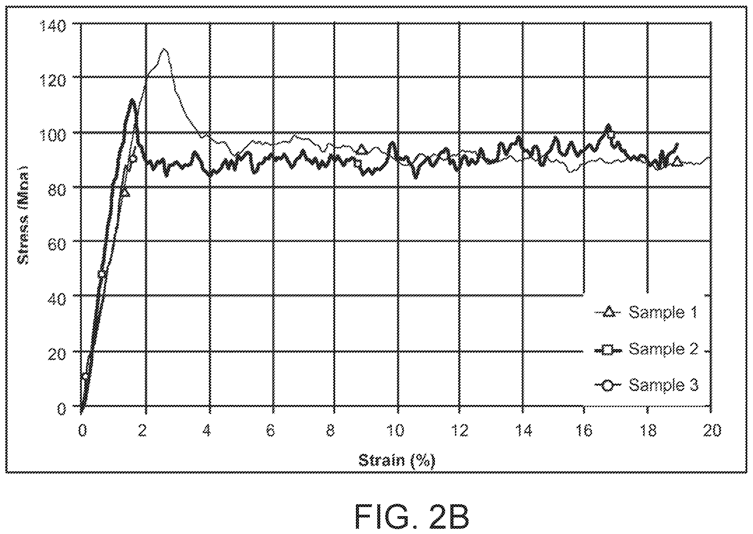

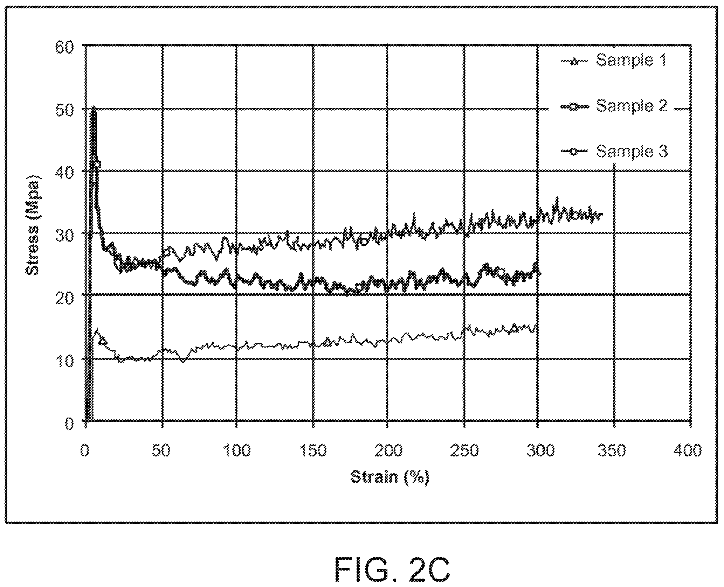

A set of the fibers described herein was tested for tensile mechanical properties using an instrument including a linear actuator and calibrated load cell. Fibers were pulled at 1% per second strain rate until failure. Fiber diameters were measured with light microscopy at 20x magnification using image processing software. Fig. 2B shows stress strain curves of fibers from the present disclosure, where the mean maximum stress ranged from 24-172 MPa. The mean maximum strain ranged from 2-342%. Fig. 2C shows stress strain curves of fibers from the present disclosure, where the mean initial modulus ranged from 1617-7040 MPa, and the mean elongation at break was from approximately 300% to 350%. The average toughness of three fibers was measured at 0.5 MJ m-3 (standard deviation of 0.2), 20 MJ m-3 (standard deviation of 0.9), and 59.2 MJ m-3 (standard deviation of 8.9). The diameters ranged from 4.48-12.7 $\mu$m.

FIG. 7 shows stress strain curves of 23 fibers of the present disclosure, which includes fibers with maximum tensile stress greater than 20 cN/tex, and the average of the maximum tensile stresses of the 23 fibers is about 18.6 cN/tex. The maximum tensile stress ranges from about 17 to 21 cN/tex, and the standard deviation of the maximum tensile stress in this example is about 1.0 cN/tex. The average initial modulus of the 23 fibers is about 575 cN/tex, and the standard deviation in this example is about 6.7 cN/tex. The average maximum elongation of the 23 fibers is about 10.2%, and the standard deviation in this example is about 3.6%. The average work of rupture (a measure of toughness) of the 23 fibers is about 0.92 cN*cm, and the standard deviation in this example is about 0.43 cN*cm. The average linear density of the 23 fibers is about 3.1 dtex, and the standard deviation in this example is about 0.11 dtex.

EXAMPLE 4: RECOMBINANT PROTEIN FIBER MOISTURE DATA

There are a number of different ways that fibers, yarns and textiles interact with water, and different measurements provide insight into different types of fiber-water interactions. Fibers in this example have compositions described herein and are produced by the methods described in Example 1.

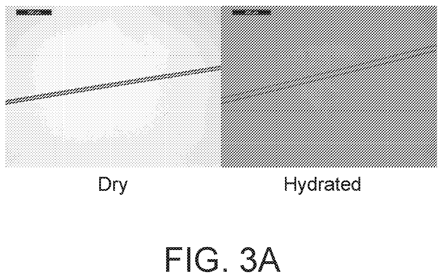

Fig. 3A shows an example of data from a fiber swelling measurement, which investigates the morphological change of fibers when submerged in water at a temperature of 21 °C +/- 1 °C. This is related to the ability of the fiber to absorb water into the fiber. The average diameter of the RPFs tested was approximately 25 microns before submerging in the water, and varied from approximately 30 to approximately 33 microns after submerging in the water and waiting 60 minutes. The RPFs therefore had a diameter change from approximately 20 to 35% when submerged under water at a temperature of 21 °C +/- 1 °C.

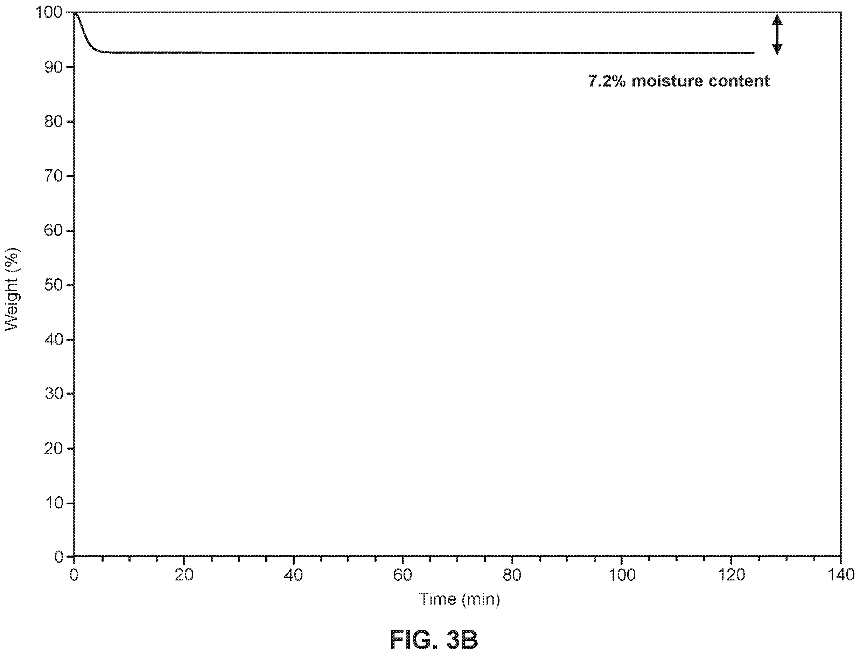

RPFs described in this disclosure were tested for their moisture regain and moisture content. Fig. 3B shows data from one sample measured in a RPF moisture regain, and moisture content experiment. In this experiment, the moisture in the RPF sample was allowed to equilibrate in an environment with approximately 65% relative humidity, and then heated to 110 °C in a thermogravimetric analysis (TGA) system and the mass change was measured over time. The conditioned weight is the weight of the RPF sample after reaching equilibrium in the approximately 65% relative humidity environment. The dry weight was the weight of the RPF sample after being held at 110 °C until approximately steady state was reached. The conditioned weight for the RPF sample in Fig. 3B was 5.1930 mg, and the lost water weight was 0.4366 mg. The mean moisture regain of the RPFs measured in this example, defined as the lost water weight divided by the dry weight, was from approximately 7.5% to 8%. The mean moisture content of the RPFs measured in this example, defined as the lost water weight divided by the conditioned weight, was approximately 7 to 7.5%.

EXAMPLE 5: RECOMBINANT PROTEIN FIBER YARNS PROPERTIES

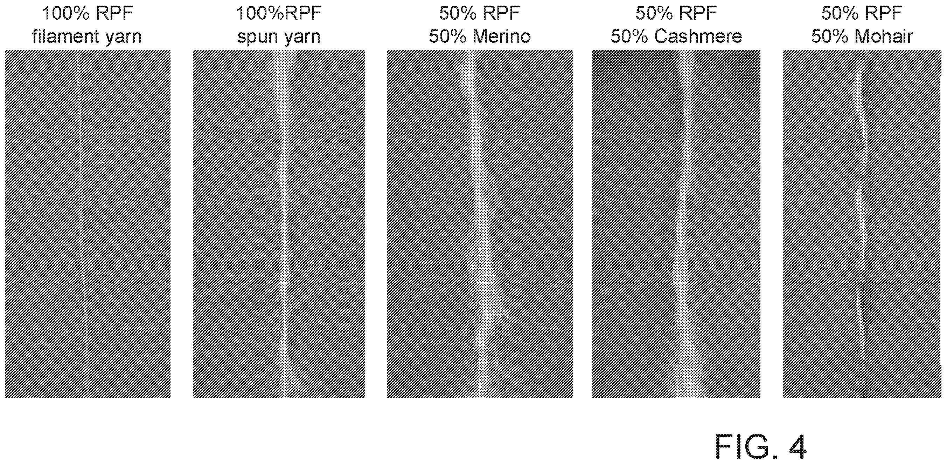

Fibers in this example have compositions described herein and are produced by the methods described in Example 1, and were manufactured into yarns using methods described in this Example. Fig. 4 illustrates 5 yarns comprising recombinant protein fibers: a filament yarn comprising recombinant protein fibers, a spun yarn comprising recombinant protein fibers, and three blended yarns comprising recombinant protein fibers and non-RPF wool fibers. The mechanical properties of samples of these yarns containing RPFs were measured using ASTM D2256-10. The initial gage length for all of the mechanical property measurements in this example was 127 mm.

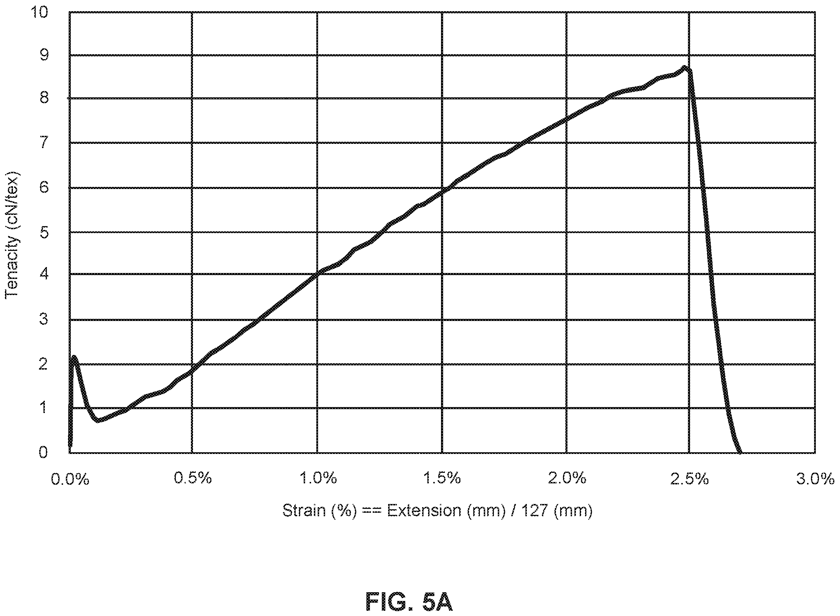

The filament yarn contains 50 recombinant protein fibers, and a twist was provided to form a filament yarn with a twist per inch of approximately 2.5. The mean diameter of the RPFs in this example was approximately 10 microns, with a mean linear density of approximately 3 dtex per filament. The mean linear density of the RPF filament yarn was approximately 750 den. The RPF filament yarn mean maximum tenacity ranged from approximately 4.4 to 8.7 cN/tex (0.50 to 0.99 gf/den), the mean elongation at break ranged from approximately 2.5% to 6%, and the mean rupture force ranged from approximately 350 to 750 gf. Fig. 5A shows the mechanical properties of one RPF filament yarn sample. This RPF filament yarn sample had a maximum tenacity of approximately 8.7 cN/tex (0.99 gf/den), an extensibility (i.e., elongation at break) of approximately 2.6%, and a rupture force of approximately 740 gf.

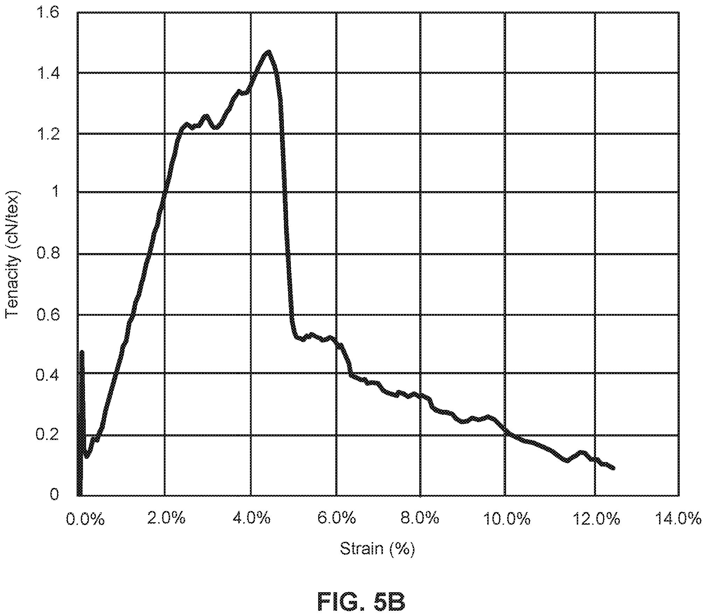

The spun yarn contains recombinant protein fiber, which was cut to approximately 1.5" staple length on average. The recombinant protein fiber was processed with a sample carder before spinning. Additionally, the carding process can break the fiber into various length staple. Following carding, the yarns were spun using a sample making type of spinning apparatus and were drafted by hand into the yarn orifice. Following this step, the yarn was plied with itself creating a 2 ply yarn. The RPF spun yarn in this example has a twist per inch of approximately 3.5. The mean diameter of the RPF in this example was approximately 10 microns, with a mean linear density of approximately 3 dtex. The mean linear density of the RPF spun yarn was approximately 3080 den. The RPF spun yarn mean maximum tenacity ranged from approximately 1.06 to 1.50 cN/tex (0.12 to 0.17 gf/den), the mean elongation at break ranged from approximately 12% to 22%, the mean rupture force ranged from approximately 350 to 515 gf, and the mean Young's modulus ranged from approximately 22 to 42 cN/tex (2.5 to 4.8 gf/den). Fig. 5B shows the mechanical properties of one RPF spun yarn sample. This RPF spun yarn sample had a maximum tenacity of approximately 1.50 cN/tex (0.17 gf/den), an extensibility (i.e., elongation at break) of approximately 12%, a Young's modulus of approximately 22 cN/tex (2.5 gf/den), and a rupture force of approximately 440 gf.

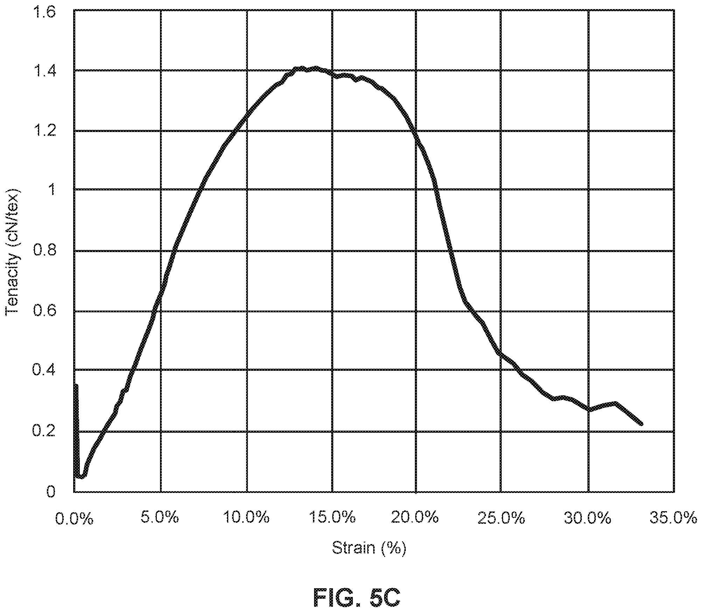

A blended yarn was spun from approximately 50% RPF and 50% cashmere wool. The blended yarn in this example contains recombinant protein fiber that was cut to approximately 1.5" staple length on average, and cashmere wool fiber that was cut to approximately 1.5" staple length on average. The recombinant protein fiber and cashmere wool fiber was processed with a carder to create an intimate blend of fiber before spinning. Additionally, the carding process can break the fiber into various length staple. Following carding, the RFP/cashmere blended yarns were spun using a sample making type of spinning apparatus and were drafted by hand into the yarn orifice. Following this step, the blended yarn was plied with itself creating a 2 ply yarn. The RPF/cashmere blended yarn in this example has a twist per inch of approximately 3.5. The mean diameter of the RPF in this example was approximately 10 microns, with a mean linear density of approximately 3 dtex. The cashmere fiber in the blended yarn has a mean diameter of approximately 16 microns. The mean linear density of the RPF/cashmere blended yarn was approximately 4200 den. The RPF/cashmere blended yarn mean maximum tenacity ranged from approximately 0.71 to 1.77 cN/tex (0.08 to 0.20 gf/den), the mean elongation at break ranged from approximately 26% to 33%, the mean rupture force ranged from approximately 350 to 840 gf, and the mean Young's modulus ranged from approximately 10.6 to 25.6 cN/tex (1.2 to 2.9 gf/den). Fig. 5C shows the mechanical properties of one RPF/cashmere blended yarn sample. This RPF / cashmere blended yarn sample had a maximum tenacity of approximately 1.4 cN/tex (0.16 gf/den), an extensibility (i.e., elongation at break) of approximately 33%, a Young's modulus (similar to an initial modulus) of approximately 18 cN/tex (2.0 gf/den), and a rupture force of approximately 680 gf.

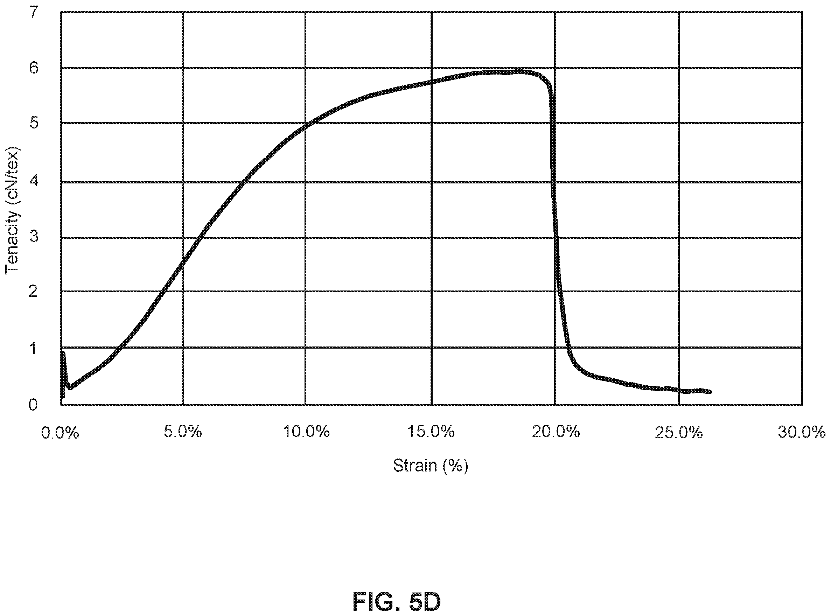

A blended yarn was spun from approximately 50% RPF and 50% merino wool. The blended yarn in this example contains recombinant protein fiber that was cut to approximately 1.5" staple length on average, and merino wool fiber that was cut to approximately 4-6" staple length on average. The recombinant protein fiber and merino wool fiber was processed with a sample carder before spinning. Additionally, the carding process can break the fiber into various length staple. Following carding, the RPF/merino blended yarns were spun using a sample making type of spinning apparatus and were drafted by hand into the yarn orifice. Following this step, the RPF/merino blended yarn was plied with itself creating a 2 ply yarn. The RPF/merino blended yarn in this example has a twist per inch of approximately 3.5. The mean diameter of the RPF in this example was approximately 10 microns, with a mean linear density of approximately 3 dtex. The merino fiber in the blended yarn has a mean diameter of approximately 18 microns. The mean linear density of the RPF/merino wool blended yarn was approximately 4200 den. The RPF/merino wool blended yarn mean maximum tenacity ranged from approximately 3.18 to 6.00 cN/tex (0.36 to 0.68 gf/den), the mean elongation at break ranged from approximately 18% to 32%, the mean rupture force ranged from approximately 770 to 1450 gf, and the mean Young's modulus ranged from approximately 44 to 74 cN/tex (5.0 to 8.4 gf/den). Fig. 5D shows the mechanical properties of one RPF/merino wool blended yarn sample. This RPF / merino wool blended yarn sample had a maximum tenacity of approximately 6.0 cN/tex (0.68) gf/den), an extensibility (i.e., elongation at break) of approximately 26%, a Young's modulus (similar to an initial modulus) of approximately 64 cN/tex (7.3 gf/den), and a rupture force of approximately 1450 gf.

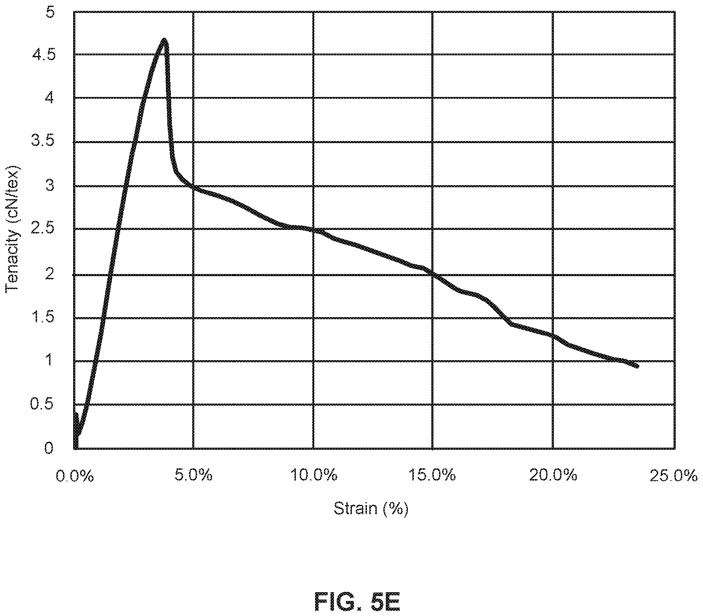

A blended plied spun yarn was spun from approximately 50% RPF and 50% mohair wool. The yarn in this example is a blended plied spun yarn that contains recombinant protein fiber that was cut to approximately 1.5" staple length on average, and mohair wool fiber that was cut to approximately 3" staple length on average. The recombinant protein fiber and mohair fiber was processed with a carder before spinning. Additionally, the carding process can break the fiber into various length staple. Following carding, a RPF yarn and a mohair wool yarn were spun using a sample making type of spinning apparatus and were drafted by hand into the yarn orifice. Then, in this case, the mohair spun yarn was plied with a RPF spun yarn, such that one ply is mohair, one ply is RPF, to create a RPF/mohair blended plied spun yarn. RPF/mohair blended plied spun yarn has a twist per inch of approximately 3.5. The mean diameter of the RPF in this example was approximately 10 microns, with a mean linear density of approximately 3 dtex. The mohair fiber in the blended yarn has a mean diameter of approximately 30 microns. The mean linear density of the RPF/mohair blended plied yarn was approximately 4100 den. The RPF/mohair blended plied spun yarn mean maximum tenacity ranged from approximately 1.60 to 4.68 cN/tex (0.18 to 0.53 gf/den), the mean elongation at break ranged from approximately 14% to 24%, the mean rupture force ranged from approximately 730 to 2180 gf, and the mean Young's modulus ranged from approximately 16.0 to 132 cN/tex (1.8 to 15.0 gf/den). Fig. 5E shows the mechanical properties of one RPF/mohair blended yarn sample. This RPF/mohair blended plied spun yarn sample had a maximum tenacity of approximately 4.68 cN/tex (0.53 gf/den), an extensibility (i.e., elongation at break) of approximately 23%, a Young's modulus (similar to an initial modulus) of approximately 132 cN/tex (15.0 gf/den), and a rupture force of approximately 1390 gf.

In the three blended yarns above, the wool imparts warmth to the blended RPF containing yarn. The RPFs in this example have smaller diameters than the wool fibers, which brings improved softness and drape to a fabric constructed from the blended RPF/wool yarns. The RPFs in this example have smoother surfaces than the wool fibers as well, and therefore the luster of the blended RPF/wool yarn is higher than a pure wool yarn.

ADDITIONAL CONSIDERATIONS

The foregoing description of the embodiments of the disclosure has been presented for the purpose of illustration; it is not intended to be exhaustive or to limit the claims to the precise forms disclosed. Persons skilled in the relevant art can appreciate that many modifications and variations are possible in light of the above disclosure.

The language used in the specification has been principally selected for readability and instructional purposes, and it may not have been selected to delineate or circumscribe the inventive subject matter. It is therefore intended that the scope of the disclosure be limited not by this detailed description, but rather by any claims that issue on an application based hereon. Accordingly, the disclosure of the embodiments is intended to be illustrative, but not limiting, of the scope of the invention, which is set forth in the following claims.

**Claims**

1. A yarn, comprising:
   a plurality of recombinant protein fibers twisted around a common axis, wherein

   the mean initial modulus of the recombinant protein fiber is greater than 550 cN/tex, and
   the recombinant protein fiber comprises a polypeptide comprising at least two occurrences of a repeat unit, the repeat unit comprising:

   more than 150 amino acid residues and having a molecular weight of at least 10kDal;
   an alanine-rich region with 6 or more consecutive amino acids, comprising an alanine content of at least 80%; and
   a glycine-rich region with 12 or more consecutive amino acids, comprising a glycine content of at least 40% and an alanine content of less than 30%.

2. The yarn of claim 1, wherein the mean initial modulus of the recombinant protein fiber is measured using ASTM D2256-10 or ASTM D3822-14.

3. The yarn of any of claims 1-2, wherein the yarn is a filament yarn, a spun yarn, or a blended yarn; and/or
   wherein the initial modulus of the recombinant protein fibers is from 550 cN/tex to 1000 cN/tex.

4. The yarn of any of claims 1-3, wherein the initial modulus of the recombinant protein fibers is measured using ASTM D2256-10; or
   wherein the initial modulus of the recombinant protein fibers is measured using ASTM D3822-14.

5. The yarn of any of claims 1-4, wherein the recombinant protein fiber repeat unit comprises from 150 to 1000 amino acid residues; and/or

   wherein the repeat unit has a molecular weight from 10 kDal to 100 kDal; and/or
   wherein the repeat unit comprises from 2 to 20 alanine-rich regions; and/or
   wherein each alanine-rich region comprises from 6 to 20 consecutive amino acids and an alanine content from 80% to 100%; and/or
   wherein the repeat unit comprises from 2 to 20 glycine-rich regions; and/or
   wherein each glycine-rich region comprises from 12 to 150 consecutive amino acids and a glycine content from 40% to 80%; and/or
   wherein the repeat unit comprises 315 amino acid residues, 6 alanine-rich regions, and 6 glycine-rich regions,

   wherein the alanine-rich regions comprise from 7 to 9 consecutive amino acids, and alanine content of 100%, and
   wherein the glycine-rich regions comprise from 30 to 70 consecutive amino acids, and glycine content from 40 to 55%; and/or

   wherein

   each repeat unit has at least 95% sequence identity to a sequence that comprises from 2 to 20 quasi-repeat units,
   each quasi-repeat unit having a composition comprising {GGY-[GPG-X1]n1-GPS-(A)n2}, wherein for each quasi-repeat unit:

   X1 is independently selected from the group consisting of SGGQQ, GAGQQ, GQGPY, AGQQ, and SQ; and
   n1 is from 4 to 8, and n2 is from 6 to 10, optionally wherein:

   **i)** n1 is from 4 to 5 for at least half of the quasi-repeat units; and/or
   **ii)** n2 is from 5 to 8 for at least half of the quasi-repeat units; and/or

**iii)** said quasi-repeat unit has at least 95% sequence identity to a MaSp2 dragline silk protein subsequence.

6. The yarn of any one of claims 1-5, wherein:

the alanine-rich regions form a plurality of nanocrystalline beta-sheets; and
the glycine-rich regions form a plurality of beta-turn structures; and/or
wherein the repeat unit comprises SEQ ID NO: 1; and/or
wherein the mean diameter change of the fibers is greater than 5% when submerged in water at a temperature of 21 °C +/- 1 °C; and/or
wherein when submerged in water at a temperature of 21 °C +/- 1 °C, the recombinant protein fiber has a mean diameter change from 20% to 35%; and/or
wherein the mean maximum tensile strength of the recombinant protein fibers is greater than 15 cN/tex; and/or
wherein the mean maximum tensile strength of the recombinant protein fibers is measured using ASTM D2256-10 or ASTM D3822-14.

7. The yarn of any of claims 1-6, wherein the mean maximum tensile strength of the recombinant protein fibers is from 15 cN/tex to 100 cN/tex; and/or
wherein the mean maximum tensile strength of the recombinant protein fibers is measured using ASTM D2256-10 or ASTM D3822-14.

8. The yarn of any of claims 1-7, wherein the mean extensibility of the recombinant protein fibers is greater than 3%; and/or
wherein the mean extensibility of the recombinant protein fibers is measured using ASTM D2256-10 or ASTM D3822-14.

9. The yarn of any of claims 1-8, wherein the mean extensibility of the recombinant protein fibers is from 3% to 20%; and/or
wherein the mean extensibility of the recombinant protein fibers is measured using ASTM D2256-10 or ASTM D3822-14.

10. The yarn of any of claims 1-9, wherein the mean linear density of the recombinant protein fibers is less than 1 denier; and/or

wherein the mean linear density of the recombinant protein fibers is less than 5 denier; and/or
wherein the mean linear density of the recombinant protein fibers is between 5 and 10 denier; and/or
wherein the recombinant protein fibers have a mean toughness greater than 2 cN/tex; and/or
wherein the mean toughness of the recombinant protein fibers is measured using ASTM D2256-10 or ASTM D3822-14.

11. The yarn of any of claims 1-10, wherein the recombinant protein fibers have a cross-section that is substantially circular; and/or

wherein the recombinant protein fibers have a longitudinal axis, an inner surface and an outer surface, the inner surface defining a hollow core parallel to the longitudinal axis of the fiber; and/or
wherein the recombinant protein fibers have a longitudinal axis and an outer surface, the outer surface including a plurality of corrugations, each corrugation of the plurality substantially parallel to the longitudinal axis of the fiber.

12. The yarn of any of claims 1-11, wherein the recombinant protein fiber does not comprise a chemical residue or finish selected from the group consisting of an antimicrobial finish, such as brominated phenols, quaternary ammonium compounds, zirconium peroxide, ethylene oxide, organo-silver and/or tin compounds, a luster finish, such as calendaring, beetling and/or burning-out, a drape finish, such as parchmentizing, acid designs, burning-out and/or sizing, a texture finish, such as shearing, brushing, 3D or raised embossing, pleating, flocking, embroidery, expanded foam, and/or napping, a softening finish, such as silicone compounds, emulsified oils, sulphonated oils, and/or waxes, a wrinkle resistant finish, such as formaldehyde, di-methylol urea, di-methylol ethylene urea, di-methylol dihydroxyl ethylene urea, and/or modified di-methylol di-hydoxyl ethylene urea, and/or a functional finish, such as a waterproof finish (such as with a resin, wax and/or oil), a water repellant finish (such as silicones, fluorocarbons, and/or paraffins), a flame retardant finish (such as tetrakis hydroxymethyl phosphonium chloride), a moth proof finish (such as fluorine compounds, naphthalene, DDT, paradichloro benzene), a mildew fungus prevention finish (such as boric acid), and an antistatic finishes (such as moisture absorbing films); or
wherein the recombinant protein fiber comprises a chemical residue or finish selected from the group consisting of an antimicrobial finish, such as brominated phenols, quaternary ammonium compounds, zirconium peroxide, ethylene oxide, organo-silver and/or tin compounds, a luster finish, such as calendaring, beetling and/or burning-out, a drape finish, such as parchmentizing, acid designs, burning-out and/or sizing, a texture finish, such as shearing, brushing, 3D or raised embossing, pleating, flocking, embroidery, expanded foam, and/or napping, a softening finish, such as silicone compounds, emulsified oils, sulphonated oils, and/or waxes, a wrinkle resistant finish, such as formaldehyde, di-methylol urea, di-methylol ethylene urea, di-methylol di-hydroxyl ethylene urea, and/or modified di-methylol di-hydoxyl ethylene urea, and/or a functional finish, such as a waterproof finish (such as with a resin, wax and/or oil), a water repellant finish (such as silicones, fluorocarbons, and/or paraffins), a flame retardant finish (such as tetrakis hydroxymethyl phosphonium chloride), a moth proof finish (such as fluorine compounds, naphthalene, DDT, paradichloro benzene), a mildew fungus prevention finish (such as boric acid), and an antistatic finishes (such as moisture absorbing films).

13. The yarn of any of claims 1-12, wherein the yarn is a blended yarn comprising recombinant protein fibers; and fibers selected from the group consisting of cotton, wool, merino, mohair, polyamide, linen, acrylic, polyester, spandex, and combinations thereof; and/or

    wherein the yarn twist is from 5 to 100 turns per centimeter; and/or
    wherein the maximum tensile strength of the yarn is greater than 1 cN/tex measured using ASTM D2256-10; and/or
    wherein the maximum tensile strength of the yarn is from 1 cN/tex to 100 cN/tex measured using ASTM D2256-10; and/or
    wherein the initial modulus of the yarn is greater than 550 cN/tex measured using ASTM D2256-10; and/or
    wherein the initial modulus of the yarn is from 550 cN/tex to 1000 cN/tex measured using ASTM D2256-10; and/or
    wherein the extensibility of the yarn is greater than 3% measured using ASTM D2256-10; and/or
    wherein the extensibility of the yarn is from 3% to 20% measured using ASTM D2256-10; and/or
    wherein the recombinant protein fibers are texturized.

14. A textile comprising the yarn of any of claims 1-13, wherein:

    **i)** the textile comprises a plain weave 1/1 textile with warp density of 72 warps/cm and pick density of 40 picks/cm and wherein the textile has a mean horizontal wicking rate greater than 1 mm/s when tested using a standard moisture wicking assay; and/or wherein the textile has an increase in colony forming units less than 100 times in 24 hours when tested using a standard antimicrobial assay; or
    **ii)** the textile is a knitted textile, optionally wherein the textile is selected from the group consisting of a circular-knitted textile, flat-knitted textile, or a warp-knitted textiles; or
    **iii)** the textile is a woven textile, optionally wherein the textile is selected from the group consisting of a plain weave textile, dobby weave textile, and jacquard weave textile; or
    **iv)** the textile is a non-woven textile, optionally wherein the textile is selected from the group consisting of a needle punched textile, a spunlace textile, a wet-laid textile, a dry-laid textile, a melt-blown textile, and 3-D printed non-woven textile.

15. A textile, comprising the yarn of any of claims 1-13, wherein:

    **i)** the textile has a high maximum tenacity, and wherein the mean maximum tensile strength is greater than 7.7 cN/tex per yarn; or
    **ii)** the textile is a highly comfortable textile, and wherein:

        when submerged in water at a temperature of 21 °C +/- 1 °C, the recombinant protein fiber has a mean diameter change of greater than 5%; and
        a mean denier less than 5; and
        when tested using a standard moisture wicking assay, the textile has a mean horizontal wicking rate greater than 1 mm/s, optionally wherein the textile comprises a plain weave 1/1 textile with warp density of 72 warps/cm, and a pick density of 40 picks/cm; or

    **iii)** the textile is an ultra-soft textile, and wherein the textile is a knitted textile, a woven textile, or a non-woven textile, and the yarn comprises:

an outer sheath comprising the recombinant protein fiber, wherein the outer sheath comprises a greater twist (lesser inclination) as compared to a twist in a center core of the filament yarn; and
wherein the mean dernier of the recombinant protein fiber is less than 5.

**Patentansprüche**

1. Garn, umfassend:

eine Vielzahl rekombinanter Proteinfasern, die um eine gemeinsame Achse gedreht sind, wobei
der mittlere Anfangsmodul der rekombinanten Proteinfaser größer als 550 cN/tex ist und
die rekombinante Proteinfaser ein Polypeptid umfasst, das mindestens zwei Vorkommen einer Wiederholungseinheit umfasst, wobei die Wiederholungseinheit Folgendes umfasst:

mehr als 150 Aminosäurereste und ein Molekulargewicht von mindestens 10 kDal;
eine alaninreiche Region mit 6 oder mehr aufeinanderfolgenden Aminosäuren, die einen Alaningehalt von mindestens 80 % umfasst; und
eine glycinreiche Region mit 12 oder mehr aufeinanderfolgenden Aminosäuren, die einen Glycingehalt von mindestens 40 % und einen Alaningehalt von weniger als 30 % umfasst.

2. Garn nach Anspruch 1, wobei der mittlere Anfangsmodul der rekombinanten Proteinfaser unter Verwendung von ASTM D2256-10 oder ASTM D3822-14 gemessen wird.

3. Garn nach einem der Ansprüche 1-2, wobei das Garn ein Filamentgarn, ein Spinngarn oder ein Mischgarn ist; und/oder
wobei der Anfangsmodul der rekombinanten Proteinfasern 550 cN/tex bis 1000 cN/tex beträgt.

4. Garn nach einem der Ansprüche 1-3, wobei der Anfangsmodul der rekombinanten Proteinfasern unter Verwendung von ASTM D2256-10 gemessen wird; oder
wobei der Anfangsmodul der rekombinanten Proteinfasern unter Verwendung von ASTM D3822-14 gemessen wird.

5. Garn nach einem der Ansprüche 1-4, wobei die Wiederholungseinheit der rekombinanten Proteinfaser 150 bis 1000 Aminosäurereste umfasst; und/oder

wobei die Wiederholungseinheit ein Molekulargewicht von 10 kDal bis 100 kDal aufweist; und/oder
wobei die Wiederholungseinheit 2 bis 20 alaninreiche Regionen umfasst; und/oder
wobei jede alaninreiche Region 6 bis 20 aufeinanderfolgende Aminosäuren und einen Alaningehalt von 80 % bis 100 % umfasst; und/oder
wobei die Wiederholungseinheit 2 bis 20 glycinreiche Regionen umfasst; und/oder
wobei jede glycinreiche Region 12 bis 150 aufeinanderfolgende Aminosäuren und einen Glycingehalt von 40 % bis 80 % umfasst; und/oder
wobei die Wiederholungseinheit 315 Aminosäurereste, 6 alaninreiche Regionen und 6 glycinreiche Regionen umfasst,
wobei die alaninreichen Regionen 7 bis 9 aufeinanderfolgende Aminosäuren und einen Alaningehalt von 100 % umfassen und
wobei die glycinreichen Regionen 30 bis 70 aufeinanderfolgende Aminosäuren und einen Glycingehalt von 40 bis 55 % umfassen; und/oder
wobei
jede Wiederholungseinheit eine Sequenzidentität von mindestens 95 % mit einer Sequenz aufweist, die 2 bis 20 Quasi-Wiederholungseinheiten umfasst,
jede Quasi-Wiederholungseinheit eine Zusammensetzung aufweist, die {GGY-[GPG-X1]n1-GPS-(A)n2} umfasst, wobei für jede Quasi-Wiederholungseinheit:

X1 unabhängig aus der Gruppe ausgewählt ist, die aus SGGQQ, GAGQQ, GQGPY, AGQQ und SQ besteht; und
n1 4 bis 8 ist und n2 6 bis 10 ist, wobei optional:

**i)** n1 von 4 bis 5 für mindestens die Hälfte der Quasi-Wiederholungseinheiten ist; und/oder
**ii)** n2 von 5 bis 8 für mindestens die Hälfte der Quasi-Wiederholungseinheiten ist; und/oder
**iii)** die Quasi-Wiederholungseinheit eine Sequenzidentität von mindestens 95 % mit einer Subsequenz des MaSp2-Dragline-Seidenproteins aufweist.

**6.** Garn nach einem der Ansprüche 1-5, wobei:

die alaninreichen Regionen eine Vielzahl nanokristalliner Beta-Faltblätter bilden; und
die glycinreichen Regionen eine Vielzahl von Beta-Schleifen-Strukturen bilden; und/oder
wobei die Wiederholungseinheit SEQ ID NO: 1 umfasst; und/oder
wobei die mittlere Durchmesseränderung der Fasern mehr als 5 % beträgt, wenn sie in Wasser mit einer Temperatur von 21 °C +/- 1 °C getaucht sind; und/oder
wobei die rekombinante Proteinfaser, wenn sie in Wasser bei einer Temperatur von 21 °C +/- 1 °C getaucht ist, eine mittlere Durchmesseränderung von 20 % bis 35 % aufweist; und/oder
wobei die mittlere maximale Zugfestigkeit der rekombinanten Proteinfasern mehr als 15 cN/tex beträgt; und/oder
wobei die mittlere maximale Zugfestigkeit der rekombinanten Proteinfasern unter Verwendung von ASTM D2256-10 oder ASTM D3822-14 gemessen wird.

**7.** Garn nach einem der Ansprüche 1-6, wobei die mittlere maximale Zugfestigkeit der rekombinanten Proteinfasern 15 cN/tex bis 100 cN/tex beträgt; und/oder
wobei die mittlere maximale Zugfestigkeit der rekombinanten Proteinfasern unter Verwendung von ASTM D2256-10 oder ASTM D3822-14 gemessen wird.

**8.** Garn nach einem der Ansprüche 1-7, wobei die mittlere Dehnbarkeit der rekombinanten Proteinfasern mehr als 3 % beträgt; und/oder
wobei die mittlere Dehnbarkeit der rekombinanten Proteinfasern unter Verwendung von ASTM D2256-10 oder ASTM D3822-14 gemessen wird.

**9.** Garn nach einem der Ansprüche 1-8, wobei die mittlere Dehnbarkeit der rekombinanten Proteinfasern 3 % bis 20 % beträgt; und/oder
wobei die mittlere Dehnbarkeit der rekombinanten Proteinfasern unter Verwendung von ASTM D2256-10 oder ASTM D3822-14 gemessen wird.

**10.** Garn nach einem der Ansprüche 1-9, wobei die mittlere lineare Dichte der rekombinanten Proteinfasern weniger als 1 Denier beträgt; und/oder

wobei die mittlere lineare Dichte der rekombinanten Proteinfasern weniger als 5 Denier beträgt; und/oder
wobei die mittlere lineare Dichte der rekombinanten Proteinfasern zwischen 5 und 10 Denier beträgt; und/oder
wobei die rekombinanten Proteinfasern eine mittlere Zähigkeit von mehr als 2 cN/tex aufweisen; und/oder
wobei die mittlere Zähigkeit der rekombinanten Proteinfasern unter Verwendung von ASTM D2256-10 oder ASTM D3822-14 gemessen wird.

**11.** Garn nach einem der Ansprüche 1-10, wobei die rekombinanten Proteinfasern einen Querschnitt aufweisen, der im Wesentlichen kreisförmig ist; und/oder

wobei die rekombinanten Proteinfasern eine Längsachse, eine innere Oberfläche und eine äußere Oberfläche aufweisen, wobei die innere Oberfläche einen hohlen Kern parallel zu der Längsachse der Faser definiert; und/oder
wobei die rekombinanten Proteinfasern eine Längsachse und eine äußere Oberfläche aufweisen, wobei die äußere Oberfläche eine Vielzahl von Wellungen enthält, wobei jede Wellung der Vielzahl im Wesentlichen parallel zu der Längsachse der Faser verläuft.

**12.** Garn nach einem der Ansprüche 1-11, wobei die rekombinante Proteinfaser keinen chemischen Rückstand oder keine Ausrüstung umfasst, die aus der Gruppe ausgewählt ist, die aus einer antimikrobiellen Ausrüstung, wie etwa bromierten Phenolen, quartären Ammoniumverbindungen, Zirkoniumperoxid, Ethylenoxid, Organo-Silber- und/oder Zinnverbindungen, einer Glanzausrüstung, wie etwa Kalandrieren, Beeteln und/oder Ausbrennen, einer Drapierausrüstung, wie etwa Pergamentierung, Säuremuster, Ausbrennen und/oder Schlichten, einer Texturausrüstung, wie etwa Scheren, Bürsten, 3D- oder Hochprägen, Plissieren, Beflocken, Sticken, expandierter Schaum und/oder Rauen, einer weichmachenden Ausrüstung, wie etwa Silikonverbindungen, emulgierte Öle, sulfonierte Öle und/oder Wachse, einer knitterfesten Ausrüstung, wie etwa Formaldehyd, Dimethylolharnstoff, Dimethylolethylenharnstoff, Dimethyloldihydroxylethylenharnstoff und /oder modifizierter Dimethyloldihydroxylethylenharnstoff, und/oder einer funktionellen Ausrüstung, wie etwa eine wasserfeste Ausrüstung (wie etwa mit einem Harz, Wachs und/oder Öl), eine wasserabweisende Ausrüstung (wie etwa Silikone, Fluorkohlenwasserstoffe und/oder Paraffine), eine flammhemmende Ausrüstung (wie Tetrakishydroxymethylphosphoniumchlorid), eine mottenfeste Ausrüstung (wie etwa Fluorverbindungen, Naphthalin, DDT, Paradichlorbenzol), eine Ausrüstung zur Verhinderung von Schimmelpilzen (wie etwa Borsäure) und eine antistatische Ausrüstungen (wie etwa feuchtigkeitsabsorbierende Folien), besteht; oder wobei die rekombinante Proteinfaser einen chemischen Rückstand oder eine Ausrüstung umfasst, die aus der Gruppe ausgewählt ist, die aus einer antimikrobiellen Ausrüstung, wie etwa bromierten Phenolen, quartären Ammoniumverbindungen, Zirkoniumperoxid, Ethylenoxid, Organo-Silber- und/oder Zinnverbindungen, einer Glanzausrüstung, wie etwa Kalandrieren, Beeteln und/oder Ausbrennen, einer Drapierausrüstung, wie etwa Pergamentierung, Säuremuster, Ausbrennen und/oder Schlichten, einer Texturausrüstung, wie etwa Scheren, Bürsten, 3D- oder Hochprägen, Plissieren, Beflocken, Sticken, expandierter Schaum und/oder Rauen, einer weichmachenden Ausrüstung, wie etwa Silikonverbindungen, emulgierte Öle, sulfonierte Öle und/oder Wachse, einer knitterfesten Ausrüstung, wie etwa Formaldehyd, Dimethylolharnstoff, Dimethylolethylenharnstoff, Dimethyloldihydroxylethylenharnstoff und /oder modifizierter Dimethyloldihydroxylethylenharnstoff, und/oder einer funktionellen Ausrüstung, wie etwa eine wasserfeste Ausrüstung (wie etwa mit einem Harz, Wachs und/oder Öl), eine wasserabweisende Ausrüstung (wie etwa Silikone, Fluorkohlenwasserstoffe und/oder Paraffine), eine flammhemmende Ausrüstung (wie Tetrakishydroxymethylphosphoniumchlorid), eine mottenfeste Ausrüstung (wie etwa Fluorverbindungen, Naphthalin, DDT, Paradichlorbenzol), eine Ausrüstung zur Verhinderung von Schimmelpilzen (wie etwa Borsäure) und eine antistatische Ausrüstungen (wie etwa feuchtigkeitsabsorbierende Folien), besteht.

13. Garn nach einem der Ansprüche 1-12, wobei das Garn ein Mischgarn ist, das rekombinante Proteinfasern umfasst; und Fasern, die aus der Gruppe ausgewählt sind, die aus Baumwolle, Wolle, Merino, Mohair, Polyamid, Leinen, Acryl, Polyester, Spandex und Kombinationen davon besteht; und/oder

wobei die Garndrehung 5 bis 100 Windungen pro Zentimeter beträgt; und/oder
wobei die maximale Zugfestigkeit des Garns größer als 1 cN/tex ist, gemessen unter Verwendung von ASTM D2256-10; und/oder
wobei die maximale Zugfestigkeit des Garns 1 cN/tex bis 100 cN/tex beträgt, gemessen unter Verwendung von ASTM D2256-10; und/oder
wobei der Anfangsmodul des Garns größer als 550 cN/tex ist, gemessen unter Verwendung von ASTM D2256-10; und/oder
wobei der Anfangsmodul des Garns 550 cN/tex bis 1000 cN/tex beträgt, gemessen unter Verwendung von ASTM D2256-10; und/oder
wobei die Dehnbarkeit des Garns mehr als 3 % beträgt, gemessen unter Verwendung von ASTM D2256-10; und/oder
wobei die Dehnbarkeit des Garns 3 % bis 20 % beträgt, gemessen unter Verwendung von ASTM D2256-10; und/oder
wobei die rekombinanten Proteinfasern texturiert sind.

14. Textil, umfassend das Garn nach einem der Ansprüche 1-13, wobei:

i) das Textil ein Textil mit 1/1-Leinwandbindung mit einer Kettdichte von 72 Kettfäden/cm und einer Schussdichte von 40 Schussfäden/cm umfasst und wobei das Textil eine mittlere horizontale Transportrate von mehr als 1 mm/s bei Prüfung unter Verwendung eines standardmäßigen Feuchtigkeitstransporttests aufweist; und/oder wobei das Textil einen Anstieg koloniebildender Einheiten um weniger als das 100-Fache innerhalb von 24 Stunden aufweist, wenn es unter Verwendung eines standardmäßigen antimikrobiellen Tests geprüft wird; oder
ii) das Textil ein gestricktes Textil ist, wobei das Textil optional aus der Gruppe ausgewählt ist, die aus einem rundgestrickten Textil, einem flachgestrickten Textil oder einem kettengewirkten Textil besteht; oder
iii) das Textil ein gewebtes Textil ist, wobei das Textil optional aus der Gruppe ausgewählt ist, die aus einem Textil mit Leinwandbindung, einem Textil mit Schaftbindung und einem Textil mit Jacquardbindung besteht; oder
iv) das Textil ein nicht gewebtes Textil ist, wobei das Textil optional aus der Gruppe ausgewählt ist, die aus einem genadelten Textil, einem Spunlace-Textil, einem Nassvlies-Textil, einem Trockenvlies-Textil, einem schmelzgesponnenen Textil und 3D-gedrucktem, nicht gewebtem Textil besteht.

15. Textil, umfassend das Garn nach einem der Ansprüche 1-13, wobei:

**i)** das Textil eine hohe maximale Reißfestigkeit aufweist und wobei die mittlere maximale Zugfestigkeit mehr als 7,7 cN/tex pro Garn beträgt; oder

**ii)** das Textil ein äußerst komfortables Textil ist und wobei:

die rekombinante Proteinfaser, wenn sie in Wasser bei einer Temperatur von 21 °C +/- 1 °C getaucht ist, eine mittlere Durchmesseränderung von mehr als 5 % aufweist; und

einen mittleren Denier von weniger als 5; und

bei Prüfung unter Verwendung eines standardmäßigen Feuchtigkeitstransporttest das Textil eine mittlere horizontale Transportrate von mehr als 1 mm/s aufweist, wobei das Textil optional ein Textil mit 1/1-Leinwandbindung mit einer Kettdichte von 72 Kettfäden/cm und einer Schussdichte von 40 Schussfäden/cm umfasst; oder

**iii)** das Textil ein ultraweiches Textil ist und wobei das Textil ein gestricktes Textil, ein gewebtes Textil oder ein nicht gewebtes Textil ist und das Garn Folgendes umfasst:

einen äußeren Mantel, der die rekombinante Proteinfaser umfasst, wobei der äußere Mantel eine stärkere Drehung (geringere Neigung) im Vergleich zu einer Drehung in einem zentralen Kern des Filamentgarns aufweist; und

wobei der mittlere Denier der rekombinanten Proteinfaser weniger als 5 beträgt.

## Revendications

1. Fil, comprenant :

   une pluralité de fibres de protéine recombinée torsadées autour d'un axe commun, dans lesquelles

   le module initial moyen de la fibre de protéine recombinée est supérieur à 550 cN/tex, et

   la fibre de protéine recombinée comprend un polypeptide comprenant au moins deux occurrences d'un motif récurrent, le motif récurrent comprenant :

   plus de 150 résidus d'acides aminés et comportant un poids moléculaire d'au moins 10 kDal ;

   une région riche en alanine avec 6 acides aminés consécutifs ou plus, comprenant une teneur en alanine d'au moins 80 % ; et

   une région riche en glycine avec 12 acides aminés consécutifs ou plus, comprenant une teneur en glycine d'au moins 40 % et une teneur en alanine inférieure à 30 %.

2. Fil selon la revendication 1, ledit module initial moyen de la fibre de protéine recombinée étant mesuré en utilisant la norme ASTM D2256-10 ou ASTM D3822-14.

3. Fil selon l'une quelconque des revendications 1 à 2, ledit fil étant un fil continu, un fil filé ou un fil mélangé ; et/ou ledit module initial des fibres de protéine recombinée étant compris entre 550 cN/tex et 1000 cN/tex.

4. Fil selon l'une quelconque des revendications 1 à 3, ledit module initial des fibres de protéine recombinée étant mesuré en utilisant la norme ASTM D2256-10 ; ou

   ledit module initial des fibres de protéine recombinée étant mesuré en utilisant la norme ASTM D3822-14.

5. Fil selon l'une quelconque des revendications 1 à 4, ledit motif récurrent de fibre de protéine recombinée comprenant de 150 à 1000 résidus d'acides aminés ; et/ou

   ledit motif récurrent comportant un poids moléculaire de 10 kDal à 100 kDal ; et/ou

   ledit motif récurrent comprenant de 2 à 20 régions riches en alanine ; et/ou

   chaque région riche en alanine comprenant de 6 à 20 acides aminés consécutifs et une teneur en alanine de 80 % à 100 % ; et/ou

   ledit motif récurrent comprenant de 2 à 20 régions riches en glycine ; et/ou

   chaque région riche en glycine comprenant de 12 à 150 acides aminés consécutifs et une teneur en glycine de 40 % à 80 % ; et/ou

   ledit motif récurrent comprenant 315 résidus d'acides aminés, 6 régions riches en alanine et 6 régions riches en glycine,

lesdites régions riches en alanine comprenant de 7 à 9 acides aminés consécutifs et une teneur en alanine de 100 %, et

lesdites régions riches en glycine comprenant de 30 à 70 acides aminés consécutifs et une teneur en glycine de 40 à 55 % ; et/ou

chaque motif de répétition comportant une identité de séquence d'au moins 95 % avec une séquence qui comprend de 2 à 20 motifs quasi-récurrents,

chaque motif quasi-récurrent comportant une composition comprenant {GGY-[GPG-X1]n1-GPS-(A)n2}, pour chaque motif quasi-récurrent :

X1 étant indépendamment choisi dans le groupe constitué par SGGQQ, GAGQQ, GQGPY, AGQQ et SQ ; et n1 valant de 4 à 8, et n2 valant de 6 à 10, éventuellement :

**i)** n1 étant compris entre 4 et 5 pour au moins la moitié des motifs quasi-récurrents ; et/ou
**ii)** n2 étant compris entre 5 et 8 pour au moins la moitié des motifs quasi-récurrents ; et/ou
**iii)** ledit motif quasi-récurrent comportant une identité de séquence d'au moins 95 % avec une sous-séquence de protéine de soie de fil de rappel MaSp2.

**6.** Fil selon l'une quelconque des revendications 1 à 5 :

lesdites régions riches en alanine formant une pluralité de feuillets bêta nanocristallins ; et
lesdites régions riches en glycine formant une pluralité de structures à virage bêta ; et/ou
ledit motif récurrent comprenant SEQ ID n° : 1 et/ou
le changement de diamètre moyen des fibres étant supérieur à 5 % lorsqu'elles sont immergées dans l'eau à une température de 21°C +/- 1°C ; et/ou
lorsqu'elle est immergée dans l'eau à une température de 21°C +/- 1°C, ladite fibre de protéine recombinée comportant un changement de diamètre moyen de 20 % à 35 % ; et/ou
la résistance à la traction maximale moyenne des fibres de protéine recombinée étant supérieure à 15 cN/tex ; et/ou
la résistance à la traction maximale moyenne des fibres de protéine recombinée étant mesurée en utilisant la norme ASTM D2256-10 ou ASTM D3822-14.

**7.** Fil selon l'une quelconque des revendications 1 à 6, ladite résistance à la traction maximale moyenne des fibres de protéine recombinée étant de 15 cN/tex à 100 cN/tex ; et/ou
ladite résistance à la traction maximale moyenne des fibres de protéine recombinée étant mesurée en utilisant la norme ASTM D2256-10 ou ASTM D3822-14.

**8.** Fil selon l'une quelconque des revendications 1 à 7, l'extensibilité moyenne des fibres de protéine recombinée étant supérieure à 3 % ; et/ou
ladite extensibilité moyenne des fibres de protéine recombinée étant mesurée en utilisant la norme ASTM D2256-10 ou ASTM D3822-14.

**9.** Fil selon l'une quelconque des revendications 1 à 8, ladite extensibilité moyenne des fibres de protéine recombinée étant de 3 % à 20 % ; et/ou
ladite extensibilité moyenne des fibres de protéine recombinée étant mesurée en utilisant la norme ASTM D2256-10 ou ASTM D3822-14.

**10.** Fil selon l'une quelconque des revendications 1 à 9, la densité linéaire moyenne des fibres de protéine recombinée étant inférieure à 1 denier ; et/ou

ladite densité linéaire moyenne des fibres de protéine recombinée étant inférieure à 5 deniers ; et/ou
ladite densité linéaire moyenne des fibres de protéine recombinée étant comprise entre 5 et 10 deniers ; et/ou
lesdites fibres de protéine recombinée comportant une ténacité moyenne supérieure à 2 cN/tex ; et/ou
ladite ténacité moyenne des fibres de protéine recombinée étant mesurée en utilisant la norme ASTM D2256-10 ou ASTM D3822-14.

**11.** Fil selon l'une quelconque des revendications 1 à 10, lesdites fibres de protéine recombinée comportant une section transversale qui est sensiblement circulaire ; et/ou

lesdites fibres de protéine recombinée comportant un axe longitudinal, une surface interne et une surface externe, la surface interne définissant une âme creuse parallèle à l'axe longitudinal de la fibre ; et/ou lesdites fibres de protéine recombinée comportant un axe longitudinal et une surface externe, ladite surface externe comprenant une pluralité d'ondulations, chaque ondulation de la pluralité étant sensiblement parallèle à l'axe longitudinal de la fibre.

**12.** Fil selon l'une quelconque des revendications 1 à 11, ladite fibre de protéine recombinée ne comprenant pas de résidu ou de finition chimique choisi dans le groupe constitué par une finition antimicrobienne, telle que des phénols bromés, des composés d'ammonium quaternaire, du peroxyde de zirconium, de l'oxyde d'éthylène, des composés d'organo-argent et/ou étain, une finition brillante, telle que le calandrage, le martelage et/ou le brûlage, une finition drapée, telle que le parcheminage, les dessins à l'acide, le brûlage et/ou l'encollage, une finition texturée, telle que le cisaillement, le brossage, le gaufrage 3D ou en relief, le plissage, le flocage, la broderie, la mousse expansée et/ou le duvetage, une finition adoucissante, telle que les composés de silicone, les huiles émulsionnées, les huiles sulfonées et/ou les cires, une finition infroissable, telle que le formaldéhyde, la di-méthylol urée, la di-méthylol éthylène urée, la di-méthylol di-hydroxy éthylène urée et/ou la di-méthylol di-hydroxy éthylène urée modifiée, et/ou une finition fonctionnelle, telle qu'une finition imperméable (telle qu'avec une résine, une cire et/ou une huile), une finition hydrofuge (telle que des silicones, des fluorocarbones et/ou des paraffines), une finition ignifuge (telle que le chlorure de tétrakis hydroxyméthylphosphonium), une finition antimite (telle que les composés fluorés, le naphtalène, le DDT, le paradichlorobenzène), une finition anti-moisissure (telle que l'acide borique) et une finition antistatique (telle que les films absorbant l'humidité) ; ou ladite fibre de protéine recombinée comprenant un résidu ou un finition chimique choisi dans le groupe constitué par une finition antimicrobienne, telle que les phénols bromés, les composés d'ammonium quaternaire, le peroxyde de zirconium, l'oxyde d'éthylène, les composés organo-argent et/ou étain, une finition brillante, telle que le calandrage, le martelage et/ou le brûlage, une finition drapée, comme le parcheminage, les dessins à l'acide, le brûlage et/ou l'encollage, une finition texturée, comme le cisaillement, le brossage, le gaufrage 3D ou en relief, le plissage, le flocage, la broderie, la mousse expansée et/ou le duvetage, une finition adoucissante, telle que les composés de silicone, les huiles émulsionnées, les huiles sulfonées et/ou les cires, une finition infroissable, telle que le formaldéhyde, la diméthylol urée, la diméthylol éthylène urée, la diméthylol dihydroxy éthylène urée et/ou de la diméthylol dihydroxy éthylène urée modifiée, et/ou une finition fonctionnelle, telle qu'une finition imperméable (comme avec une résine, une cire et/ou une huile), une finition hydrofuge (telle que les silicones, les fluorocarbones et/ou les paraffines), une finition ignifuge (telle que le chlorure de tétrakis hydroxyméthylphosphonium), une finition antimite (telle que les composés fluorés, le naphtalène, le DDT, le paradichlorobenzène), une finition anti-moisissure (telle que l'acide borique) et une finition antistatique (telle que les films absorbant l'humidité).

**13.** Fil selon l'une quelconque des revendications 1 à 12, ledit fil étant un fil mélangé comprenant des fibres de protéine recombinée ; et des fibres sélectionnées dans le groupe constitué par le coton, la laine, le mérinos, le mohair, le polyamide, le lin, l'acrylique, le polyester, le spandex et les combinaisons de ceux-ci ; et/ou

la torsion du fil étant de 5 à 100 tours par centimètre ; et/ou ladite résistance à la traction maximale du fil étant supérieure à 1 cN/tex mesurée en utilisant la norme ASTM D2256-10 ; et/ou ladite résistance à la traction maximale du fil étant de 1 cN/tex à 100 cN/tex mesurée en utilisant la norme ASTM D2256-10 ; et/ou ledit module initial du fil étant supérieur à 550 cN/tex mesuré en utilisant la norme ASTM D2256-10 ; et/ou ledit module initial du fil étant compris entre 550 cN/tex et 1000 cN/tex mesuré en utilisant la norme ASTM D2256-10 ; et/ou ladite extensibilité du fil étant supérieure à 3 % mesurée en utilisant la norme ASTM D2256-10 ; et/ou ladite extensibilité du fil étant de 3 % à 20 % mesurée en utilisant la norme ASTM D2256-10 ; et/ou lesdites fibres de protéine recombinée étant texturées.

**14.** Textile comprenant le fil selon l'une quelconque des revendications 1 à 13 :

**i)** ledit textile comprenant un textile à tissage toile 1/1 avec une densité de chaîne de 72 chaînes/cm et une densité de duites de 40 duites/cm et ledit textile comportant une vitesse de capillarité horizontale moyenne supérieure à 1 mm/s lorsqu'il est testé en utilisant un test de capillarité d'humidité standard ; et/ou ledit textile comportant une augmentation des unités formant colonies inférieure à 100 fois en 24 heures lorsqu'il est testé en utilisant un test antimicrobien standard ; ou **ii)** ledit textile étant un textile tricoté, éventuellement ledit textile étant choisi dans le groupe constitué par un textile tricoté circulaire, un textile tricoté à plat ou un textile tricoté en chaîne ; ou

**iii)** ledit textile étant un textile tissé, éventuellement ledit textile étant choisi dans le groupe constitué par un textile à tissage toile, un textile à tissage en ratière et un textile à tissage jacquard ; ou

**iv)** ledit textile étant un textile non tissé, éventuellement choisi dans le groupe constitué par un textile aiguilleté, un textile filé, un textile obtenu par voie humide, un textile obtenu par voie sèche, un textile soufflé par fusion, et un textile non tissé imprimé en 3D.

**15.** Textile comprenant le fil selon l'une quelconque des revendications 1 à 13 :

**i)** ledit textile comportant une ténacité maximale élevée, et ladite résistance à la traction maximale moyenne étant supérieure à 7,7 cN/tex par fil ; ou

**ii)** ledit textile étant un textile très confortable, et :

lorsqu'elle est immergée dans l'eau à une température de 21°C +/- 1°C, ladite fibre de protéine recombinée comportant un changement de diamètre moyen supérieur à 5 % ; et

un denier moyen inférieur à 5 ; et

lorsqu'il est testé en utilisant un test de capillarité d'humidité standard, ledit textile comportant une vitesse de capillarité horizontale moyenne supérieure à 1 mm/s, éventuellement ledit textile comprenant un textile à tissage toile 1/1 avec une densité de chaîne de 72 chaînes/cm, et une densité de duines de 40 duines/cm ; ou

**iii)** ledit textile étant un textile ultra-doux, et ledit textile étant un textile tricoté, un textile tissé ou un textile non tissé, et ledit fil comprenant :

une gaine externe comprenant la fibre de protéine recombinée, ladite gaine externe comprenant une torsion plus importante (inclinaison moindre) par rapport à une torsion dans une âme centrale du fil de filaments ; et ledit denier moyen de la fibre de protéine recombinée étant inférieur à 5.

Motif $X_1$

Region $[GPG - X_1]_{n_1}$ AND $[GPS(A)]_{n_2}$

Quasi-repeat $\{GGY - [GPG - X_1]_{n_1} - GPS(A)_{n_2}\}$

| 1 | 2 | 3 | 4 | 5 | 6 |

Repeat Unit

FIG. 1

EP 3 307 765 B1

FIG. 2A

FIG. 2B

FIG. 2C

FIG. 2D

Dry          Hydrated

FIG. 3A

FIG. 3B

EP 3 307 765 B1

FIG. 4

EP 3 307 765 B1

FIG. 5A

FIG. 5B

FIG. 5C

FIG. 5D

EP 3 307 765 B1

FIG. 5E

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010123450 A
- WO 2015042164 A
- WO 2015042164 A2
- US 20140058066

**Non-patent literature cited in the description**

- **SMITH ; WATERMAN.** *Adv. Appl. Math.,* 1981, vol. 2, 482
- **NEEDLEMAN ; WUNSCH.** *J. Mol. Biol.,* 1970, vol. 48, 443
- **PEARSON ; LIPMAN.** *Proc. Nat'l. Acad. Sci. USA,* 1988, vol. 85, 2444
- **ALTSCHUL et al.** *J. Mol. Biol.,* 1990, vol. 215, 403-410
- **H.K., KAYNAK ; O. BABAARSLAN.** Woven Fabrics. InTech, 2012